# EUROPEAN PATENT APPLICATION

(11) **EP 1 574 567 A1**
(43) Date of publication of application: **14.09.2005**
(21) Application number: 04447062.3
(22) Date of filing: 11.03.2004
(51) Int. Cl.: C12N 9/24, A21D 2/26, A21D 8/04

(54) **Novel xylanases and their use**

(71) Applicant: Puratos Naamloze Vennootschap, 1702 Groot-Bijgaarden (BE)
(72) Inventor: Georis, Jacques, 6820 Florenville (BE); Dauvrin, Thierry, 4218 Couthuin (BE); Hoyoux, Anne, 4130 Tilff (BE); Collins, Tony, 4031 Angleur (BE); Feller, Georges, 4870 Trooz (BE)
(74) Representative: Van Malderen, Joelle

(57) **Abstract**

The present invention relates to novel enzymes with xylanolytic activity that belong to the glycoside hydrolase Family 8. The present invention in particular relates to enzymes isolated from bacterial psychrophilic strains that produce xylanases with an amino acid sequence as identified by any of SEQ ID NOs: 2, 4, 6, 8, 10, 12, 14, 16, 21, 23, 25, 27, 29, 31, 33, or a variant thereof. Another aspect of the invention relates to the corresponding genes.

These enzymes find many applications and are advantageously used for instance in feed and food applications such as baking. Compared to conventional xylanases, only small amounts of enzymes are needed to obtain a desired effect, such as an increase of the loaf volume and/or an increase in the width of cut on the surface of baked products.

## Description

### Field of the invention

The present invention concerns novel enzymes with xylanolytic activity as well as their corresponding genes. In particular, the present invention concerns xylanases isolated from psychrophilic microorganisms and/or belonging to glycoside hydrolase Family 8. The enzymes of the invention find many applications and are highly suitable for use in bread improving compositions.

### Background of the invention

Xylans are heteropolysaccharides that form the major component of hemicellulose in plant biomass. The backbone of these polysaccharides consists of a chain of β-1,4-linked xylopyranoside residues. This backbone chain may be substituted to varying degrees with various side chain groups, including acetyl, arabinosyl and/or glucuronosyl substituents. Furthermore, phenolic compounds such as ferulic or hydroxycinnamic acids may also be involved, through ester binding, in the cross linking of the xylan chains or/and in the cross-linking of xylan and lignin chains.

Endoxylanases (also referred to as endo-β-1,4-xylanases, pentosanases or hemicellulases) specifically hydrolyse the backbone of xylan. In some cases, however, their activity may be sterically hindered by side groups. Different types of xylanases have been described and their specificity towards their substrate may vary from one to another, some being more active on insoluble arabinoxylans for instance. In addition, the length of the oligomers produced also depends on the type of xylanase considered.

Glycoside hydrolases have been classified into 93 families (http://afmb.cnrs-mrs.fr/CAZY/) based on sequence homologies and thus reflects structural and mechanistic features. Because the fold of proteins is better conserved than the sequence, some of the families can be grouped in 'clans' (Henrissat B. 1991, Biochem. J. Vol. 280, p. 309). Endo-beta-1,4-xylanases are generally classified in Families 10 (formerly Family F) and 11 (formerly Family G) and are found to frequently have an inverse relationship between their pI and molecular weight. The Family 10 xylanases (EXs10) are generally larger and more complex than the Family 11 xylanases (EXs11). Moreover, these families display significant differences in their structures and catalytic properties. EXs10 present an (α/β)₈ barrel fold, have about 40% α-helix structures and belong to clan GH-A (Dominguez *et al*. 1995, Nat. Struct. Biol. Vol. 2, p. 569) while EXs11 exhibit a β-jelly roll fold, have about 3-5% α-helix structures and belong to clan GH-C (Törrönen *et al*. 1994, EMBO J. Vol. 13, p. 2493). EXs10 have a smaller substrate binding site and a lower substrate specificity. Furthermore, they frequently have endoglucanase activity and produce smaller oligosaccharides as compared to EXs11 (Biely *et* al. 1997, J. Biotechnol. Vol. 57, p. 151). Xylanases of both families as characterized to date retain the anomeric configuration of the glycosidic oxygen following hydrolysis in which two glutamates typically function as the catalytic residues (Jeffries, 1997, Curr. Opin. Biotechnol. Vol. 7, p. 337).

Xylanases are used in various industrial sectors including the food, feed and some technical industries.

In the food industry, xylanases are used in fruit, vegetable and plant processing, in wine making and brewing, in baking, milling, pastry and confectionery and in coffee processing. Their functions in these industries are very diverse. For example, in fruit and plant processing they improve the maceration process, juice clarification, the extraction yield and filtration efficiency, hence improving the process performance and product quality. Xylanases also reduce the wort viscosity in beer making, improve grape skin maceration in wine making and reduce haze in the final products. In baking, xylanases improve elasticity and strength of doughs, thereby allowing easier handling, larger loaf volume and improved bread texture. In coffee processing, xylanases reduce the viscosity of coffee extracts and improve the drying/lyophilization processes.

Xylanases are further used in feed for monogastric animals (swine and poultry) and ruminants. They decrease among others the content of non-starch polysaccharides, thereby reducing the intestinal viscosity and improving the utilization of proteins and starch present in the feed. Xylanases improve animal performance and increase the digestibility and nutritive value of poorly degradable feeds such as barley and wheat.

In the starch industry, the use of xylanases improves the gluten-starch separation process.

In the pulp and paper industry, xylanases are used to facilitate the pulping process and to reduce the use of mechanical pulping methods, hence reducing energy consumption. Xylanases also improve the fibrillation and drainage properties of pulp, hence improving the process efficiency and the paper strength. They also facilitate the de-inking processes and reduce the use of alkali in these processes.

Xylanases are further used in the enzymatic retting of textiles (flax, jute, ramie, hemp,...) and therefore reduce or replace chemical retting methods. They are also used in bioremediation for the treatment of agricultural and food industry wastes. Some bioconversion processes such as the production of fermentable products, renewable fuel (bioethanol) or fine chemicals for instance are also performed with the aid of xylanases.

Further applications for xylanases may still arrive in the future.

The use of xylanases (also referred to as endoxylanases, endo-β-1,4-xylanases, pentosanases or hemicellulases) in baking has been well known for a number of years. These dough-conditioning enzymes can improve the dough machinability and stability as well as the oven-spring and the crumb structure. Other effects of the enzymes are a larger loaf volume and a softer crumb.

The mechanism of action of xylanases in bread preparation is still not clearly elucidated. Wheat flour contains about 3 to 4% pentosans. These pentosans can absorb large amounts of water (up to 30%) and this water absorption contributes to the properties of the dough as well as to the quality of the final product. Partial hydrolysis of pentosans by pentosanases into water soluble short chain oligosaccharides increases the water binding capacity. In addition, the pentosans strongly interact with the gluten fraction of the flour to form a network. Pentosanases may help to relax this strong and rigid network, thereby allowing better dough expansion by the carbon dioxide formed by the yeast.

Many types of hemicellulase preparations have been used for the applications mentioned above, and are commercially available. They are produced by microbial fermentation using various microorganisms as enzyme sources. Some of these enzymes are also produced by genetically modified microorganisms. All documented commercial uses of xylanases relate to xylanases belonging to either glycoside hydrolase Family 10 or Family 11, as defined previously.

Examples of commercial xylanases are the xylanases from *Bacillus sp., Trichoderma sp., Humicola sp.* and *Aspergillus sp.*

A number of reviews describing the actual state of art in the field of xylanases have been recently published (see for example Shallom D & Shoham Y. 2003, Curr. Opin. Microbiol. Vol. 6, p. 219 - Subramaniyan S & Prema P. 2002, Crit Rev Biotechnol. Vol 22, p. 33 - Beg QK, Kapoor M, Mahajan L & Hoondal GS. 2001, Appl Microbiol Biotechnol. Vol. 56, p. 326).

Xylanases belonging to families other than glycoside hydrolase Families 10 and 11 have been recently described (see e.g. http://afmb.cnrs-mrs.fr/CAZY/ with the EC code for xylanase 3.1.2.8. for an overview). Among these, one example is the xylanase from *Pseudoalteromonas haloplanktis* TAH3a belonging to glycoside hydrolase Family 8 which has been characterized and for which the corresponding gene has been cloned (Collins T. *et al*. 2002, J. Biol. Chem. Vol. 277, p. 35133; Collins T. *et al*. 2003, J. Mol. Biol. Vol 328, P. 419; Van Petegem F. *et al.* 2003, J. Biol. Chem. Vol 278, p. 7531). This enzyme is a typical psychrophilic enzyme and presents a high catalytic activity at low temperatures. It is not homologous to family 10 or 11 xylanases, but has 20 to 30% identity with glycoside hydrolase Family 8 members (formerly Family D), a family that comprises mainly endoglucanases, but also lichenases and chitosanases. Furthermore, a FingerPRINTScan against PRINTS using the InterPro Scan search program (Zdobnov and Apweiler, 2001, Bioinformatics Vol. 17, p. 847) indicated that the isolated sequence contained the glycosyl hydrolase Family 8 fingerprint. In addition, the isolated sequence contains Family 8 residues that are strictly conserved in the 53 Family 8 enzymes analyzed thus far.

In contrast to most EXs10 and EXs11, this Family 8 xylanase (EXs8) has both a high pI and a high molecular weight. Structural and catalytic properties are different from those of both EXs10 and EXs11. The EXs8 xylanase presents a distorted (α/α)₆ barrel fold with 13 α-helices and 13 β-strands and belongs to clan GH-M (Van Petegem *et al.* 2003, J. Biol. Chem. Vol. 278(9), p7531-9). This enzyme has no endoglucanase, chitosanase or licheninase activity and appears to be functionally similar to EXs11, being more active on long chain xylo-oligosaccharides.

In contrast to other characterized EXs10 and EXs11 that retain the anomeric configuration, Family 8 glycoside hydrolases (Fierobe *et al*., 1993. Eur. J. Biochem. Vol. 217, p557; http://afmb.cnrs-mrs.fr/CAZY/) tend to hydrolyse the substrate with inversion of the anomeric configuration of the glycosidic oxygen. This has been shown for the psychrophilic xylanase from *Pseudoalteromonas haloplanktis* (Collins, T. *et al.* 2002. J. Biol. Chem. Vol. 277, p. 35133). In addition, the catalytic residues involved in hydrolysis are believed to be a glutamate and an aspartate.

A variety of methods exist for evaluating the xylanase activity in an enzyme preparation. Examples of such methods for xylanase activity determination include: the measurement of the release of reducing sugars from xylan (Miller G.L. 1959, Anal. Chem. Vol. 31, p. 426), or the measurement of the release of coloured compounds from modified substrates (for examples AzoWAX or Xylazyme AX from Megazyme). However, no direct correlation has been shown between the xylanolytic activity found in various enzyme preparations and the effect in a particular application. For example in baking, dose-related results can be observed to a certain extent for a single enzyme, but the same dosage of two xylanases of different origins does not give the same result in the dough or in the bread. Several reasons could explain this: differences in substrate specificity, differences in temperature and pH optimum, differences in catalytic efficiency, ...

It is of great interest to develop novel enzyme preparations, such as, but not restricted to ingredients for bread improver compositions or agents with new or improved properties. One of these properties could be that the xylanase content is as low as possible (in terms of weight of enzymes needed to obtain a particular result).

### Aims of the invention

The present invention aims to provide novel enzymes and enzyme preparations with xylanolytic activity.

The present invention further aims to provide amino acid and nucleotide sequences encoding such enzymes and their variants.

Another aim of the invention is to provide enzymes and enzyme preparations for use in agrofood, feed and many other technical processes.

Yet another aim of the present invention is to provide enzymes and enzyme preparations with xylanolytic activity of which the amount needed to observe a specific effect in a particular application is far lower than for any conventional commercial enzymes.

Still another aim of the present invention is to provide enzymes and enzyme preparations with xylanolytic activity, which are remarkably more stable than enzymes known in the art, and which preferably are more effective at the same time (reflected by a significantly lower amount needed to obtain a given effect).

### Summary of the invention

The present invention is directed to novel xylanolytic enzymes that preferably belong to glycoside hydrolase Family 8 and preferably are psychrophilic enzymes, most preferably psychrophilic enzymes that hydrolyse with inversion of the anomeric configuration, with the proviso that said xylanolytic enzyme is not encoded by SEQ ID NO: 1.

Advantageously, the enzymes of the present invention increase the loaf volume of a baked product by at least 10% when applied in a concentration of 1500 to 6000 xylanase units/100 kg flour and/or increase the width of cut on the surface of a baked product when applied in a concentration of 1500 to 6000 xylanase units/100 kg flour. The enzymes of the present invention may be advantageously used in frozen doughs and/or in applications that involve low temperature proofing.

In a preferred embodiment of the invention, the enzymes comprise an amino acid sequence, or consist of an amino acid sequence, corresponding to any of SEQ ID NOs: 4, 6, 8, 10, 12, 14 or 16. These sequences contain 426 amino acids (aa), of which the first 21 constitute a signal peptide.

In a preferred embodiment, said enzymes - identified by one of the above SEQ ID NOs - belong to glycoside hydrolase Family 8 and are preferably psychrophilic enzymes.

Another aspect of the invention concerns the mature polypeptides of any of the above, corresponding to aa 22-426 of any of the above sequences. A preferred embodiment of the invention concerns SEQ ID NOs: 21, 23, 25, 27, 29, 31 and 33 and SEQ ID NOs: 20, 22, 24, 26, 28, 30 and 32, the corresponding nucleotide sequences encoding such mature polypeptides.

The enzymes of the invention may also have an amino acid sequence that differs in less than 10, 9, 8, 7, 6, 5, 4, 3, 2 or 1 aa from any of the above (mature protein or propeptide), or have an amino acid sequence that has more than 50%, 70%, more preferably more than 80%, 85%, 90%, most preferably more than 95%, 97%, 98% or even more than 99% sequence identity with any of SEQ ID NOs: 2, 4, 6, 8, 10, 12, 14, 16, 21, 23, 25, 27, 29, 31 or 33.

The enzymes of the invention preferably are isolated from any of the following bacterial strains: TAH 2a, TAH 4a, TAH 7a, Sp.10.1, Sp.10.2, Sp.11.2 or Sp.23.2. These strains concern another aspect of the invention.

Another aspect of the invention concerns nucleotide sequences that encode any of the above xylanolytic enzymes.

A preferred such nucleotide is one that comprises SEQ ID NO: 19 and codes for a TAH 2a, TAH 4a, TAH 7a, Sp.10.1, Sp.10.2 or Sp.11.2 xylanase as identified by any of SEQ ID NOs: 4, 6, 8, 10, 12, 14, 21, 23, 25, 27, 29 or 31.

Other preferred nucleotide sequences are those coding for one of the above-identified preproteins or mature polypeptides. Examples of such nucleotide sequences include SEQ ID NOs: 3, 5, 7, 9, 11, 13, 15, 20, 22, 24, 26, 28, 30 and 32.

A further aspect of the present invention concerns recombinant nucleotide sequences that comprise one or more of the above nucleotide sequences, operably linked to one or more adjacent regulatory sequence(s). Another aspect of the invention concerns vectors harboring the latter and host cells transformed therewith.

A preferred embodiment of the invention is directed to a plasmid incorporated in *Escherichia coli* and having a deposit selected from the group consisting of LMBP 4860, LMBP 4861, LMBP 4862, LMBP 4863, LMBP 4864, LMBP 4865 and LMBP 4866.

The enzymes of the invention may be extra-cellularly expressed or may be intracellularly expressed. It is possible to use directly cells or cell cultures that express the enzymes according to the invention and/or to use the supernatant only of cell cultures in which the enzymes are secreted. Alternatively, cell extracts and/or cell-free extracts may be used as source of the enzymes of the invention. Finally, the enzymes may be used after being purified, id est in more or less pure form.

The xylanases of the invention are highly suitable for use in applications that involve the degradation of plant cell wall components. They are for instance advantageously used in processes for decomposing plants and fruits, preferably fruit, legume juice, beer, paper, starch, gluten or vegetable oil preparation processes. Other applications include their use in fruit, vegetable and plant processing, wine making or brewing, coffee processing, the processing of paper or textile, in bioconversion processes, in processes for decomposing wastes, preferably for decomposing agricultural wastes or wastes from paper mills, in starch-gluten separation processes, in feed preparations, in baking, milling, pastry and confectionery processes.

The enzymes of the invention are particularly suitable for use in food applications.

For some applications it is advantageous to fix the above described cells, components of the above cell extracts and/or one or more purified enzymes with xylanolytic activity according to the invention to a solid support.

The enzymes of the invention may be used in a bread improver or bread improving composition. Apart from one or more xylanolytic enzymes according to the invention, said improver or improving composition may further contain at least one other bread-improving agent selected from the list consisting of other enzymes, emulsifiers, oxidants, milk powder, fats, sugars, amino acids, salts and proteins (gluten, cellulose binding sites) or a mixture thereof. Said other enzyme preferably is selected from the list consisting of alpha-amylases, beta-amylases, maltogenic amylases, other xylanases, proteases, glucose oxidase, oxido-reductases, glucanases, cellulases, transglutaminases, isomerases, lipases, phospholipases, pectinases or a mixture thereof. Said alpha-amylase preferably is an alpha-amylase obtained from Aspergillus *oryzae*.

Preferably the above-described bread improving composition is added during the mixing of the dough.

The enzymes of the invention may be combined with any other enzyme or ingredient and may be used in the form of for instance a dry powder or a granulate, in particular a non-dusting granulate. Alternatively, they may be used in the form of a liquid, preferably with one or more stabilizer(s) such as polyols, sugars, organic acids or sugar alcohols.

A last aspect of the invention concerns compositions comprising at least one, preferably at least two or three of the enzymes of the invention, possibly combined with a *Pseudoalteromonas haloplanktis* TAH 3a xylanase, encoded for instance by SEQ ID NO:1.

### Brief description of the drawings

Figures 1a and b represent, respectively, the nucleotide (a) and the amino acid (b) sequences of the *Pseudoalteromonas haloplanktis* TAH 3a xylanase.

Figure 2 represents the thermodependendency of the xylanolytic activity of TAH 2a (circles, continuous line), TAH 4a (squares, dashed line) and TAH 7a (triangles, short dashed lines).

Figures 3a and b represent, respectively, the nucleotide (a) and the amino acid (b) sequences of the strain TAH 2a xylanase.

Figures 4a and b represent, respectively, the nucleotide (a) and the amino acid (b) sequences of the strain TAH 4a xylanase.

Figures 5a and b represent, respectively, the nucleotide (a) and the amino acid (b) sequences of the strain TAH 7a xylanase.

Figures 6a and b represent, respectively, the nucleotide (a) and the amino acid (b) sequences of the strain Sp.10.1 xylanase.

Figures 7a and b represent, respectively, the nucleotide (a) and the amino acid (b) sequences of the strain Sp.10.2 xylanase.

Figures 8a and b represent, respectively, the nucleotide (a) and the amino acid (b) sequences of the strain Sp 11.2 xylanase.

Figures 9a and b represent, respectively, the nucleotide (a) and the amino acid (b) sequences of the strain Sp.23.2 xylanase.

Figure 10 shows an alignment of the nucleotide sequences of the xylanases from strains TAH 3a, TAH 2a, TAH 4a, TAH 7a, Sp.10.1., Sp.10.2., Sp.11.2. & Sp.23.2. The symbol * under the alignment shows identical nucleotides in all sequences listed.

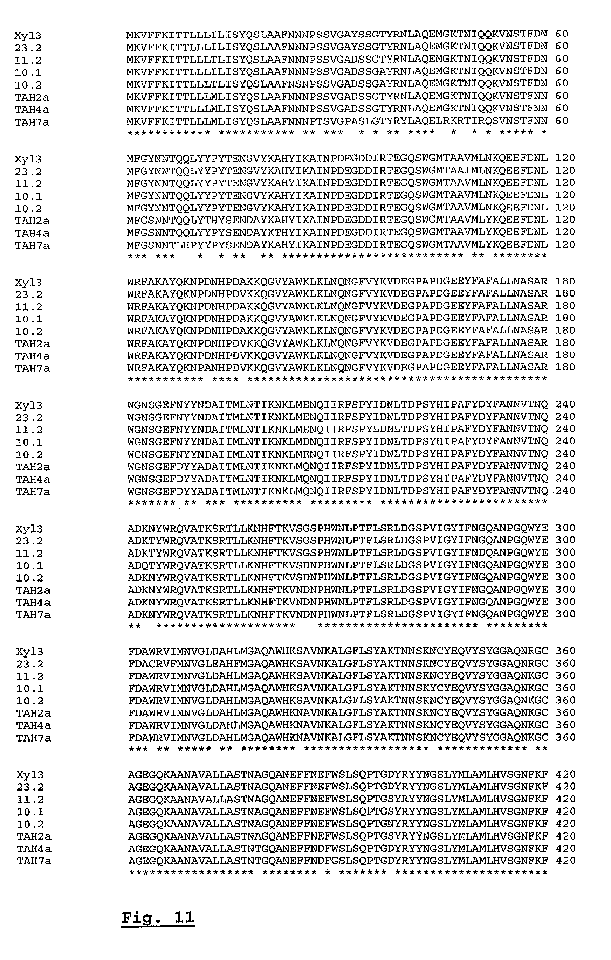

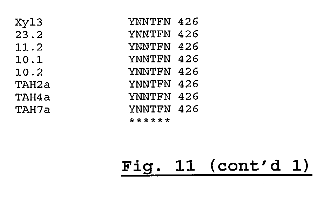

Figure 11 shows an alignment of the amino acid sequences of the xylanases from strains TAH 3a (xyl3), TAH 2a, TAH 4a, TAH 7a, Sp.10.1., Sp.10.2., Sp.11.2. & Sp.23.2. The symbol * under the alignment shows identical amino acids in all sequences listed.

Figure 12 shows the results of an SDS polyacrylamide gel electrophoresis with Sp.11.2, Sp.23.2, TAH 2a and TAH 7a purified xylanases.

Figure 13 shows the relationship between the relative amounts of protein (xylanase) and the performance in baking of different xylanases according to the invention.

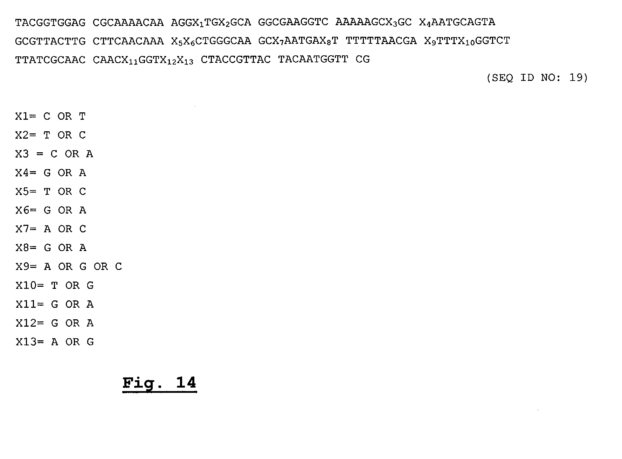

Figure 14 shows a preferred nucleotide sequence: SEQ ID NO: 19.

The invention will be described in further details in the following examples and embodiments by reference to the enclosed drawings. Particular embodiments and examples are not in any way intended to limit the scope of the invention as claimed.

### Detailed description of the invention

### A. Isolation of the enzyme producing microorganisms according to the invention

A first aspect of the invention is related to the isolation of uncharacterized microorganisms able to produce xylanases with new properties.

Advantageously, these microorganisms are psychrophilic microorganisms isolated from environmental samples collected in the Antarctic and Arctic environments. Methods are described in the literature that relate to the isolation of psychrophilic microorganisms from such samples (Collins T. *et al*., 2002, J. Biol. Chem. Vol. 277, p. 35133). Typically these methods consist of spread-plating appropriate dilutions of the samples onto solid media of various compositions. Incubations are generally performed at low temperatures (about 4 to about 20°C), to allow the growth of the psychrophilic microorganisms.

According to a preferred embodiment of the present invention, the solid medium contains a substrate for xylanase such as a hemicellulose-containing material or xylan.

According to a more preferred embodiment of the invention, the solid medium contains a derivatized substrate for the xylanase such as for example RBB-xylan (Remazol Brilliant Blue xylan, Sigma). This substrate allows a better, generally more sensitive, screening of the positive xylanase-producing microorganisms.

It is obvious that techniques other than those described here, exist for the screening of xylanase-positive microorganisms. One such technique involves systematic liquid culturing of microorganisms using, eventually, media that contain an inducer for the xylanase such as, but not restricted to, xylan, wheat bran, etc., followed by enzymatic analysis of the culture supernatant and/or of a cell extract.

According to another embodiment of the present invention, the isolated xylanase-positive microorganisms may or may not be identified at the genus or the species level. Techniques such as fatty acid analysis or 16S rDNA sequence analysis are examples of well known identification techniques.

The present invention is more in particular related to isolated strains TAH 2a, TAH 4a, TAH 7a, Sp10.1, Sp10.2, Sp11.2 and Sp23.2.

TAH2a, TAH4a and TAH7a are bacterial strains, more specifically bacterial strains from the genus *Pseudoalteromonas*.

### B. Isolation of xylanase encoding genes

The present invention relates to the isolation of xylanase encoding genes from the above mentioned strains.

It is well know to persons skilled in the art that several techniques may be used to isolate a particular gene from microorganisms. Examples of such techniques include the construction of a gene library from the genomic DNA of the strain in a suitable vector, followed by screening of this library by direct expression of the gene of interest, or by hybridization with a presumed closely related gene or with oligonucleotides designed from reverse transcription of the partial or complete amino acid sequence of the said enzyme.

Other techniques include the amplification of the gene by molecular techniques, for instance PCR techniques, using degenerate or non-degenerate oligonucleotide primers that are designed from a presumed closely related gene.

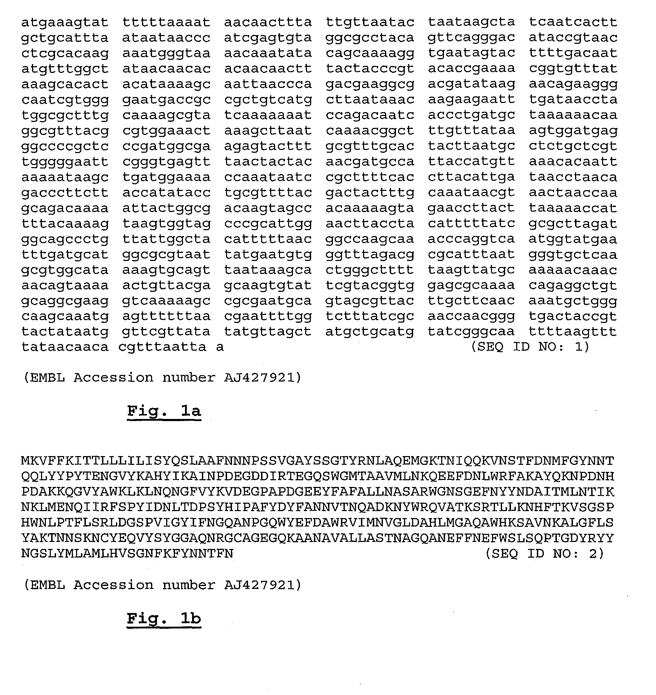

According to a preferred embodiment of the present invention, the starting sequence used to design such oligonucleotide primers is the sequence of the xylanase gene isolated from *Pseudoalteromonas haloplanktis* TAH 3a (EMBL Accession number AJ427921, sequence enclosed by reference thereto, see also Figure 1a or SEQ ID NO 1).

An aspect of the invention is related to isolated nucleotide sequences SEQ ID NOs: 3, 5, 7, 9, 11, 13, 15, 20, 22, 24, 26, 28, 30, 32, or variants thereof that encode an enzyme according to the invention.

"Variant", in the present context, is defined as an isolated nucleotide sequence wherein one, or several, nucleotides have been replaced by other nucleotide(s) or wherein one, or several, nucleotides have been added and/or deleted while the encoding enzyme still presents xylanolytic activity.

According to a preferred embodiment of the present invention, the nucleotide sequence of the variant presents more than 50%, 60%, 70%, 80%, 85%, 90%, 95%, preferably more than 97%, 98% or even more than 99% sequence identity to the isolated sequences. According to a more preferred embodiment of the present invention, the nucleotide sequence of the variant presents less than 20, 19, 18, 17, 16, 15, 14, 13, 12, 11, 10, 9, 8, 7, 6, 5, 4, 3, 2 or 1 modified nucleotide(s) compared to SEQ ID NOs: 3, 5, 7, 9, 11, 13, 15, 20, 22, 24, 26, 28, 30 or 32.

### C. Characterization of the xylanases of the invention

Another aspect of the present invention is related to enzymes with xylanolytic activity encoded by any of the nucleotide sequences described above.

According to a preferred embodiment of the present invention, the amino acid sequence of such an enzyme is selected from the group consisting of SEQ ID NOs: 4, 6, 8, 10, 12, 14, 16, 21, 23, 25, 27, 29, 31, 33, or a variant thereof.

In the present context, "variant" is defined as an isolated amino acid sequence wherein one, or several, amino acids have been substituted by one, or several, others, or as an isolated amino acid sequence wherein one, or several, amino acids have been added and/or deleted while preserving all or most of the xylanase activity. Variant enzymes preferably retain at least 80%, more preferably at least 90% and most preferably at least 95% or even 99% of the xylanolytic activity of any of the enzymes characterized by SEQ ID NO: 4, 6, 8, 10, 12, 14, 16, 21, 23, 25, 27, 29, 31 or 33.

According to a preferred embodiment of the present invention, the amino acid sequence of the variant presents more than 50%, 70%, more preferably more than 80%, 85%, 90%, most preferably more than 95%, 97%, 98% or even more than 99% sequence identity with any of SEQ ID NOs: 4, 6, 8, 10, 12, 14, 16, 21, 23, 25, 27, 29, 31 or 33. According to a more preferred embodiment of the present invention, the amino acid sequence of the variant presents less than 4, 3, 2 or 1 modified amino acid(s) compared to any of SEQ ID NOs: 4, 6, 8, 10, 12 14, 16, 21, 23, 25, 27, 29, 31 or 33.

It is well known that most secreted enzymes, such as the enzymes of the present invention, possess a small amino acid sequence at their N terminus that drives the enzyme out of the cell. It is therefore an objective of the present invention to also consider the mature portions of the enzymes as an integral part of the present invention (see e.g. SEQ ID NOs: 21, 23, 25, 27, 29, 31 and 33). However, variants containing modifications in the signal sequence could advantageously be used, as these could eventually be better expressed and/or be better secreted in a recombinant host.

The mature protein starts at amino aid position No. 22 for the enzymes identified by any of SEQ ID NOs: 2, 4, 6, 8, 10, 12, 14 or 16.

Another means of characterizing proteins and/or enzymes is to study their identity or their similarity with already known proteins. A first approach could be to use the Blast program that compares a defined sequence to all available published sequences. This program can be used on-line at the following URL (http://www.ncbi.nlm.nih.gov/blast/). To refine and precise the percentage of identity/similarity between two or more proteins the programs Clustal (http://www.ebi.ac.uk/clustalw/) or Align (http://www2.igh.cnrs.fr/bin/align-guess.cgi) may advantageously be used.

A further means of characterizing new proteins and/or enzymes is to analyze their three-dimensional structures. In some cases, this three-dimensional structure is not known. However, by comparing with other available structures of related proteins, this three-dimensional structure could be deduced by a person skilled in the art by using for instance specialized software. Furthermore, for very closely related proteins, e.g. proteins with more than 90% identity/similarity, the changes in properties caused by a particular modification in the amino acid sequence (e.g. amino acid substitution) could be predicted. Such changes could be e.g. substrate binding, protein stabilisation, ...

Another aspect of the present invention concerns the characterization of the xylanases of the invention by their biochemical properties such as their molecular weight, their temperature optimum or pH optimum. Indeed the enzymes described in the present invention, by means of the examples below, have a relatively low temperature optimum activity. Furthermore, they retain a high catalytic efficiency at low temperature and are therefore considered as psychrophilic xylanases. One way of defining a psychrophilic enzyme is to consider that it is an enzyme that retains at least 40% of its maximal activity at 5°C. Other properties may also be used to define psychrophilic enzymes (see e.g. Georlette D. *et al*., 2004, FEMS Microbiol. Rev. Vol 28, p. 25 ; D'Amico S. 2002, Philos Trans R Soc Lond B Biol Sci. Vol. 357, p.917).

However, it can not be excluded that there exist enzymes with the sequence characteristics described above that have different biochemical properties.

### D. Expression and/or purification of the enzymes

Another aspect of the present invention is related to a recombinant nucleotide sequence comprised of one or more adjacent regulatory sequence(s) operably linked to one or more of the nucleotide sequence(s) of the invention as described above.

Said adjacent regulatory sequences may originate from homologous and/or from heterologous microorganisms.

These adjacent regulatory sequences are specific sequences such as but not restricted to promoters, regulators, secretion signals and/or terminators.

Another aspect of the invention is related to a vector containing one or more nucleotide sequences of the invention, possibly operably linked to one or more adjacent regulatory sequence(s) which originate from homologous or/and from heterologous microorganisms.

In the present context, "vector" is defined as any biochemical construct which may be used for the introduction of a nucleotide sequence (by transduction, transfection, transformation, infection, conjugation, etc.) into a cell. Advantageously, the vector described in the invention is selected from the group consisting of plasmids, viruses, phagemids, chromosomes, transposons, liposomes, cationic vesicles, and/or a mixture thereof. Said vector may already contain one or more of the above-described adjacent regulatory sequence(s) (allowing its expression and its transcription into a corresponding peptide by said microorganism). Preferably, said vector is a plasmid incorporated in *E. coli* and having a deposit number selected from the group consisting of numbers LMBP 4860, LMBP 4861, LMBP 4862, LMBP 4863, LMBP 4864, LMBP 4865 and LMBP 4866.

The present invention is also related to a host cell, preferably a recombinant host cell, "transformed" with one or more of the nucleotide sequences and/or vectors according to the invention that have been described above.

In the present context, "host cell "transformed by one or more of the nucleotide sequences and/or vectors according to the invention" is defined as a cell having incorporated said nucleotide sequence(s) and/or said vector. The transformed host cell may be a cell in which said vector and/or said nucleotide sequence(s) are introduced by means of genetic transformation, preferably by means of homologous recombination, or by another well known method to generate/create a recombinant organism.

A "host cell" may be a cell that, prior to transformation, did not naturally (originally) contain said nucleotide sequence(s) and/or said vector. Alternatively, the "host cell" may also be the original cell containing already one of the nucleotide sequences of the present invention, and genetically modified (recombinant host cell) to overexpress, or express more efficiently, said enzyme (better pH or temperature profile, higher extracellular expression, ...).

Preferably, said host cell is capable of overexpressing (higher expression than the expression observed in the original or wild-type microorganism) said nucleotide sequence(s) and/or said vector, advantageously allowing a high production of an amino acid sequence encoded by said nucleotide sequence and/or said vector. The isolated and purified nucleotide sequence(s) described in the present invention may be integrated into the genome of the selected host cell and/or may be present on an episomal vector in said host cell.

Advantageously, the recombinant host cell of the invention is selected from the microbial world, preferably from bacteria or fungi, including yeast.

More preferably the recombinant host cell belongs to the genus *Bacillus.*

Preferably, said recombinant host cell is modified to obtain an expression of the xylanase enzyme at high levels. This may be obtained by the use of adjacent regulatory sequences capable of directing the overexpression of one or more of the nucleotide sequences of the invention in the recombinant host cell, or by increasing the number of nucleotide copies of the sequences according to the invention.

Above, some of the conditions (culture media, temperature and pH conditions, etc.) have been described that preferably are applied for culturing of the host cell selected for the expression of the xylanase of the invention. For this purpose, the original production species and/or a suitable host cell, transformed with a DNA construct designed to express the said enzyme, are present in or on a suitable growth medium and/or expression medium.

According to the present invention, said protein with xylanolytic activity may be obtained by first culturing the strain in/on a medium suitable for expressing the xylanase, followed by one or several purification steps, such as, but not limited to, centrifugation, cell disruption, microfiltration, ultrafiltration, precipitation, liquid chromatography, freeze-drying, etc. All these techniques are described in the scientific literature and are well known to persons skilled in the art.

Accordingly, the enzyme with xylanolytic activity which is produced according to the present invention, can be used directly for one of the purposes of the present invention and/or, alternatively, may undergo one or more purification and/or (further) culturing steps as described above. In a particular embodiment of the invention, the enzyme(s) of the invention may be used in pure form.

Preparation methods for the enzyme of the present invention include, among others, the preparation and/or purification from cultures of microorganisms, recombinant or not, in shake flasks or in fermentors, and the preparation or purification from immobilized cultures as well as the extraction and/or purification from living cells (plants, ...).

### E. Application of the enzyme

The xylanase of the invention may be used in different types of applications.

The enzymes with xylanolytic activity described in the present invention, purified or not purified, are particularly suited as bread improving agents. Bread improving agents or compositions are products that are able to improve and/or increase texture, flavour, anti-staling effects, softness, crumb softness upon storage, freshness, dough machinability and/or volume of a dough and/or a final baked product. Preferably, said enzymes with xylanolytic activity improve the dough handling and/or increase the specific volume of the final baked product.

The term "baked product" includes any product prepared from a dough and obtained after baking of the dough, and includes in particular yeast raised baked products. Dough is obtained from any type of flour or meal (e.g. based on wheat, rye, barley, oat, or maize). Preferably, dough is prepared with wheat and/or with mixes including wheat.

It is one of the aspects of the present invention to show that the enzymes with xylanolytic activity described in the present invention could advantageously be used in a bread improver formula or bread improving composition.

In another embodiment of the present invention, these enzymes with xylanolytic activity could advantageously be selected from glycoside hydrolase Family 8 enzymes.

In yet another embodiment of the present invention, these enzymes with xylanolytic activity could advantageously be psychrophilic enzymes.

In a further embodiment of the present invention, it is shown that said enzymes increase the loaf volume of a baked product.

In a further embodiment of the present invention, it is shown that the amount of said enzymes, needed to obtain a particular result in baking, is much lower than the amount generally used/needed when working with commercially available enzymes. Such amount is further referred to as the amount sufficient to obtain the desired result and/or as the effective amount.

In a further aspect, the present invention relates to the additive effect of said enzymes with xylanolytic activity with other enzymes, in particular with an alpha-amylase, preferably an alpha-amylase from *Aspergillus oryzae*. Said enzymes with xylanolytic activity may be used in combination with other bread improving agents, such as, but not limited to enzymes, emulsifiers, oxidants, milk powder, fats, sugars, amino acids, salts, proteins (gluten, cellulose binding sites), such improving agents being well known to persons skilled in the art. Examples of such enzymes include, but are not restricted to, alpha-amylases, beta-amylases, maltogenic amylases, xylanases, proteases, glucose oxidases, oxido-reductases, glucanases, cellulases, transglutaminases, isomerases, lipases, phospholipases, pectinases, etc.

According to the present invention, the enzymes with xylanolytic activity, purified or not, show hydrolytic activities in the presence of cell wall components. Particularly, said enzymes degrade the wheat cell wall components. Said enzymes may thus be advantageously used in the separation of components of plant cell materials, such as cereal components. Particularly, said enzymes may be used to improve the separation of wheat into gluten and starch by the so-called batter process.

According to the present invention, the enzymes with xylanolytic activity, purified or not, may be used in food processing. They may be used in fruit, vegetable and plant processing. They may be used in wine making and brewing as well as in coffee processing.

Another application of said enzymes resides in feed, where they may be used to improve the growth rate or the feed conversion ratio of animals such as poultry, swine or ruminants.

Yet other applications are the use of the xylanases of the invention in the pulp and paper industry (bio-mechanical pulping, bio-modification of fibers, bio-de-inking,...), in textile processing, in bioremediation, in bioconversion processes, ....

The enzymes with xylanolytic activity according to the present invention may be used under several forms. Cells expressing the enzyme, such as yeast, fungi, Archeal bacteria or bacteria, may be used directly in the process. Said enzymes may further be used as a cell extract, a cell-free extract (i.e. portions of the host cell that have been submitted to one or more disruption, centrifugation, extraction and/or other purification steps) or as a purified protein. Any of the above-described forms may be used in combination with one or more enzyme(s) under any of the above-described forms. These whole cells, cell extracts, cell-free extracts and/or purified enzymes may be immobilized by any conventional means on a solid support to allow protection of the enzyme, continuous hydrolysis of the substrate and/or recycling of the enzyme preparation. Said cells, cell extracts (including crude and partially purified extracts), cell-free extracts and/or enzymes may be mixed with different ingredients, and be used e.g. in the form of a dry powder or a granulate, in particular a non-dusting granulate, in the form of a liquid, for example with stabilisers such as polyols, sugars, organic acids or sugar alcohols according to established methods.

### Examples

### Example 1: Isolation and characterization of xylanase producing microorganisms

The xylanase producing microorganisms were isolated from either wood samples collected in the vicinity of the abandoned French Antarctic station in Port Martin, Terre Adelie, Antarctica (66°40'S; 140°01'E) or from sea water samples collected in the vicinity of Ny-Alesund, Spitzberg, Svalbard, Norway. Screening for xylanase activity was carried out on marine agar (5 g/l tryptone (Difco), 1 g/l yeast extract (Difco), 33 g/l marine salts (Wiegandt), 18 g/l agar (Difco)) supplemented with 0.15% Remazol Brilliant Blue-xylan at 4°C. A clear halo appeared around the xylanase producing microorganisms, allowing the isolation of 7 strains. Those isolated from Antarctica were designated TAH 2a, TAH 4a, TAH 7a, and those isolated from Spitzberg were termed Sp.10.1, Sp.10.2, Sp.11.2 and Sp.23.2.

The cellular fatty acid composition of strains TAH 2a, TAH 4a and TAH 7a has been determined. These three strains belong most probably to the *Pseudoalteromonas sp.*

### Example 2: Production of wild-type xylanases

### Culture conditions

The isolated xylanase producing bacterial strains TAH 2a, TAH 4a, TAH 7a, Sp.10.1, Sp.10.2, Sp.11.2 and Sp.23.2 were cultivated in modified marine broth (5g/liter tryptone (Difco), 1g/liter yeast extract (Difco), 20g/liter marine salts (Wiegandt)) supplemented with 15g/liter birchwood xylan (Sigma) (in the case of TAH2a, TAH7a, Sp.10.1, Sp10.2, Sp.11.2 and Sp.23.2) or 15g/liter wheat hemicellulose (Beldem) (in the case of TAH 4a) for 72 hours at 4°C.

### Preparation of the enzymes

After centrifugation of the above cultures (see cultures conditions above) for 1h at 18,000 x g and 4°C, the supernatant was concentrated by precipitation with 80% ammonium sulphate and resuspended in 20mM MOPS (3-(N-Morpholino)propanesulfonic acid) at pH 8.0. This suspension was used in baking trials with TAH 4a, Sp.10.1, Sp.10.2, Sp.11.2 and Sp.23.2 xylanases. In the case of TAH 2a and TAH 7a, however, the resuspended samples were first dialysed overnight against 20mM MOPS containing 300mM NaCl and centrifuged at 18,000 x g for 1 hour. The soluble (supernatant) and insoluble fractions hence obtained were used in the baking trials for these samples (see example 3).

### Characterization of the enzymes

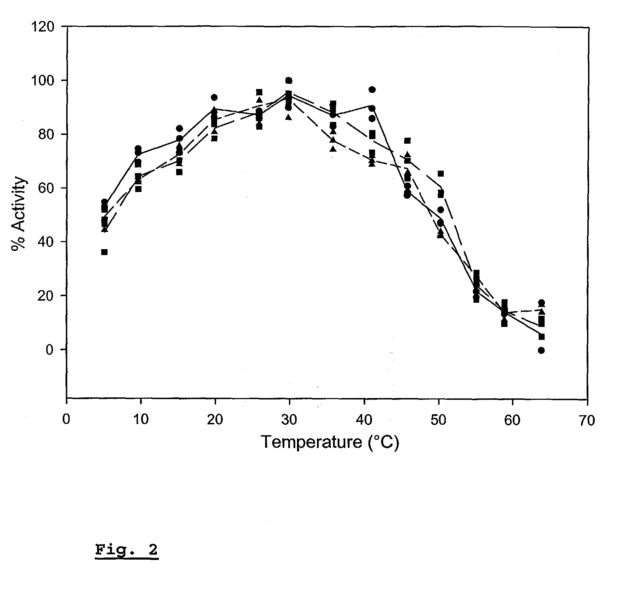

The activity of the xylanases obtained from strains TAH 2a, TAH 7a (soluble fractions as described above) and obtained from strain TAH 4a was determined at various temperatures. The results of this experiment are presented in Figure 2. The xylanases display maximum activity between 20°C and 40°C and retain more than 40% of their activity at 5°C.

### Example 3: Baking trials

Baking trials were performed to demonstrate the positive effect of the xylanases obtained in example 2 in baking. The positive effect was evaluated by the increase in bread volume as compared to a commercially available xylanase and as compared to a reference not containing any of these enzymes.

The xylanases of the invention were tested in Belgian hard rolls that are produced on a large scale every day in Belgium. The procedure described is well known to the craft baker and it is obvious to one skilled in the art that the same results may be obtained by using other protocols or equipment from other suppliers.

The ingredients used are listed in table 1 below:

**Table 1**

| Ingredients (g) | |
|---|---|
| Flour (Surbi -Molens van Deinze) | 1500 |
| Water | 915 |
| Fresh yeast (Bruggeman-Belgium) | 90 |
| Sodium chloride | 30 |
| Multec Data HP 20 (Beldem-Belgium) | 3.9 |
| Ascorbic acid | 0.12 |
| Xylanase | See table 2 |

The ingredients were mixed for 2 min at low and 8 min at high speed in a Diosna SP24 mixer. The final dough temperature as well as the resting and proofing temperature was 25°C. After resting for 15 min at 25°C, the dough was reworked manually and rested for another 10 min. Thereafter, 2 kg dough pieces were made up and proofed for 10 min. The 2-kg dough pieces were divided and made up using the Eberhardt Optimat. 66 g round dough pieces were obtained. After another 5 min resting time, the dough pieces were cut by pressing and submitted to a final proofing stage for 70 min. The dough pieces were baked at 230°C in a MIWE Condo™ oven with steam (Michael Wenz-Arnstein-Germany). The volume of 6 rolls was measured using the commonly used rapeseed displacement method.

The results of the baking trials are presented in table 2 below:

**Table 2**

| Xylanase sample | Xylanase units /100 kg flour(*) | Rolls volume increase (%) compared to control without xylanase |
|---|---|---|
| Belase B210 (Beldem-Belgium) | 1050 | 16 |
| TAH 2a soluble (**) | 6000 | 15 |
| TAH 2a insoluble | 6000 | 12 |
| TAH 4a | 6000 | 25 |
| TAH 7a soluble | 6000 | 12 |
| TAH 7a insoluble | 6000 | 14 |
| Sp.10.1. | 6000 | 15 |
| Sp.10.2. | 6000 | 14 |
| Sp.11.2. | 6000 | 13 |
| Sp.23.2. | 6000 | 20 |

| | | |
|---|---|---|
| (*)One unit of Belase B210 xylanase is defined as the amount of enzyme needed to release 1 µmole of reducing sugar (expressed as xylose) from birchwood xylan at 30°C and pH 4.5 (Nelson-Somogyi method). One unit of the other xylanases is defined as the amount of enzyme needed to release 1 µmole of reducing sugar (expressed as xylose) from birchwood xylan at 25°C and pH 6.5 (dinitrosalicylic acid method, Miller, G. 1959, Anal.Chem. Vol. 31, p. 426). | | |
| (**)With soluble is meant the (soluble) enzyme fraction present in the supernatant (see above). | | |

The above results show that all the xylanases tested had a positive effect on the volume of bread.

### Example 4: Cloning of the xylanase genes

### Bacterial strains and culture conditions

The strains TAH 2a, TAH 4a, TAH 7a, Sp10.1, Sp10.2, Sp11.2 and Sp23.2 described in example 1 were cultivated for 16 h in the modified Marine broth described above (see example 2).

### Preparation of genomic DNA

Genomic DNA was extracted and purified from 16 hour cultures, cultivated at 37°C in the medium described above, with the Wizard® Genomic DNA Purification kit (Promega).

### Cloning of the xylanase genes

The xylanase genes were PCR-amplified using VENT polymerase (Biolabs) with the sense primer: 
5'-GGGCATATGAAAGTATTTTTTAAAATAACAACTT-3'     SEQ ID NO:
17
 containing an NdeI site (underlined) and the antisense primer: 5'-GGGCTCGAGTTAATTAAACGTGTTGTTATAAAA-3' SEQ ID NO: 18 containing an *Xho* I site (underlined) and the stop codon (in italics). The concentration of the genomic DNA template used was optimized for each source isolate. After 3 min initial denaturation at 95 °C, 25 cycles of amplification were performed using a Progene apparatus (Techne Cambridge, UK). Each cycle included denaturation at 95 °C for 1 min, hybridization at 54 °C for 30 sec, and elongation at 72 °C for 1.5 min.

The fragment obtained from each isolate was cloned into a PCRScript Amp SK (+) vector (Stratagene), using the procedure recommended by the supplier, and used to transform XL10-Gold® Kan ultracompetent cells (Stratagene). Blue-white selection allowed selection of white colonies carrying the PCR-fragment. The sequence of the inserted fragment was determined with an ALF DNA sequencer (Pharmacia Biotech) using purified plasmid preparation (Nucleospin plasmid, Macherey-Nagel).

### Sequences of the xylanases genes

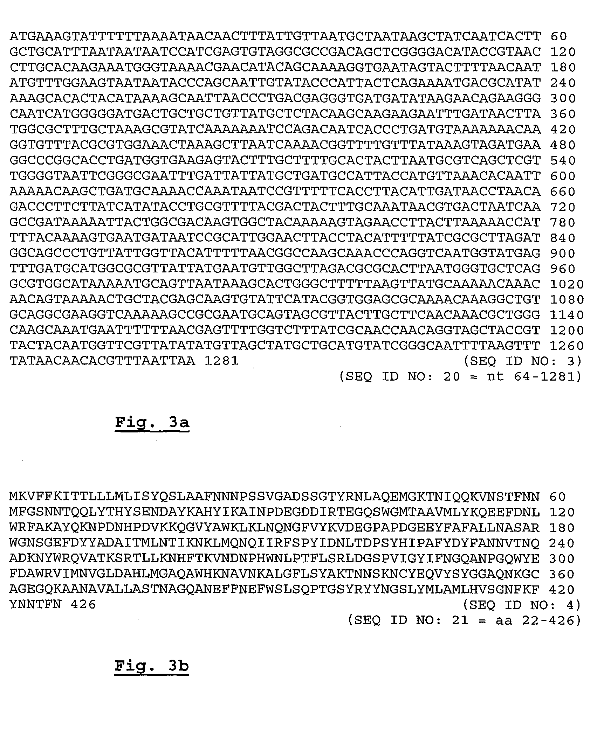

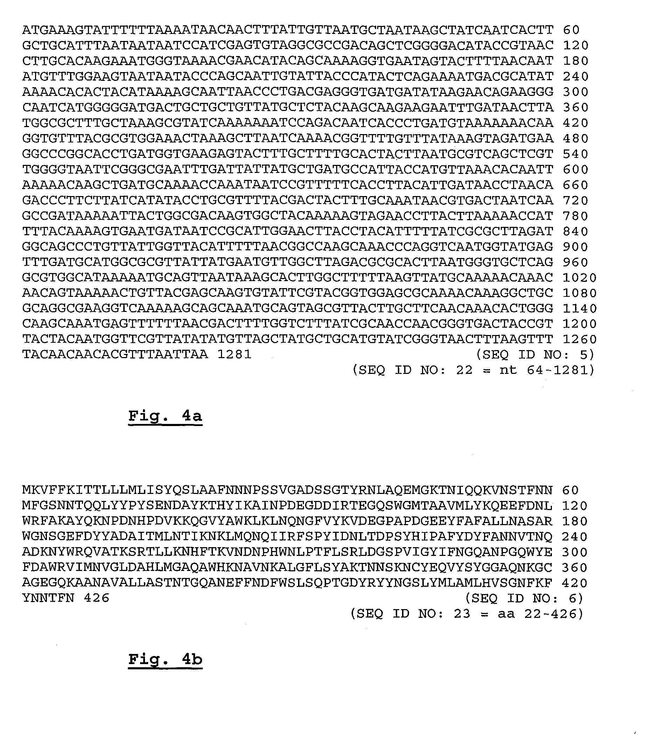

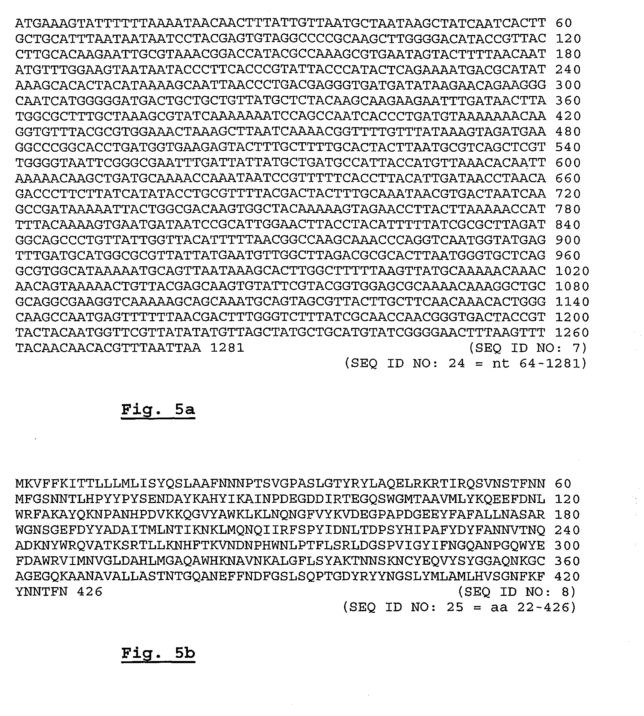

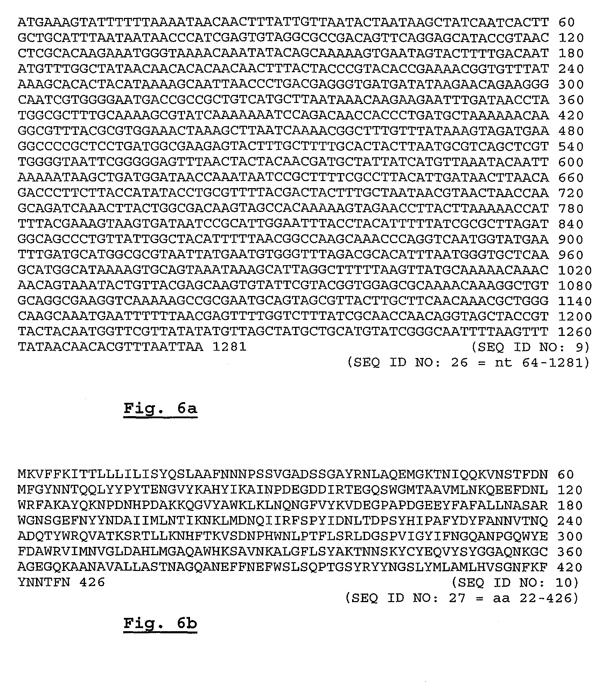

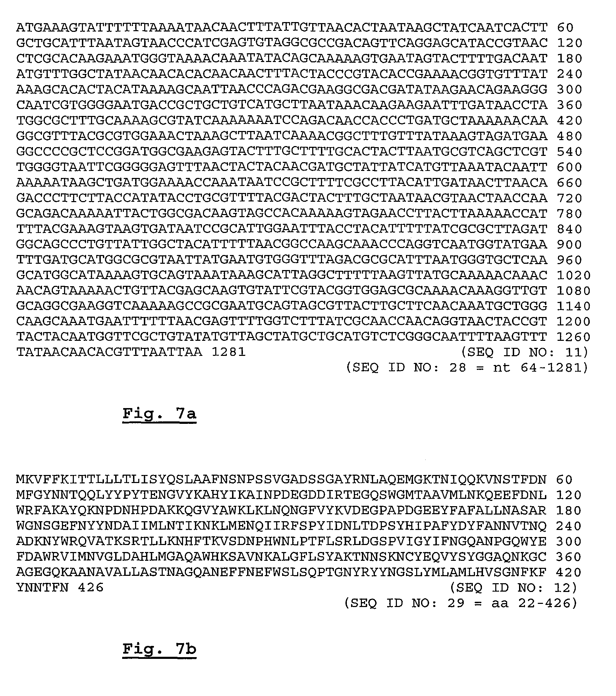

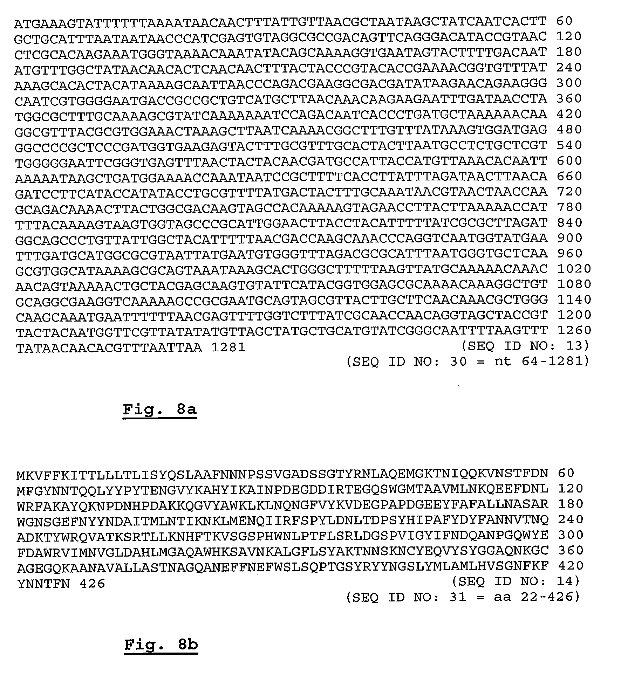

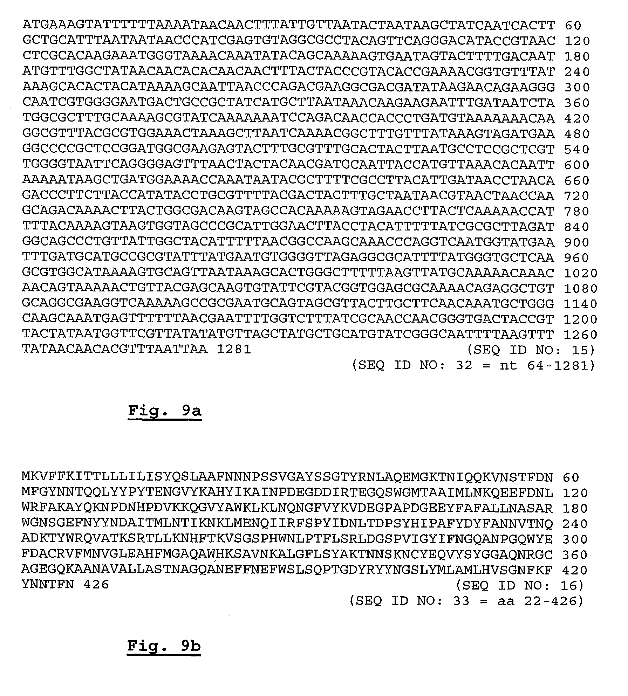

The sequences of the TAH 2a, TAH 4a, TAH 7a, Sp.10.1, Sp.10.2, Sp.11.2 and Sp.23.2 xylanase genes are shown in figures 3-10a (SEQ ID NOs: 3, 5, 7, 9, 11, 13, 15) and their corresponding amino acid sequences are shown in figure 3-10b (SEQ ID NOs: 4, 6, 8, 10, 12, 14, 16). The sequences encoding for the mature protein portions are given in SEQ ID NOS: 20, 22, 24, 26, 28, 30 and 32.

These sequences are highly similar to each other and are also homologous to the published sequence of the xylanase gene from *Pseudoalteromonas haloplanktis* TAH 3a (EMBL Accession number AJ427921). All enzymes described in the present invention belong to glycoside hydrolase Family 8.

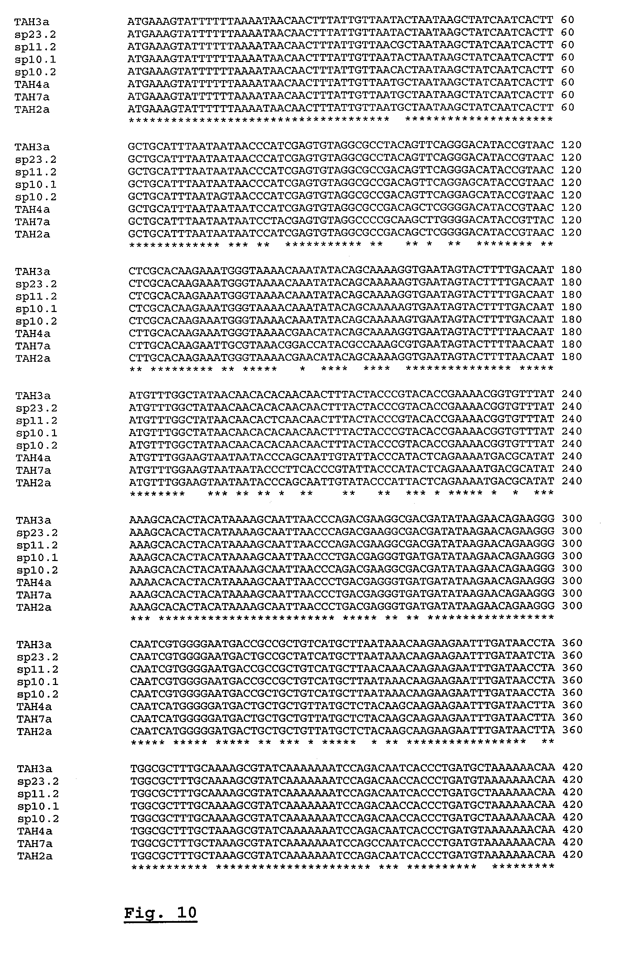

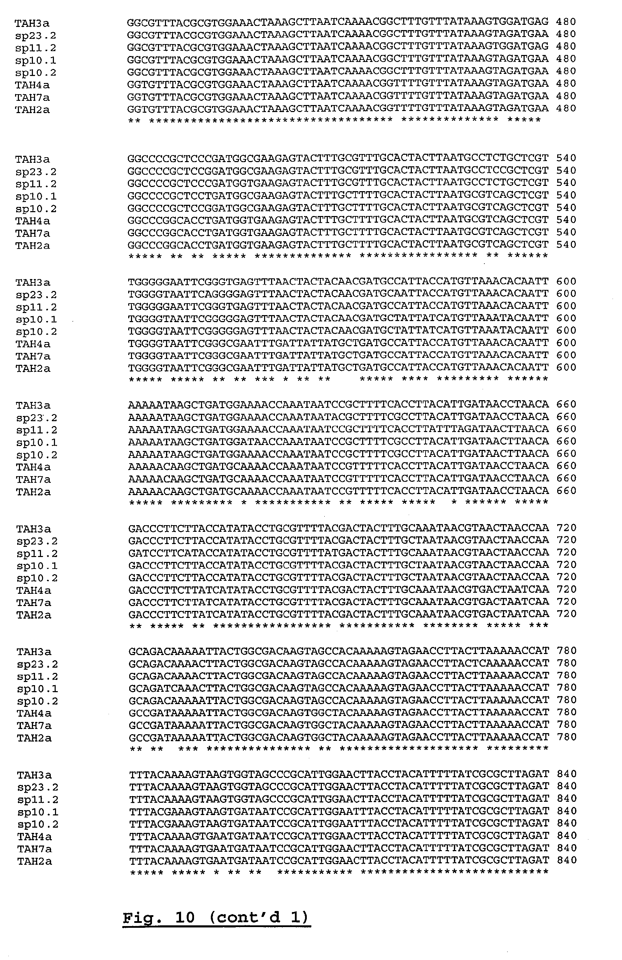

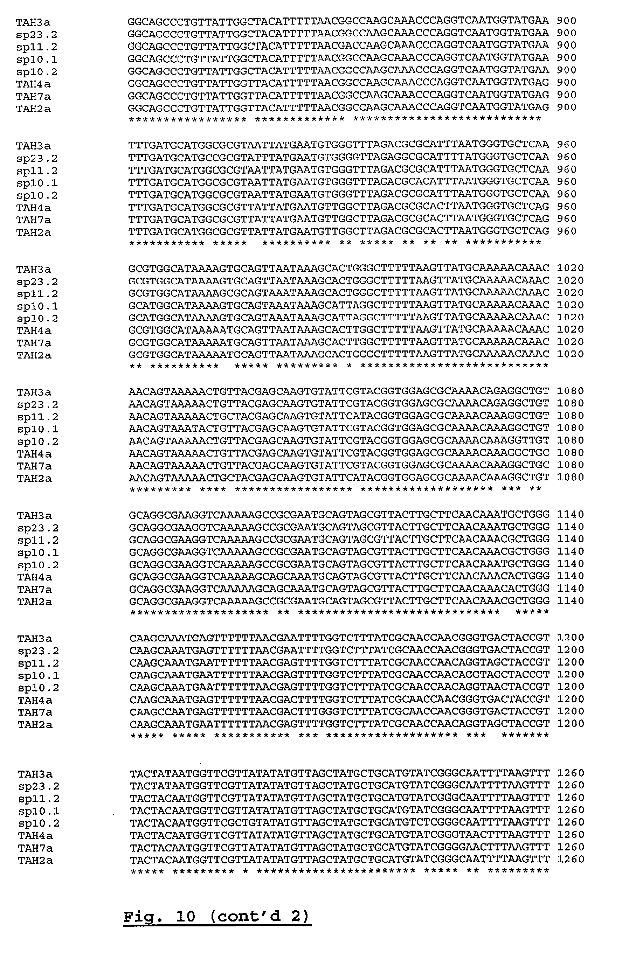

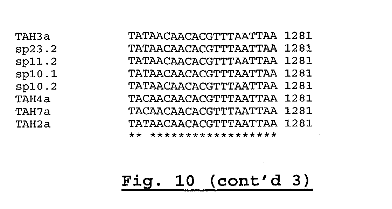

The result of a Clustalw multiple sequence alignment using the default parameters (http://www.ebi.ac.uk/clustalw/) are presented in figures 10 (nucleotide sequences) and 11 (amino acid sequences).

All new sequences differ by at least 20 nucleotides or by at least 4 amino acids from the known published sequence (see below).

In all of the above-identified amino acid sequences, the mature protein (enzyme) starts at amino acid position n°22.

The closest related amino acid sequences identified by a Blast search using the default parameters (http://www.ncbi.nlm.nih.gov/blast/) are:
1/ the already characterized Family 8 xylanase from *Pseudoalteromonas haloplanktis* TAH3a (between 92 and 98% sequence identity)
2/ an uncharacterized 1748 amino acids long protein with a C-terminal OMP (outer membrane protein) domain from *Cytophaga hutchinsonii* (33 to 34% identity on a 412 amino acids span)
3/ xylanase Y from *Bacillus halodurans* (31 to 32% identity)
4/ xylanase Y from Bacillus sp. KK1 (28 to 30% identity)

The amino acid differences between the published *Pseudoalteromonas haloplanktis* TAH3a xylanase sequence and the xylanase sequences of the present invention have been identified and are listed in Table 3 below. Their respective positions in the structure, as based on the published structure (Van Petegem F. *et al.*, 2003, J. Biol. Chem. Vol. 278, p. 7531) are also shown.

**Table 3**

| **TAH3a xylanase residue (mature sequence)** | **New xylanase involved** | **Mutation in the new xylanase (mature sequence)** | **Localization in the 3D structure** |
|---|---|---|---|
| Asn 4 | Sp. 10.2 | Ser 4 | Loop (N-term) |
| Ser 7 | TAH7a | Thr 7 | Loop |
| Ala 11 | TAH7a | Pro 11 | α helix like |
| Ser 14 | TAH7a | Leu 14 | α helix like |
| Asn 19 | TAH7a | Tyr 19 | Helix α1 |
| Met 24 | TAH7a | Leu 24 | Helix α1 |
| Gly 25 | TAH7a | Arg 25 | Helix α1 (C-term) |
| Thr 27 | TAH7a | Arg 27 | Loop |
| Asn 28 | TAH7a | Thr 28 | Loop |
| Gln 30 | TAH7a | Arg 30 | Helix α2 |
| Lys 32 | TAH7a | Ser 32 | Helix αa2 |
| Asp 38 | TAH2a, TAH4a, TAH7a | Asn 38 | Helix α2 |
| Tyr 43 | TAH2a, TAH4a, TAH7a | Ser 43 | Surface Loop between α2 and β1 |
| Gln 47 | TAH7a | Leu 47 | Surface Loop between α2 and β1 |
| Gln 48 | TAH7a | His 48 | Surface Loop between α2 and β1 |
| Leu 49 | TAH7a | Pro 49 | β-sheet 1(N-term) |
| Tyr 51 | TAH2a | Thr 51 | Surface β1 |
| Thr 54 | TAH2a, TAH4a, TAH7a | Ser 54 | β1 |
| Gly 57 | TAH2a, TAH4a, TAH7a | Asp 57 | β1-β2 |
| Val 58 | TAH2a, TAH4a, TAH7a | Ala 58 | β-sheet 2 (N-term) |
| Ala 61 | TAH4a | Thr 61 | β-sheet 2 |
| Val 88 | Sp. 23.2 | Ile 88 | Helix α3 |
| Asn 91 | TAH2a, TAH4a, TAH7a | Tyr 91 | Loop α3-α4 |
| Asp 111 | TAH7a | Ala 111 | Surface after β4 |
| Ala 116 | TAH2a, TAH4a, TAH7a, sp. 23.2 | Val 116 | After β4 |
| Asn 167 | TAH2a, TAH4a, TAH7a | Asp 167 | Helix α6 (N-term) |
| Asn 170 | TAH2a, TAH4a, TAH7a | Ala 170 | Helix α6 |
| Tyr 174 | Sp. 10.1, Sp. 10.2 | Ile 174 | Helix α6 |
| Glu 185 | Sp. 10.1 | Asp 185 | β-sheet 7 (C-term) (surface) |
| | TAH2a, TAH4a, TAH7a | Gln 185 | Idem |
| Ile 195 | Sp. 11.2 | Leu 195 | Loop between β8 and α7 |
| Lys 222 | Sp. 10.1 | Gln 222 | Helix α8 |
| Asn 223 | Sp. 23.2, Sp. 11.2, Sp. 10.1 | Thr 223 | Helix α8 |
| Ser 244 | TAH2a, TAH4a, TAH7a | Asn 244 | Loop after α8 |
| Gly 245 | Sp. 10.1, Sp. 10.2, TAH2a, TAH4a, TAH7a | Asp 245 | Loop after α8 (surface) |
| Ser 246 | Sp. 10.1, Sp. 10.2, TAH2a, TAH4a, TAH7a | Asn 246 | Loop after α8 |
| Gly 270 | Sp. 11.2 | Asp 270 | Loop β10 and α9 (near active site) |
| Trp 283 | Sp. 23.2 | Cys 283 | Helix α9, active site |
| Ile 286 | Sp. 23.2 | Phe 286 | Helix α9 |
| Asp 292 | Sp. 23.2 | Glu 292 | Helix α9 |
| Leu 295 | Sp. 23.2 | Phe 295 | Helix α9 (C-term) |
| Ser 304 | TAH2a, TAH4a, TAH7a | Asn 304 | Helix α10 |
| Asn 323 | Sp. 10.1 | Tyr 323 | Loop α10-β11 (Surface) |
| Arg 337 | Sp. 11.2, Sp 10.1, Sp 10.2, TAH2a, TAH4a, TAH7a | lys 337 | β12-β13 |
| Ala 358 | TAH4a, TAH7a | Thr 358 | Helix α12 (N-term) |
| Glu 367 | TAH4a, TAH7a | Asp 367 | Helix α12 |
| Asp 377 | Sp. 11.2, Sp. 10.1, TAH2a | Ser 377 | Loop α12-α13 |
| | Sp. 10.2 | Asn 377 | Loop α12-α13 |

### Example 5: Purification of the xylanases

### Overexpression of the isolated xylanases

The xylanase gene fragments were excised from PCRScript Amp SK(+) (see example 4 above) with Nde I and *Xho* I and ligated into the pET 22b(+) expression vector (Novagen). The resulting recombinant plasmids were transformed to *E. coli* BL21 (DE3) cells (Stratagene).

Plasmids pXYP4 to pXYP10 were transformed to *Escherichia coli* JM109 (LMBP 4860 to LMBP 4866).

The plasmid containing the xylanase gene from strain TAH 4a was labeled pXYP4 (see LMBP 4860).

The plasmid containing the xylanase gene from strain TAH 7a was labeled pXYP5 (see LMBP 4861).

The plasmid containing the xylanase gene from strain TAH 2a was labeled pXYP6 (see LMBP 4862).

The plasmid containing the xylanase gene from strain Sp.10.1 was labeled pXYP7 (see LMBP 4863).

The plasmid containing the xylanase gene from strain Sp.10.2 was labeled pXYP8 (see LMBP 4864).

The plasmid containing the xylanase gene from strain Sp.11.2 was labeled pXYP9 (see LMBP 4865).

The plasmid containing the xylanase gene from strain Sp.23.2. was labeled pXYP10 (see LMBP 4866).

### Purification of the xylanases

The xylanases from the TAH 2a, TAH 4a, TAH 7a, Sp10.1, Sp10.2, Sp11.2 and Sp23.2 strains were purified as described (Collins *et al*, 2002, see above) from liquid cultures of recombinant *E. coli* BL21 (DE3) strains. Five ml of an overnight preculture (18°C) of the *E. coli* recombinant strain, expressing one of the cloned genes of example 2, was centrifuged at 10,000 x g for 1 min and the pellet was resuspended in 300 ml of Terrific broth (12g/l Bacto tryptone (Difco), 24 g/l yeast extract (Difco), 4 ml/l glycerol, 12.54 g/l K₂HPO₄, 2.31 g/l KH₂PO₄) containing 200 µg/ml ampicillin in a 3-liter shake flask. The culture was incubated at 18°C and 250 rpm until an absorbance at 550 nm of between 3 and 4 was reached, whereupon the expression of the enzyme was induced with 1 mM isopropyl-1-thio-β-galactopyranoside.

Following a further 20 hours incubation at 18°C, the cells were harvested by centrifugation at 18,000 x g for 30 min at 4°C, resuspended in buffer A (50 mM BICINE, pH 8.5), disrupted in a prechilled cell disrupter (Constant Systems Ltd.) at 28 Kpounds/square inch, centrifuged at 40.000 x g, and dialyzed against buffer A. The dialysate was loaded on a Q-Sepharose Fast flow (Amersham Biosciences) column (50 x 2.5 cm) equilibrated in the same buffer, and the void was collected. In the case of the xylanases from TAH 2a (SEQ ID NO: 4), TAH 4a (SEQ ID NO: 6), TAH 7a (SEQ ID NO: 8), Sp.11.2 (SEQ ID NO: 14) and Sp.23.2 (SEQ ID NO: 16), this solution was immediately loaded on a S-Sepharose Fast flow (Amersham Biosciences) column (50 x 2.5 cm), also equilibrated in the above mentioned buffer, while in the case of the xylanase from Sp10.1 (SEQ ID NO: 10) and Sp10.2 (SEQ ID NO: 12) the pH of this solution was first adjusted to pH 7.6 before loading on a S-Sepharose Fast flow (Amersham Biosciences) column (50 x 2.5 cm) equilibrated in 50mM BICINE, pH7.6. Bound protein was eluted with a linear NaCl gradient (0-100mM in 350ml) and active fractions were pooled and concentrated by ultrafiltration (Millipore PBGC 10,000 NMWL). This solution was used in the baking trials with xylanases from the strains Sp.10.1 and Sp.10.2, while the xylanases from TAH 2a, TAH 4a, TAH 7a, Sp.11.2 and Sp.23.2 strains were firstly further purified on a Sephacryl S-100 (Amersham Biosciences) column (90 x 2.5 cm) equilibrated in 20 mM MOPS, 100 mM NaCl, pH 7.5 at 1ml/min.

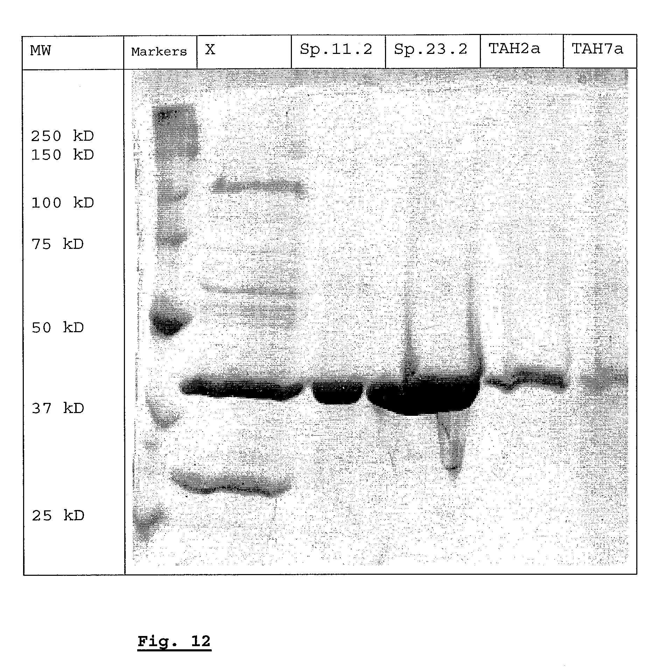

The purity of the enzymes was demonstrated by electrophoresis on a SDS-polyacrylamide gel. The results thereof are presented in figure 12.

### Example 6: Effect of the recombinant xylanases in baking of Belgian hard rolls

Baking trials were performed to demonstrate similar positive effect of the recombinant xylanases of the present invention in baking. The positive effect was evaluated by the increase in bread volume as compared to a commercially available xylanase and as compared to a reference not containing any of these enzymes.

The purified or partially purified xylanases from example 5 were evaluated in mini baking tests consisting of preparing dough with 100g of flour.

The procedure described is well known to one skilled in the art and it is obvious that the same results may be obtained by using other protocols or equipment from other suppliers.

The ingredients used are listed in table 4 below:

**Table 4**

| Ingredients (g) | RECIPE |
|---|---|
| Flour (Surbi -Molens van Deinze) | 100 |
| Water | 57 |
| Fresh yeast (Bruggeman-Belgium) | 5 |
| Sodium chloride | 2 |
| Dextrose | 2 |
| Ascorbic acid (g/100kg flour) | 4 |
| Xylanase | see Table 5 |

The ingredients were mixed for 4.5 min in a National mixer. 150 g dough pieces were weighed and rested for 20 min at 25°C in plastic boxes. The doughs were reworked and rested for a further 20 min. The final proofing time was 50 min at 36°C. The dough pieces were then baked at 225°C for 20 min. The volume of the bread was measured using the commonly used rapeseed displacement method.

The results of the baking trials with the recombinant enzymes are presented in table 5 below:

**Table 5**

| Xylanase sample | Xylanase units /100 kg flour(*) | Rolls volume increase (%) compared to control without xylanase |
|---|---|---|
| Belase B210 (Beldem-Belgium) | 1050 | 18 (¹) |
| TAH 2a | 6000 | 23 (²) |
| TAH 4a | 6000 | 21 |
| TAH 7a | 6000 | 23 |
| Sp.10.1. | 6000 | 27 |
| Sp.10.2. | 6000 | 21 |
| Sp.11.2. | 6000 | 20 |
| Sp.23.2. | 6000 | 17 |

| | | |
|---|---|---|
| (*)One unit of Belase B210 xylanase is defined as the amount of enzyme needed to release 1 µmole of reducing sugar (expressed as xylose) from birchwood xylan at 30°C and pH 4.5 (Nelson-Somogyi method). One unit of the other xylanases is defined as the amount of enzyme needed to release 1 µmole of reducing sugar (expressed as xylose) from birchwood xylan at 25°C and pH 6.5 (dinitrosalicylic acid method, Miller, G. 1959, Anal.Chem. Vol. 31, p. 426). | | |
| (¹) mean value of three independent trials | | |
| (²) mean value of two independent trials | | |

The above results show that the xylanases produced by a recombinant host cell have a similar positive effect on the volume of bread as compared to the wild type enzymes.

### Example 7: Absolute amounts of xylanase used in baking

The relative amounts of the xylanases described in the above examples, needed to obtain a defined effect on the volume in the Belgian hard rolls assay, have been compared to a commercial commonly used bacterial xylanase (Bel'ase B210 - Beldem - Belgium).

The protein concentration of the purified xylanases was determined by absorbance measurement at 280nm using extinction coefficients of 92,860 (in the case of TAH 2a), 94,140 (in the case of TAH 4a and Sp.11.2), 89,730 (in the case of TAH 7a and Sp.23.2), 95,420 (in the case of Sp.10.1) and 94,140 (in the case of Sp.10.2) M⁻¹cm⁻¹, and/or with the Bradford protein assay kit (Pierce) as described by the manufacturers.

Several baking trials were performed, according to the method described in example 6, with various amounts of the purified enzymes as described in example 5.

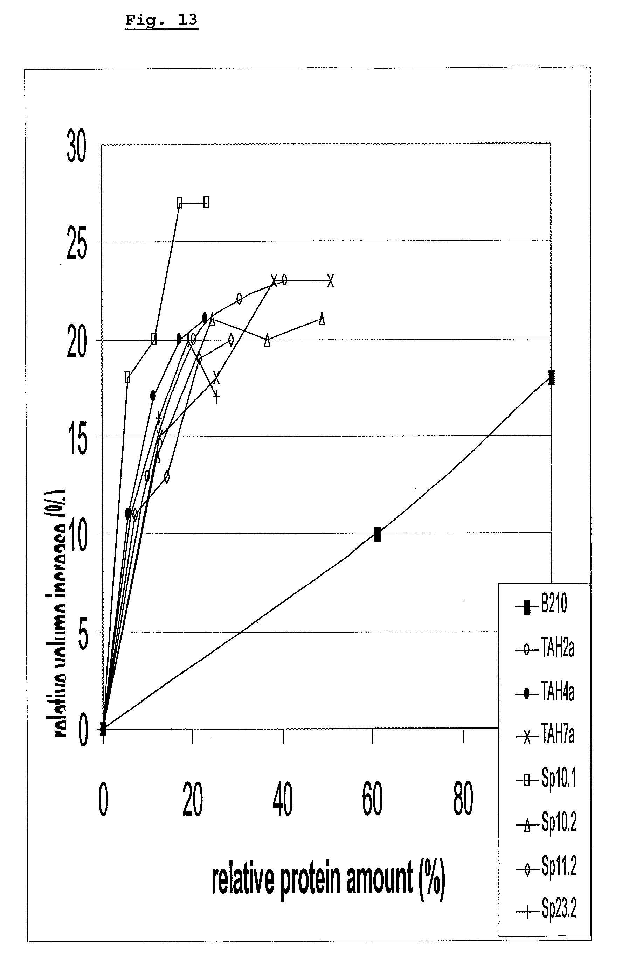

Figure 13 shows the increase of the bread volume as a function of the relative quantity of the different enzymes. For the Bel'ase B210 xylanase (about half the size of the enzymes of the invention), the results are the mean values of three independent trials. For the TAH2a xylanase, the results are the mean values of two independent trials.

Figure 13 clearly shows that the specific "in pano" activity (i.e. volume of bread expressed in ml/protein amount expressed in mg) is significantly higher for the xylanases of the present invention as compared to the commercial enzyme.

The applicant has made on 4 march 2004 a deposit (under the expert solution) of the microorganisms *Escherichia coli* JM109 (pXYP4), *Escherichia coli* JM109 (pXYP5), *Escherichia coli* JM109 (pXYP6), *Escherichia coli* JM109 (pXYP7), *Escherichia coli* JM109 (pXYP8), *Escherichia coli* JM109 (pXYP9), *Escherichia coli* JM109 (pXYP10) according to the invention, at the BCCM/LMBP culture collection (Laboratorium voor Moleculaire Biologie, Universiteit Ghent, 'Fiers-Schell-Van Montagu' building, Technologiepark 927, B-9052 Gent-Zwijnaarde, Belgium). These deposits received accession numbers LMBP 4860, LMBP 4861, LMBP 4862, LMBP 4863, LMBP 4864, LMBP 4865 and LMBP 4866 respectively.

## Claims

1. An isolated and purified enzyme with xylanolytic activity belonging to glycoside hydrolase Family 8, **characterized in that** the enzyme is a psychrophilic enzyme, with the proviso that said xylanolytic enzyme is not encoded by SEQ ID NO: 1.

2. The enzyme according to claim 1, **characterized in that** it increases the volume of a baked product by at least 10% and/or increases the width of cut of said baked product when added to a dough in a concentration of between 1500 and 6000 xylanase units/100 kg flour.

3. An isolated and purified enzyme with xylanolytic activity according to claim 1 or 2, comprising an amino acid sequence corresponding to
- aa 1 to aa 426 of any of SEQ ID NOs: 4, 6, 8, 10, 12, 14 or 16;
- aa 22 to aa 426 of any of SEQ ID NOs: 4, 6, 8, 10, 12, 14 or 16;
or a variant thereof
- comprising an amino acid sequence that differs in less than 4 amino acids from any of the above sequences, or
- comprising an amino acid sequence that has more than 50%, 70%, more preferably more than 80%, 85%, 90%, most preferably more than 95%, 97%, 98% or even more than 99% sequence identity with any of the above sequences.

4. An isolated and purified enzyme with xylanolytic activity according to claim 1 or 2, consisting of an amino acid sequence corresponding to
- aa 1 to aa 426 of any of SEQ ID NOs: 4, 6, 8, 10, 12, 14 or 16;
- aa 22 to aa 426 of any of SEQ ID NOs: 4, 6, 8, 10, 12, 14 or 16; or a variant thereof
- consisting of an amino acid sequence that differs in less than 4 amino acids from any of the above sequences, or
- consisting of an amino acid sequence that has more than 50%, 70%, more preferably more than 80%, 85%, 90%, most preferably more than 95%, 97%, 98% or even more than 99% sequence identity with any of the above sequences.

5. The enzyme according to claim 1 which is isolated from a bacterial strain selected from the group consisting of TAH 2a, TAH 4a, TAH 7a, Sp.10.1, Sp.10.2, Sp.11.2 and Sp.23.2.

6. An isolated and purified nucleotide sequence encoding an enzyme according to any of claims 1 to 5.

7. The nucleotide sequence according to claim 6 comprising SEQ ID NO: 19.

8. The nucleotide sequence according to claim 6 comprising a nucleotide sequence corresponding to
- nt 1 to nt 1281 of any of SEQ ID NOs: 4, 6, 8, 10, 12, 14 or 16;
- nt 64 to nt 1281 of any of SEQ ID NOs: 4, 6, 8, 10, 12, 14 or 16;
- or a variant of any of the above having a nucleotide sequence that differs in less than 20 nucleotides from any of the above sequences.

9. The nucleotide sequence according to claim 6 consisting of a nucleotide sequence corresponding to
- nt 1 to nt 1281 of any of SEQ ID NOs: 4, 6, 8, 10, 12, 14 or 16;
- nt 64 to nt 1281 of any of SEQ ID NOs: 4, 6, 8, 10, 12, 14 or 16; or
a variant of any of the above having a nucleotide sequence that differs in less than 20 nucleotides from any of the above sequences.

10. A recombinant nucleotide sequence comprising, operably linked to one or more nucleotide sequences according to any one of the claims 6 to 9, one or more adjacent regulatory sequence(s).

11. A vector comprising a nucleotide sequence according to any one of the claims 6 to 10.

12. **.** The vector according to claim 11, being a plasmid incorporated in *Escherichia coli* and having a deposit number selected from the group consisting of LMBP 4860, LMBP 4861, LMBP 4862, LMBP 4863, LMBP 4864, LMBP 4865 and LMBP 4866.

13. A recombinant host cell transformed with a nucleotide according to any one of the claims 6 to 10 or with the vector according to claim 11 or 12.

14. The recombinant host cell according to claim 13, **characterized in that** it is selected from the group consisting of bacteria or fungi, including yeast.

15. The enzyme according to any of claims 1 to 5, **characterized in that** it is extra-cellularly expressed by the recombinant host cell according to claim 13 or 14.

16. The enzyme to any of claims 1 to 5, **characterized in that** it is intra-cellularly expressed by the recombinant host cell according to claim 13 or 14.

17. A solid support fixing an element selected from the group consisting of the cell according to claim 13 or 14, a cell extract of the cell according to claim 13 or 14 and/or the isolated and purified enzymes with xylanolytic activity according to any one of the claims 1 to 5, 15 or 16.

18. A bread improving composition comprising at least one of the enzymes according to any of claims 1 to 5, 15 or 16.

19. The bread improving composition according to claim 18 comprising another bread improving agent selected from the list consisting of other enzymes, emulsifiers, oxidants, milk powder, fats, sugars, amino acids, salts and proteins (gluten, cellulose binding sites) or a mixture thereof.

20. The bread improving composition according to claim 19, wherein said other enzyme is selected from the list consisting of alpha-amylases, beta-amylases, maltogenic amylases, other xylanases, proteases, glucose oxidase, oxido-reductases, glucanases, cellulases, transglutaminases, isomerases, lipases, phospholipases, pectinases or a mixture thereof.

21. The bread improving composition of claim 20, wherein said alpha-amylase is an alpha-amylase obtained from *Aspergillus oryzae*.

22. Use of the recombinant host cell according to claim 13 or 14 or of the enzyme with xylanolytic activity according to any one of the claims 1 to 5, 15 or 16 for the degradation of plant cell wall components.

23. The use according to claim 22 in processes for decomposing plants and fruits, preferably fruit, legume juice, beer, paper, starch, gluten or vegetable oil preparation processes.

24. The use according to claim 22 in fruit, vegetable and plant processing, wine making or brewing, coffee processing, the processing of paper or textile, in bioconversion processes, in processes for decomposing wastes, preferably for decomposing agricultural wastes or wastes from paper mills, in starch-gluten separation processes, in feed preparations.

25. Use of the recombinant host cell according to claim 13 or 14, of the enzyme with xylanolytic activity according to any one of the claims 1 to 5, 15 or 16 or of the bread improver composition according to any of claims 18 to 21 in baking, milling, pastry and confectionery processes, especially for increasing the volume of baked products and/or for increasing the width of cut on the surface of said baked products.

26. The use according to any one of claims 22 to 25 wherein said host cell according to claim 13 or 14 or said enzyme according to any of 1 to 5, 15 or 16 is combined with any other enzyme.

27. The use according to claim 26 wherein said other enzyme is selected from the list consisting of alpha-amylases, beta-amylases, maltogenic amylases, other xylanases, proteases, glucose oxidase, oxido-reductases, glucanases, cellulases, transglutaminases, isomerases, lipases, phospholipases, pectinases or a mixture thereof.

28. The use according to claim 26 or 27 wherein said host cell or enzyme is incorporated in a bread improving composition according to any of claims 18 to 21.

29. The use according to claim 28, wherein the bread improving composition is added during the mixing of the dough.

30. The use according to any of claims 21 to 29, wherein said enzyme with xylanolytic activity as defined in any of the claims 1 to 5, 15 or 16 is present as a cell extract, a cell-free extract or as a purified protein.

31. The use according to any of the claims 22 to 30, wherein said enzyme with xylanolytic activity as defined in any of the claims 1 to 5, 15 or 16 is mixed with other ingredients in the form of a dry powder or a granulate, in particular a non-dusting granulate, or in the form of a liquid, preferably with one or more stabilizer(s) such as polyols, sugars, organic acids or sugar alcohols.

32. A composition comprising at least one of the enzymes identified by any of SEQ ID NOs: 2, 4, 6, 8, 10, 12, 14, 16, 21, 23, 25, 27, 29, 31 or 33, possibly in combination with an enzyme identified by SEQ ID NO: 2.


SEQUENCE LISTING

<110>  PURATOS NV

<120>  NOVEL FAMILY 8 XYLANASES AND THEIR USES

<130>  BP.PURA.27A/EP

<160>  33

<170>  PatentIn version 3.2

<210>  1
<211>  1281
<212>  DNA
<213>  Pseudoalteromonas haloplanktis strain TAH 3a


<220>
<221>  CDS
<222>  (1)..(1281)

<400>  1
atg aaa gta ttt ttt aaa ata aca act tta ttg tta ata cta ata agc        48
Met Lys Val Phe Phe Lys Ile Thr Thr Leu Leu Leu Ile Leu Ile Ser
1               5                   10                  15

tat caa tca ctt gct gca ttt aat aat aac cca tcg agt gta ggc gcc        96
Tyr Gln Ser Leu Ala Ala Phe Asn Asn Asn Pro Ser Ser Val Gly Ala
            20                  25                  30

tac agt tca ggg aca tac cgt aac ctc gca caa gaa atg ggt aaa aca       144
Tyr Ser Ser Gly Thr Tyr Arg Asn Leu Ala Gln Glu Met Gly Lys Thr
        35                  40                  45

aat ata cag caa aag gtg aat agt act ttt gac aat atg ttt ggc tat       192
Asn Ile Gln Gln Lys Val Asn Ser Thr Phe Asp Asn Met Phe Gly Tyr
    50                  55                  60

aac aac aca caa caa ctt tac tac ccg tac acc gaa aac ggt gtt tat       240
Asn Asn Thr Gln Gln Leu Tyr Tyr Pro Tyr Thr Glu Asn Gly Val Tyr
65                  70                  75                  80

aaa gca cac tac ata aaa gca att aac cca gac gaa ggc gac gat ata       288
Lys Ala His Tyr Ile Lys Ala Ile Asn Pro Asp Glu Gly Asp Asp Ile
                85                  90                  95

aga aca gaa ggg caa tcg tgg gga atg acc gcc gct gtc atg ctt aat       336
Arg Thr Glu Gly Gln Ser Trp Gly Met Thr Ala Ala Val Met Leu Asn
            100                 105                 110

aaa caa gaa gaa ttt gat aac cta tgg cgc ttt gca aaa gcg tat caa       384
Lys Gln Glu Glu Phe Asp Asn Leu Trp Arg Phe Ala Lys Ala Tyr Gln
        115                 120                 125

aaa aat cca gac aat cac cct gat gct aaa aaa caa ggc gtt tac gcg       432
Lys Asn Pro Asp Asn His Pro Asp Ala Lys Lys Gln Gly Val Tyr Ala
    130                 135                 140

tgg aaa cta aag ctt aat caa aac ggc ttt gtt tat aaa gtg gat gag       480
Trp Lys Leu Lys Leu Asn Gln Asn Gly Phe Val Tyr Lys Val Asp Glu
145                 150                 155                 160

ggc ccc gct ccc gat ggc gaa gag tac ttt gcg ttt gca cta ctt aat       528
Gly Pro Ala Pro Asp Gly Glu Glu Tyr Phe Ala Phe Ala Leu Leu Asn
                165                 170                 175

gcc tct gct cgt tgg ggg aat tcg ggt gag ttt aac tac tac aac gat       576

Ala Ser Ala Arg Trp Gly Asn Ser Gly Glu Phe Asn Tyr Tyr Asn Asp
            180                 185                 190

gcc att acc atg tta aac aca att aaa aat aag ctg atg gaa aac caa 624
Ala Ile Thr Met Leu Asn Thr Ile Lys Asn Lys Leu Met Glu Asn Gln
        195                 200                 205

ata atc cgc ttt tca cct tac att gat aac cta aca gac cct tct tac 672
Ile Ile Arg Phe Ser Pro Tyr Ile Asp Asn Leu Thr Asp Pro Ser Tyr
    210                 215                 220

cat ata cct gcg ttt tac gac tac ttt gca aat aac gta act aac caa 720
His Ile Pro Ala Phe Tyr Asp Tyr Phe Ala Asn Asn Val Thr Asn Gln
225                 230                 235                 240

gca gac aaa aat tac tgg cga caa gta gcc aca aaa agt aga acc tta 768
Ala Asp Lys Asn Tyr Trp Arg Gln Val Ala Thr Lys Ser Arg Thr Leu
                245                 250                 255

ctt aaa aac cat ttt aca aaa gta agt ggt agc ccg cat tgg aac tta 816
Leu Lys Asn His Phe Thr Lys Val Ser Gly Ser Pro His Trp Asn Leu
            260                 265                 270

cct aca ttt tta tcg cgc tta gat ggc agc cct gtt att ggc tac att 864
Pro Thr Phe Leu Ser Arg Leu Asp Gly Ser Pro Val Ile Gly Tyr Ile
        275                 280                 285

ttt aac ggc caa gca aac cca ggt caa tgg tat gaa ttt gat gca tgg 912
Phe Asn Gly Gln Ala Asn Pro Gly Gln Trp Tyr Glu Phe Asp Ala Trp
    290                 295                 300

cgc gta att atg aat gtg ggt tta gac gcg cat tta atg ggt gct caa 960
Arg Val Ile Met Asn Val Gly Leu Asp Ala His Leu Met Gly Ala Gln
305                 310                 315                 320

gcg tgg cat aaa agt gca gtt aat aaa gca ctg ggc ttt tta agt tat 1008
Ala Trp His Lys Ser Ala Val Asn Lys Ala Leu Gly Phe Leu Ser Tyr
                325                 330                 335

gca aaa aca aac aac agt aaa aac tgt tac gag caa gtg tat tcg tac 1056
Ala Lys Thr Asn Asn Ser Lys Asn Cys Tyr Glu Gln Val Tyr Ser Tyr
            340                 345                 350

ggt gga gcg caa aac aga ggc tgt gca ggc gaa ggt caa aaa gcc gcg 1104
Gly Gly Ala Gln Asn Arg Gly Cys Ala Gly Glu Gly Gln Lys Ala Ala
        355                 360                 365

aat gca gta gcg tta ctt gct tca aca aat gct ggg caa gca aat gag 1152
Asn Ala Val Ala Leu Leu Ala Ser Thr Asn Ala Gly Gln Ala Asn Glu
    370                 375                 380

ttt ttt aac gaa ttt tgg tct tta tcg caa cca acg ggt gac tac cgt 1200
Phe Phe Asn Glu Phe Trp Ser Leu Ser Gln Pro Thr Gly Asp Tyr Arg
385                 390                 395                 400

tac tat aat ggt tcg tta tat atg tta gct atg ctg cat gta tcg ggc 1248
Tyr Tyr Asn Gly Ser Leu Tyr Met Leu Ala Met Leu His Val Ser Gly
                405                 410                 415

aat ttt aag ttt tat aac aac acg ttt aat taa 1281
Asn Phe Lys Phe Tyr Asn Asn Thr Phe Asn
            420                 425

<210> 2
<211> 426
<212> PRT
<213> Pseudoalteromonas haloplanktis strain TAH 3a

<400> 2

Met Lys Val Phe Phe Lys Ile Thr Thr Leu Leu Leu Ile Leu Ile Ser
1               5                   10                  15

Tyr Gln Ser Leu Ala Ala Phe Asn Asn Asn Pro Ser Ser Val Gly Ala
            20                  25                  30

Tyr Ser Ser Gly Thr Tyr Arg Asn Leu Ala Gln Glu Met Gly Lys Thr
        35                  40                  45

Asn Ile Gln Gln Lys Val Asn Ser Thr Phe Asp Asn Met Phe Gly Tyr
    50                  55                  60

Asn Asn Thr Gln Gln Leu Tyr Tyr Pro Tyr Thr Glu Asn Gly Val Tyr
65                  70                  75                  80

Lys Ala His Tyr Ile Lys Ala Ile Asn Pro Asp Glu Gly Asp Asp Ile
                85                  90                  95

Arg Thr Glu Gly Gln Ser Trp Gly Met Thr Ala Ala Val Met Leu Asn
            100                 105                 110

Lys Gln Glu Glu Phe Asp Asn Leu Trp Arg Phe Ala Lys Ala Tyr Gln
        115                 120                 125

Lys Asn Pro Asp Asn His Pro Asp Ala Lys Lys Gln Gly Val Tyr Ala
    130                 135                 140

Trp Lys Leu Lys Leu Asn Gln Asn Gly Phe Val Tyr Lys Val Asp Glu
145                 150                 155                 160

Gly Pro Ala Pro Asp Gly Glu Glu Tyr Phe Ala Phe Ala Leu Leu Asn
                165                 170                 175

Ala Ser Ala Arg Trp Gly Asn Ser Gly Glu Phe Asn Tyr Tyr Asn Asp
            180                 185                 190

Ala Ile Thr Met Leu Asn Thr Ile Lys Asn Lys Leu Met Glu Asn Gln
        195                 200                 205

Ile Ile Arg Phe Ser Pro Tyr Ile Asp Asn Leu Thr Asp Pro Ser Tyr
    210                 215                 220

His Ile Pro Ala Phe Tyr Asp Tyr Phe Ala Asn Asn Val Thr Asn Gln
225                 230                 235                 240

Ala Asp Lys Asn Tyr Trp Arg Gln Val Ala Thr Lys Ser Arg Thr Leu
                245                 250                 255

Leu Lys Asn His Phe Thr Lys Val Ser Gly Ser Pro His Trp Asn Leu

260                    265                    270

Pro Thr Phe Leu Ser Arg Leu Asp Gly Ser Pro Val Ile Gly Tyr Ile
        275                    280                    285

Phe Asn Gly Gln Ala Asn Pro Gly Gln Trp Tyr Glu Phe Asp Ala Trp
    290                    295                    300

Arg Val Ile Met Asn Val Gly Leu Asp Ala His Leu Met Gly Ala Gln
305                    310                    315                    320

Ala Trp His Lys Ser Ala Val Asn Lys Ala Leu Gly Phe Leu Ser Tyr
                325                    330                    335

Ala Lys Thr Asn Asn Ser Lys Asn Cys Tyr Glu Gln Val Tyr Ser Tyr
                340                    345                    350

Gly Gly Ala Gln Asn Arg Gly Cys Ala Gly Glu Gly Gln Lys Ala Ala
        355                    360                    365

Asn Ala Val Ala Leu Leu Ala Ser Thr Asn Ala Gly Gln Ala Asn Glu
    370                    375                    380

Phe Phe Asn Glu Phe Trp Ser Leu Ser Gln Pro Thr Gly Asp Tyr Arg
385                    390                    395                    400

Tyr Tyr Asn Gly Ser Leu Tyr Met Leu Ala Met Leu His Val Ser Gly
                405                    410                    415

Asn Phe Lys Phe Tyr Asn Asn Thr Phe Asn
            420                    425

<210>  3
<211>  1281
<212>  DNA
<213>  strain TAH2a

<220>
<221>  CDS
<222>  (1)..(1281)

<400>  3
atg aaa gta ttt ttt aaa ata aca act tta ttg tta atg cta ata agc        48
Met Lys Val Phe Phe Lys Ile Thr Thr Leu Leu Leu Met Leu Ile Ser
1               5                   10                  15

tat caa tca ctt gct gca ttt aat aat aat cca tcg agt gta ggc gcc        96
Tyr Gln Ser Leu Ala Ala Phe Asn Asn Asn Pro Ser Ser Val Gly Ala
            20                  25                  30

gac agc tcg ggg aca tac cgt aac ctt gca caa gaa atg ggt aaa acg       144
Asp Ser Ser Gly Thr Tyr Arg Asn Leu Ala Gln Glu Met Gly Lys Thr
        35                  40                  45

aac ata cag caa aag gtg aat agt act ttt aac aat atg ttt gga agt       192
Asn Ile Gln Gln Lys Val Asn Ser Thr Phe Asn Asn Met Phe Gly Ser

50                  55                  60

aat aat acc cag caa ttg tat acc cat tac tca gaa aat gac gca tat        240
Asn Asn Thr Gln Gln Leu Tyr Thr His Tyr Ser Glu Asn Asp Ala Tyr
65                  70                  75                  80

aaa gca cac tac ata aaa gca att aac cct gac gag ggt gat gat ata        288
Lys Ala His Tyr Ile Lys Ala Ile Asn Pro Asp Glu Gly Asp Asp Ile
                85                  90                  95

aga aca gaa ggg caa tca tgg ggg atg act gct gct gtt atg ctc tac        336
Arg Thr Glu Gly Gln Ser Trp Gly Met Thr Ala Ala Val Met Leu Tyr
            100                 105                 110

aag caa gaa gaa ttt gat aac tta tgg cgc ttt gct aaa gcg tat caa        384
Lys Gln Glu Glu Phe Asp Asn Leu Trp Arg Phe Ala Lys Ala Tyr Gln
        115                 120                 125

aaa aat cca gac aat cac cct gat gta aaa aaa caa ggt gtt tac gcg        432
Lys Asn Pro Asp Asn His Pro Asp Val Lys Lys Gln Gly Val Tyr Ala
    130                 135                 140

tgg aaa cta aag ctt aat caa aac ggt ttt gtt tat aaa gta gat gaa        480
Trp Lys Leu Lys Leu Asn Gln Asn Gly Phe Val Tyr Lys Val Asp Glu
145                 150                 155                 160

ggc ccg gca cct gat ggt gaa gag tac ttt gct ttt gca cta ctt aat        528
Gly Pro Ala Pro Asp Gly Glu Glu Tyr Phe Ala Phe Ala Leu Leu Asn
                165                 170                 175

gcg tca gct cgt tgg ggt aat tcg ggc gaa ttt gat tat tat gct gat        576
Ala Ser Ala Arg Trp Gly Asn Ser Gly Glu Phe Asp Tyr Tyr Ala Asp
            180                 185                 190

gcc att acc atg tta aac aca att aaa aac aag ctg atg caa aac caa        624
Ala Ile Thr Met Leu Asn Thr Ile Lys Asn Lys Leu Met Gln Asn Gln
        195                 200                 205

ata atc cgt ttt tca cct tac att gat aac cta aca gac cct tct tat        672
Ile Ile Arg Phe Ser Pro Tyr Ile Asp Asn Leu Thr Asp Pro Ser Tyr
    210                 215                 220

cat ata cct gcg ttt tac gac tac ttt gca aat aac gtg act aat caa        720
His Ile Pro Ala Phe Tyr Asp Tyr Phe Ala Asn Asn Val Thr Asn Gln
225                 230                 235                 240

gcc gat aaa aat tac tgg cga caa gtg gct aca aaa agt aga acc tta        768
Ala Asp Lys Asn Tyr Trp Arg Gln Val Ala Thr Lys Ser Arg Thr Leu
                245                 250                 255

ctt aaa aac cat ttt aca aaa gtg aat gat aat ccg cat tgg aac tta        816
Leu Lys Asn His Phe Thr Lys Val Asn Asp Asn Pro His Trp Asn Leu
            260                 265                 270

cct aca ttt tta tcg cgc tta gat ggc agc cct gtt att ggt tac att        864
Pro Thr Phe Leu Ser Arg Leu Asp Gly Ser Pro Val Ile Gly Tyr Ile
        275                 280                 285

ttt aac ggc caa gca aac cca ggt caa tgg tat gag ttt gat gca tgg        912
Phe Asn Gly Gln Ala Asn Pro Gly Gln Trp Tyr Glu Phe Asp Ala Trp
    290                 295                 300

cgc gtt att atg aat gtt ggc tta gac gcg cac tta atg ggt gct cag        960
Arg Val Ile Met Asn Val Gly Leu Asp Ala His Leu Met Gly Ala Gln
305                 310                 315                 320

gcg tgg cat aaa aat gca gtt aat aaa gca ctg ggc ttt tta agt tat       1008
Ala Trp His Lys Asn Ala Val Asn Lys Ala Leu Gly Phe Leu Ser Tyr

                325                 330                 335

gca aaa aca aac aac agt aaa aac tgc tac gag caa gtg tat tca tac 1056
Ala Lys Thr Asn Asn Ser Lys Asn Cys Tyr Glu Gln Val Tyr Ser Tyr
            340                 345                 350

ggt gga gcg caa aac aaa ggc tgt gca ggc gaa ggt caa aaa gcc gcg 1104
Gly Gly Ala Gln Asn Lys Gly Cys Ala Gly Glu Gly Gln Lys Ala Ala
        355                 360                 365

aat gca gta gcg tta ctt gct tca aca aac gct ggg caa gca aat gaa 1152
Asn Ala Val Ala Leu Leu Ala Ser Thr Asn Ala Gly Gln Ala Asn Glu
    370                 375                 380

ttt ttt aac gag ttt tgg tct tta tcg caa cca aca ggt agc tac cgt 1200
Phe Phe Asn Glu Phe Trp Ser Leu Ser Gln Pro Thr Gly Ser Tyr Arg
385                 390                 395                 400

tac tac aat ggt tcg tta tat atg tta gct atg ctg cat gta tcg ggc 1248
Tyr Tyr Asn Gly Ser Leu Tyr Met Leu Ala Met Leu His Val Ser Gly
                405                 410                 415

aat ttt aag ttt tat aac aac acg ttt aat taa 1281
Asn Phe Lys Phe Tyr Asn Asn Thr Phe Asn
            420                 425

<210> 4
<211> 426
<212> PRT
<213> strain TAH2a

<400> 4

Met Lys Val Phe Phe Lys Ile Thr Thr Leu Leu Leu Met Leu Ile Ser
1               5                   10                  15

Tyr Gln Ser Leu Ala Ala Phe Asn Asn Asn Pro Ser Ser Val Gly Ala
            20                  25                  30

Asp Ser Ser Gly Thr Tyr Arg Asn Leu Ala Gln Glu Met Gly Lys Thr
        35                  40                  45

Asn Ile Gln Gln Lys Val Asn Ser Thr Phe Asn Asn Met Phe Gly Ser
    50                  55                  60

Asn Asn Thr Gln Gln Leu Tyr Thr His Tyr Ser Glu Asn Asp Ala Tyr
65                  70                  75                  80

Lys Ala His Tyr Ile Lys Ala Ile Asn Pro Asp Glu Gly Asp Asp Ile
                85                  90                  95

Arg Thr Glu Gly Gln Ser Trp Gly Met Thr Ala Ala Val Met Leu Tyr
            100                 105                 110

Lys Gln Glu Glu Phe Asp Asn Leu Trp Arg Phe Ala Lys Ala Tyr Gln
        115                 120                 125

Lys Asn Pro Asp Asn His Pro Asp Val Lys Lys Gln Gly Val Tyr Ala
    130                 135                 140

Trp Lys Leu Lys Leu Asn Gln Asn Gly Phe Val Tyr Lys Val Asp Glu
145                 150                 155                 160

Gly Pro Ala Pro Asp Gly Glu Glu Tyr Phe Ala Phe Ala Leu Leu Asn
                165                 170                 175

Ala Ser Ala Arg Trp Gly Asn Ser Gly Glu Phe Asp Tyr Tyr Ala Asp
            180                 185                 190

Ala Ile Thr Met Leu Asn Thr Ile Lys Asn Lys Leu Met Gln Asn Gln
        195                 200                 205

Ile Ile Arg Phe Ser Pro Tyr Ile Asp Asn Leu Thr Asp Pro Ser Tyr
    210                 215                 220

His Ile Pro Ala Phe Tyr Asp Tyr Phe Ala Asn Asn Val Thr Asn Gln
225                 230                 235                 240

Ala Asp Lys Asn Tyr Trp Arg Gln Val Ala Thr Lys Ser Arg Thr Leu
                245                 250                 255

Leu Lys Asn His Phe Thr Lys Val Asn Asp Asn Pro His Trp Asn Leu
            260                 265                 270

Pro Thr Phe Leu Ser Arg Leu Asp Gly Ser Pro Val Ile Gly Tyr Ile
        275                 280                 285

Phe Asn Gly Gln Ala Asn Pro Gly Gln Trp Tyr Glu Phe Asp Ala Trp
    290                 295                 300

Arg Val Ile Met Asn Val Gly Leu Asp Ala His Leu Met Gly Ala Gln
305                 310                 315                 320

Ala Trp His Lys Asn Ala Val Asn Lys Ala Leu Gly Phe Leu Ser Tyr
                325                 330                 335

Ala Lys Thr Asn Asn Ser Lys Asn Cys Tyr Glu Gln Val Tyr Ser Tyr
            340                 345                 350

Gly Gly Ala Gln Asn Lys Gly Cys Ala Gly Glu Gly Gln Lys Ala Ala
        355                 360                 365

Asn Ala Val Ala Leu Leu Ala Ser Thr Asn Ala Gly Gln Ala Asn Glu
    370                 375                 380

Phe Phe Asn Glu Phe Trp Ser Leu Ser Gln Pro Thr Gly Ser Tyr Arg
385                 390                 395                 400

Tyr Tyr Asn Gly Ser Leu Tyr Met Leu Ala Met Leu His Val Ser Gly
                405                 410                 415

Asn Phe Lys Phe Tyr Asn Asn Thr Phe Asn
            420                 425

<210>  5
<211>  1281
<212>  DNA
<213>  strain TAH4a  haloplanktis

<220>
<221>  CDS
<222>  (1)..(1281)

<400>  5
atg aaa gta ttt ttt aaa ata aca act tta ttg tta atg cta ata agc        48
Met Lys Val Phe Phe Lys Ile Thr Thr Leu Leu Leu Met Leu Ile Ser
1               5                   10                  15

tat caa tca ctt gct gca ttt aat aat aat cca tcg agt gta ggc gcc        96
Tyr Gln Ser Leu Ala Ala Phe Asn Asn Asn Pro Ser Ser Val Gly Ala
            20                  25                  30

gac agc tcg ggg aca tac cgt aac ctt gca caa gaa atg ggt aaa acg       144
Asp Ser Ser Gly Thr Tyr Arg Asn Leu Ala Gln Glu Met Gly Lys Thr
        35                  40                  45

aac ata cag caa aag gtg aat agt act ttt aac aat atg ttt gga agt       192
Asn Ile Gln Gln Lys Val Asn Ser Thr Phe Asn Asn Met Phe Gly Ser
    50                  55                  60

aat aat acc cag caa ttg tat tac cca tac tca gaa aat gac gca tat       240
Asn Asn Thr Gln Gln Leu Tyr Tyr Pro Tyr Ser Glu Asn Asp Ala Tyr
65                  70                  75                  80

aaa aca cac tac ata aaa gca att aac cct gac gag ggt gat gat ata       288
Lys Thr His Tyr Ile Lys Ala Ile Asn Pro Asp Glu Gly Asp Asp Ile
                85                  90                  95

aga aca gaa ggg caa tca tgg ggg atg act gct gct gtt atg ctc tac       336
Arg Thr Glu Gly Gln Ser Trp Gly Met Thr Ala Ala Val Met Leu Tyr
            100                 105                 110

aag caa gaa gaa ttt gat aac tta tgg cgc ttt gct aaa gcg tat caa       384
Lys Gln Glu Glu Phe Asp Asn Leu Trp Arg Phe Ala Lys Ala Tyr Gln
        115                 120                 125

aaa aat cca gac aat cac cct gat gta aaa aaa caa ggt gtt tac gcg       432
Lys Asn Pro Asp Asn His Pro Asp Val Lys Lys Gln Gly Val Tyr Ala
    130                 135                 140

tgg aaa cta aag ctt aat caa aac ggt ttt gtt tat aaa gta gat gaa       480
Trp Lys Leu Lys Leu Asn Gln Asn Gly Phe Val Tyr Lys Val Asp Glu
145                 150                 155                 160

ggc ccg gca cct gat ggt gaa gag tac ttt gct ttt gca cta ctt aat       528
Gly Pro Ala Pro Asp Gly Glu Glu Tyr Phe Ala Phe Ala Leu Leu Asn
                165                 170                 175

gcg tca gct cgt tgg ggt aat tcg ggc gaa ttt gat tat tat gct gat       576
Ala Ser Ala Arg Trp Gly Asn Ser Gly Glu Phe Asp Tyr Tyr Ala Asp
            180                 185                 190

gcc att acc atg tta aac aca att aaa aac aag ctg atg caa aac caa       624
Ala Ile Thr Met Leu Asn Thr Ile Lys Asn Lys Leu Met Gln Asn Gln
        195                 200                 205

ata atc cgt ttt tca cct tac att gat aac cta aca gac cct tct tat 672
Ile Ile Arg Phe Ser Pro Tyr Ile Asp Asn Leu Thr Asp Pro Ser Tyr
    210                 215                 220

cat ata cct gcg ttt tac gac tac ttt gca aat aac gtg act aat caa 720
His Ile Pro Ala Phe Tyr Asp Tyr Phe Ala Asn Asn Val Thr Asn Gln
225                 230                 235                 240

gcc gat aaa aat tac tgg cga caa gtg gct aca aaa agt aga acc tta 768
Ala Asp Lys Asn Tyr Trp Arg Gln Val Ala Thr Lys Ser Arg Thr Leu
                245                 250                 255

ctt aaa aac cat ttt aca aaa gtg aat gat aat ccg cat tgg aac tta 816
Leu Lys Asn His Phe Thr Lys Val Asn Asp Asn Pro His Trp Asn Leu
            260                 265                 270

cct aca ttt tta tcg cgc tta gat ggc agc cct gtt att ggt tac att 864
Pro Thr Phe Leu Ser Arg Leu Asp Gly Ser Pro Val Ile Gly Tyr Ile
        275                 280                 285

ttt aac ggc caa gca aac cca ggt caa tgg tat gag ttt gat gca tgg 912
Phe Asn Gly Gln Ala Asn Pro Gly Gln Trp Tyr Glu Phe Asp Ala Trp
    290                 295                 300

cgc gtt att atg aat gtt ggc tta gac gcg cac tta atg ggt gct cag 960
Arg Val Ile Met Asn Val Gly Leu Asp Ala His Leu Met Gly Ala Gln
305                 310                 315                 320

gcg tgg cat aaa aat gca gtt aat aaa gca ctt ggc ttt tta agt tat 1008
Ala Trp His Lys Asn Ala Val Asn Lys Ala Leu Gly Phe Leu Ser Tyr
                325                 330                 335

gca aaa aca aac aac agt aaa aac tgt tac gag caa gtg tat tcg tac 1056
Ala Lys Thr Asn Asn Ser Lys Asn Cys Tyr Glu Gln Val Tyr Ser Tyr
            340                 345                 350

ggt gga gcg caa aac aaa ggc tgc gca ggc gaa ggt caa aaa gca gca 1104
Gly Gly Ala Gln Asn Lys Gly Cys Ala Gly Glu Gly Gln Lys Ala Ala
        355                 360                 365

aat gca gta gcg tta ctt gct tca aca aac act ggg caa gca aat gag 1152
Asn Ala Val Ala Leu Leu Ala Ser Thr Asn Thr Gly Gln Ala Asn Glu
    370                 375                 380

ttt ttt aac gac ttt tgg tct tta tcg caa cca acg ggt gac tac cgt 1200
Phe Phe Asn Asp Phe Trp Ser Leu Ser Gln Pro Thr Gly Asp Tyr Arg
385                 390                 395                 400

tac tac aat ggt tcg tta tat atg tta gct atg ctg cat gta tcg ggt 1248
Tyr Tyr Asn Gly Ser Leu Tyr Met Leu Ala Met Leu His Val Ser Gly
                405                 410                 415

aac ttt aag ttt tac aac aac acg ttt aat taa 1281
Asn Phe Lys Phe Tyr Asn Asn Thr Phe Asn
            420                 425

<210> 6
<211> 426
<212> PRT
<213> strain TAH4a haloplanktis

<400> 6

Met Lys Val Phe Phe Lys Ile Thr Thr Leu Leu Leu Met Leu Ile Ser
1               5                   10                  15

Tyr Gln Ser Leu Ala Ala Phe Asn Asn Asn Pro Ser Ser Val Gly Ala
            20                  25                  30

Asp Ser Ser Gly Thr Tyr Arg Asn Leu Ala Gln Glu Met Gly Lys Thr
        35                  40                  45

Asn Ile Gln Gln Lys Val Asn Ser Thr Phe Asn Asn Met Phe Gly Ser
    50                  55                  60

Asn Asn Thr Gln Gln Leu Tyr Tyr Pro Tyr Ser Glu Asn Asp Ala Tyr
65                  70                  75                  80

Lys Thr His Tyr Ile Lys Ala Ile Asn Pro Asp Glu Gly Asp Asp Ile
                85                  90                  95

Arg Thr Glu Gly Gln Ser Trp Gly Met Thr Ala Ala Val Met Leu Tyr
            100                 105                 110

Lys Gln Glu Glu Phe Asp Asn Leu Trp Arg Phe Ala Lys Ala Tyr Gln
        115                 120                 125

Lys Asn Pro Asp Asn His Pro Asp Val Lys Lys Gln Gly Val Tyr Ala
    130                 135                 140

Trp Lys Leu Lys Leu Asn Gln Asn Gly Phe Val Tyr Lys Val Asp Glu
145                 150                 155                 160

Gly Pro Ala Pro Asp Gly Glu Glu Tyr Phe Ala Phe Ala Leu Leu Asn
                165                 170                 175

Ala Ser Ala Arg Trp Gly Asn Ser Gly Glu Phe Asp Tyr Tyr Ala Asp
            180                 185                 190

Ala Ile Thr Met Leu Asn Thr Ile Lys Asn Lys Leu Met Gln Asn Gln
        195                 200                 205

Ile Ile Arg Phe Ser Pro Tyr Ile Asp Asn Leu Thr Asp Pro Ser Tyr
    210                 215                 220

His Ile Pro Ala Phe Tyr Asp Tyr Phe Ala Asn Asn Val Thr Asn Gln
225                 230                 235                 240

Ala Asp Lys Asn Tyr Trp Arg Gln Val Ala Thr Lys Ser Arg Thr Leu
                245                 250                 255

Leu Lys Asn His Phe Thr Lys Val Asn Asp Asn Pro His Trp Asn Leu
            260                 265                 270

Pro Thr Phe Leu Ser Arg Leu Asp Gly Ser Pro Val Ile Gly Tyr Ile
        275                 280                 285

Phe Asn Gly Gln Ala Asn Pro Gly Gln Trp Tyr Glu Phe Asp Ala Trp
    290                 295                 300

Arg Val Ile Met Asn Val Gly Leu Asp Ala His Leu Met Gly Ala Gln
305                 310                 315                 320

Ala Trp His Lys Asn Ala Val Asn Lys Ala Leu Gly Phe Leu Ser Tyr
                325                 330                 335

Ala Lys Thr Asn Asn Ser Lys Asn Cys Tyr Glu Gln Val Tyr Ser Tyr
            340                 345                 350

Gly Gly Ala Gln Asn Lys Gly Cys Ala Gly Glu Gly Gln Lys Ala Ala
        355                 360                 365

Asn Ala Val Ala Leu Leu Ala Ser Thr Asn Thr Gly Gln Ala Asn Glu
    370                 375                 380

Phe Phe Asn Asp Phe Trp Ser Leu Ser Gln Pro Thr Gly Asp Tyr Arg
385                 390                 395                 400

Tyr Tyr Asn Gly Ser Leu Tyr Met Leu Ala Met Leu His Val Ser Gly
                405                 410                 415

Asn Phe Lys Phe Tyr Asn Asn Thr Phe Asn
            420                 425

<210>  7
<211>  1281
<212>  DNA
<213>  strain TAH7a

<220>
<221>  CDS
<222>  (1)..(1281)

<400>  7
atg aaa gta ttt ttt aaa ata aca act tta ttg tta atg cta ata agc        48
Met Lys Val Phe Phe Lys Ile Thr Thr Leu Leu Leu Met Leu Ile Ser
1               5                   10                  15

tat caa tca ctt gct gca ttt aat aat aat cct acg agt gta ggc ccc        96
Tyr Gln Ser Leu Ala Ala Phe Asn Asn Asn Pro Thr Ser Val Gly Pro
            20                  25                  30

gca agc ttg ggg aca tac cgt tac ctt gca caa gaa ttg cgt aaa cgg       144
Ala Ser Leu Gly Thr Tyr Arg Tyr Leu Ala Gln Glu Leu Arg Lys Arg
        35                  40                  45

acc ata cgc caa agc gtg aat agt act ttt aac aat atg ttt gga agt       192
Thr Ile Arg Gln Ser Val Asn Ser Thr Phe Asn Asn Met Phe Gly Ser
    50                  55                  60

aat aat acc ctt cac ccg tat tac cca tac tca gaa aat gac gca tat       240
Asn Asn Thr Leu His Pro Tyr Tyr Pro Tyr Ser Glu Asn Asp Ala Tyr
65                  70                  75                  80

aaa gca cac tac ata aaa gca att aac cct gac gag ggt gat gat ata        288
Lys Ala His Tyr Ile Lys Ala Ile Asn Pro Asp Glu Gly Asp Asp Ile
                85                  90                  95

aga aca gaa ggg caa tca tgg ggg atg act gct gct gtt atg ctc tac        336
Arg Thr Glu Gly Gln Ser Trp Gly Met Thr Ala Ala Val Met Leu Tyr
            100                 105                 110

aag caa gaa gaa ttt gat aac tta tgg cgc ttt gct aaa gcg tat caa        384
Lys Gln Glu Glu Phe Asp Asn Leu Trp Arg Phe Ala Lys Ala Tyr Gln
        115                 120                 125

aaa aat cca gcc aat cac cct gat gta aaa aaa caa ggt gtt tac gcg        432
Lys Asn Pro Ala Asn His Pro Asp Val Lys Lys Gln Gly Val Tyr Ala
    130                 135                 140

tgg aaa cta aag ctt aat caa aac ggt ttt gtt tat aaa gta gat gaa        480
Trp Lys Leu Lys Leu Asn Gln Asn Gly Phe Val Tyr Lys Val Asp Glu
145                 150                 155                 160

ggc ccg gca cct gat ggt gaa gag tac ttt gct ttt gca cta ctt aat        528
Gly Pro Ala Pro Asp Gly Glu Glu Tyr Phe Ala Phe Ala Leu Leu Asn
                165                 170                 175

gcg tca gct cgt tgg ggt aat tcg ggc gaa ttt gat tat tat gct gat        576
Ala Ser Ala Arg Trp Gly Asn Ser Gly Glu Phe Asp Tyr Tyr Ala Asp
            180                 185                 190

gcc att acc atg tta aac aca att aaa aac aag ctg atg caa aac caa        624
Ala Ile Thr Met Leu Asn Thr Ile Lys Asn Lys Leu Met Gln Asn Gln
        195                 200                 205

ata atc cgt ttt tca cct tac att gat aac cta aca gac cct tct tat        672
Ile Ile Arg Phe Ser Pro Tyr Ile Asp Asn Leu Thr Asp Pro Ser Tyr
    210                 215                 220

cat ata cct gcg ttt tac gac tac ttt gca aat aac gtg act aat caa        720
His Ile Pro Ala Phe Tyr Asp Tyr Phe Ala Asn Asn Val Thr Asn Gln
225                 230                 235                 240

gcc gat aaa aat tac tgg cga caa gtg gct aca aaa agt aga acc tta        768
Ala Asp Lys Asn Tyr Trp Arg Gln Val Ala Thr Lys Ser Arg Thr Leu
                245                 250                 255

ctt aaa aac cat ttt aca aaa gtg aat gat aat ccg cat tgg aac tta        816
Leu Lys Asn His Phe Thr Lys Val Asn Asp Asn Pro His Trp Asn Leu
            260                 265                 270

cct aca ttt tta tcg cgc tta gat ggc agc cct gtt att ggt tac att        864
Pro Thr Phe Leu Ser Arg Leu Asp Gly Ser Pro Val Ile Gly Tyr Ile
        275                 280                 285

ttt aac ggc caa gca aac cca ggt caa tgg tat gag ttt gat gca tgg        912
Phe Asn Gly Gln Ala Asn Pro Gly Gln Trp Tyr Glu Phe Asp Ala Trp
    290                 295                 300

cgc gtt att atg aat gtt ggc tta gac gcg cac tta atg ggt gct cag        960
Arg Val Ile Met Asn Val Gly Leu Asp Ala His Leu Met Gly Ala Gln
305                 310                 315                 320

gcg tgg cat aaa aat gca gtt aat aaa gca ctt ggc ttt tta agt tat       1008
Ala Trp His Lys Asn Ala Val Asn Lys Ala Leu Gly Phe Leu Ser Tyr
                325                 330                 335

gca aaa aca aac aac agt aaa aac tgt tac gag caa gtg tat tcg tac       1056
Ala Lys Thr Asn Asn Ser Lys Asn Cys Tyr Glu Gln Val Tyr Ser Tyr
            340                 345                 350

ggt gga gcg caa aac aaa ggc tgc gca ggc gaa ggt caa aaa gca gca 1104
Gly Gly Ala Gln Asn Lys Gly Cys Ala Gly Glu Gly Gln Lys Ala Ala
        355                 360                 365

aat gca gta gcg tta ctt gct tca aca aac act ggg caa gcc aat gag 1152
Asn Ala Val Ala Leu Leu Ala Ser Thr Asn Thr Gly Gln Ala Asn Glu
    370                 375                 380

ttt ttt aac gac ttt ggg tct tta tcg caa cca acg ggt gac tac cgt 1200
Phe Phe Asn Asp Phe Gly Ser Leu Ser Gln Pro Thr Gly Asp Tyr Arg
385                 390                 395                 400

tac tac aat ggt tcg tta tat atg tta gct atg ctg cat gta tcg ggg 1248
Tyr Tyr Asn Gly Ser Leu Tyr Met Leu Ala Met Leu His Val Ser Gly
                405                 410                 415

aac ttt aag ttt tac aac aac acg ttt aat taa 1281
Asn Phe Lys Phe Tyr Asn Asn Thr Phe Asn
            420                 425

<210> 8
<211> 426
<212> PRT
<213> strain TAH7a

<400> 8

Met Lys Val Phe Phe Lys Ile Thr Thr Leu Leu Leu Met Leu Ile Ser
1               5                   10                  15

Tyr Gln Ser Leu Ala Ala Phe Asn Asn Asn Pro Thr Ser Val Gly Pro
            20                  25                  30

Ala Ser Leu Gly Thr Tyr Arg Tyr Leu Ala Gln Glu Leu Arg Lys Arg
        35                  40                  45

Thr Ile Arg Gln Ser Val Asn Ser Thr Phe Asn Asn Met Phe Gly Ser
    50                  55                  60

Asn Asn Thr Leu His Pro Tyr Tyr Pro Tyr Ser Glu Asn Asp Ala Tyr
65                  70                  75                  80

Lys Ala His Tyr Ile Lys Ala Ile Asn Pro Asp Glu Gly Asp Asp Ile
                85                  90                  95

Arg Thr Glu Gly Gln Ser Trp Gly Met Thr Ala Ala Val Met Leu Tyr
            100                 105                 110

Lys Gln Glu Glu Phe Asp Asn Leu Trp Arg Phe Ala Lys Ala Tyr Gln
        115                 120                 125

Lys Asn Pro Ala Asn His Pro Asp Val Lys Lys Gln Gly Val Tyr Ala
    130                 135                 140

Trp Lys Leu Lys Leu Asn Gln Asn Gly Phe Val Tyr Lys Val Asp Glu
145                 150                 155                 160

Gly Pro Ala Pro Asp Gly Glu Glu Tyr Phe Ala Phe Ala Leu Leu Asn
                165                 170                 175

Ala Ser Ala Arg Trp Gly Asn Ser Gly Glu Phe Asp Tyr Tyr Ala Asp
            180                 185                 190

Ala Ile Thr Met Leu Asn Thr Ile Lys Asn Lys Leu Met Gln Asn Gln
        195                 200                 205

Ile Ile Arg Phe Ser Pro Tyr Ile Asp Asn Leu Thr Asp Pro Ser Tyr
    210                 215                 220

His Ile Pro Ala Phe Tyr Asp Tyr Phe Ala Asn Asn Val Thr Asn Gln
225                 230                 235                 240

Ala Asp Lys Asn Tyr Trp Arg Gln Val Ala Thr Lys Ser Arg Thr Leu
                245                 250                 255

Leu Lys Asn His Phe Thr Lys Val Asn Asp Asn Pro His Trp Asn Leu
            260                 265                 270

Pro Thr Phe Leu Ser Arg Leu Asp Gly Ser Pro Val Ile Gly Tyr Ile
        275                 280                 285

Phe Asn Gly Gln Ala Asn Pro Gly Gln Trp Tyr Glu Phe Asp Ala Trp
    290                 295                 300

Arg Val Ile Met Asn Val Gly Leu Asp Ala His Leu Met Gly Ala Gln
305                 310                 315                 320

Ala Trp His Lys Asn Ala Val Asn Lys Ala Leu Gly Phe Leu Ser Tyr
                325                 330                 335

Ala Lys Thr Asn Asn Ser Lys Asn Cys Tyr Glu Gln Val Tyr Ser Tyr
            340                 345                 350

Gly Gly Ala Gln Asn Lys Gly Cys Ala Gly Glu Gly Gln Lys Ala Ala
        355                 360                 365

Asn Ala Val Ala Leu Leu Ala Ser Thr Asn Thr Gly Gln Ala Asn Glu
    370                 375                 380

Phe Phe Asn Asp Phe Gly Ser Leu Ser Gln Pro Thr Gly Asp Tyr Arg
385                 390                 395                 400

Tyr Tyr Asn Gly Ser Leu Tyr Met Leu Ala Met Leu His Val Ser Gly
                405                 410                 415

Asn Phe Lys Phe Tyr Asn Asn Thr Phe Asn
            420                 425

<210>  9
<211>  1281
<212>  DNA
<213>  strain Sp.10.1


<220>
<221>  CDS
<222>  (1)..(1281)

<400>  9
atg aaa gta ttt ttt aaa ata aca act tta ttg tta ata cta ata agc      48
Met Lys Val Phe Phe Lys Ile Thr Thr Leu Leu Leu Ile Leu Ile Ser
1               5                   10                  15

tat caa tca ctt gct gca ttt aat aat aac cca tcg agt gta ggc gcc      96
Tyr Gln Ser Leu Ala Ala Phe Asn Asn Asn Pro Ser Ser Val Gly Ala
            20                  25                  30

gac agt tca gga gca tac cgt aac ctc gca caa gaa atg ggt aaa aca      144
Asp Ser Ser Gly Ala Tyr Arg Asn Leu Ala Gln Glu Met Gly Lys Thr
        35                  40                  45

aat ata cag caa aaa gtg aat agt act ttt gac aat atg ttt ggc tat      192
Asn Ile Gln Gln Lys Val Asn Ser Thr Phe Asp Asn Met Phe Gly Tyr
    50                  55                  60

aac aac aca caa caa ctt tac tac ccg tac acc gaa aac ggt gtt tat      240
Asn Asn Thr Gln Gln Leu Tyr Tyr Pro Tyr Thr Glu Asn Gly Val Tyr
65                  70                  75                  80

aaa gca cac tac ata aaa gca att aac cct gac gag ggt gat gat ata      288
Lys Ala His Tyr Ile Lys Ala Ile Asn Pro Asp Glu Gly Asp Asp Ile
                85                  90                  95

aga aca gaa ggg caa tcg tgg gga atg acc gcc gct gtc atg ctt aat      336
Arg Thr Glu Gly Gln Ser Trp Gly Met Thr Ala Ala Val Met Leu Asn
            100                 105                 110

aaa caa gaa gaa ttt gat aac cta tgg cgc ttt gca aaa gcg tat caa      384
Lys Gln Glu Glu Phe Asp Asn Leu Trp Arg Phe Ala Lys Ala Tyr Gln
        115                 120                 125

aaa aat cca gac aac cac cct gat gct aaa aaa caa ggc gtt tac gcg      432
Lys Asn Pro Asp Asn His Pro Asp Ala Lys Lys Gln Gly Val Tyr Ala
    130                 135                 140

tgg aaa cta aag ctt aat caa aac ggc ttt gtt tat aaa gta gat gaa      480
Trp Lys Leu Lys Leu Asn Gln Asn Gly Phe Val Tyr Lys Val Asp Glu
145                 150                 155                 160

ggc ccc gct cct gat ggc gaa gag tac ttt gct ttt gca cta ctt aat      528
Gly Pro Ala Pro Asp Gly Glu Glu Tyr Phe Ala Phe Ala Leu Leu Asn
                165                 170                 175

gcg tca gct cgt tgg ggt aat tcg ggg gag ttt aac tac tac aac gat      576
Ala Ser Ala Arg Trp Gly Asn Ser Gly Glu Phe Asn Tyr Tyr Asn Asp
            180                 185                 190

gct att atc atg tta aat aca att aaa aat aag ctg atg gat aac caa      624
Ala Ile Ile Met Leu Asn Thr Ile Lys Asn Lys Leu Met Asp Asn Gln
        195                 200                 205

ata atc cgc ttt tcg cct tac att gat aac tta aca gac cct tct tac      672
Ile Ile Arg Phe Ser Pro Tyr Ile Asp Asn Leu Thr Asp Pro Ser Tyr
    210                 215                 220

cat ata cct gcg ttt tac gac tac ttt gct aat aac gta act aac caa      720

His Ile Pro Ala Phe Tyr Asp Tyr Phe Ala Asn Asn Val Thr Asn Gln
225                 230                 235                 240

gca gat caa act tac tgg cga caa gta gcc aca aaa agt aga acc tta      768
Ala Asp Gln Thr Tyr Trp Arg Gln Val Ala Thr Lys Ser Arg Thr Leu
                245                 250                 255

ctt aaa aac cat ttt acg aaa gta agt gat aat ccg cat tgg aat tta      816
Leu Lys Asn His Phe Thr Lys Val Ser Asp Asn Pro His Trp Asn Leu
            260                 265                 270

cct aca ttt tta tcg cgc tta gat ggc agc cct gtt att ggc tac att      864
Pro Thr Phe Leu Ser Arg Leu Asp Gly Ser Pro Val Ile Gly Tyr Ile
        275                 280                 285

ttt aac ggc caa gca aac cca ggt caa tgg tat gaa ttt gat gca tgg      912
Phe Asn Gly Gln Ala Asn Pro Gly Gln Trp Tyr Glu Phe Asp Ala Trp
    290                 295                 300

cgc gta att atg aat gtg ggt tta gac gca cat tta atg ggt gct caa      960
Arg Val Ile Met Asn Val Gly Leu Asp Ala His Leu Met Gly Ala Gln
305                 310                 315                 320

gca tgg cat aaa agt gca gta aat aaa gca tta ggc ttt tta agt tat     1008
Ala Trp His Lys Ser Ala Val Asn Lys Ala Leu Gly Phe Leu Ser Tyr
                325                 330                 335

gca aaa aca aac aac agt aaa tac tgt tac gag caa gtg tat tcg tac     1056
Ala Lys Thr Asn Asn Ser Lys Tyr Cys Tyr Glu Gln Val Tyr Ser Tyr
            340                 345                 350

ggt gga gcg caa aac aaa ggc tgt gca ggc gaa ggt caa aaa gcc gcg     1104
Gly Gly Ala Gln Asn Lys Gly Cys Ala Gly Glu Gly Gln Lys Ala Ala
        355                 360                 365

aat gca gta gcg tta ctt gct tca aca aac gct ggg caa gca aat gaa     1152
Asn Ala Val Ala Leu Leu Ala Ser Thr Asn Ala Gly Gln Ala Asn Glu
    370                 375                 380

ttt ttt aac gag ttt tgg tct tta tcg caa cca aca ggt agc tac cgt     1200
Phe Phe Asn Glu Phe Trp Ser Leu Ser Gln Pro Thr Gly Ser Tyr Arg
385                 390                 395                 400

tac tac aat ggt tcg tta tat atg tta gct atg ctg cat gta tcg ggc     1248
Tyr Tyr Asn Gly Ser Leu Tyr Met Leu Ala Met Leu His Val Ser Gly
                405                 410                 415

aat ttt aag ttt tat aac aac acg ttt aat taa                         1281
Asn Phe Lys Phe Tyr Asn Asn Thr Phe Asn
            420                 425

<210>  10
<211>  426
<212>  PRT
<213>  strain Sp.10.1

<400>  10

Met Lys Val Phe Phe Lys Ile Thr Thr Leu Leu Leu Ile Leu Ile Ser
1               5                   10                  15

Tyr Gln Ser Leu Ala Ala Phe Asn Asn Asn Pro Ser Ser Val Gly Ala
            20                  25                  30

Asp Ser Ser Gly Ala Tyr Arg Asn Leu Ala Gln Glu Met Gly Lys Thr

35                  40                  45

Asn Ile Gln Gln Lys Val Asn Ser Thr Phe Asp Asn Met Phe Gly Tyr
    50                  55                  60

Asn Asn Thr Gln Gln Leu Tyr Tyr Pro Tyr Thr Glu Asn Gly Val Tyr
65                  70                  75                  80

Lys Ala His Tyr Ile Lys Ala Ile Asn Pro Asp Glu Gly Asp Asp Ile
                85                  90                  95

Arg Thr Glu Gly Gln Ser Trp Gly Met Thr Ala Ala Val Met Leu Asn
            100                 105                 110

Lys Gln Glu Glu Phe Asp Asn Leu Trp Arg Phe Ala Lys Ala Tyr Gln
        115                 120                 125

Lys Asn Pro Asp Asn His Pro Asp Ala Lys Lys Gln Gly Val Tyr Ala
    130                 135                 140

Trp Lys Leu Lys Leu Asn Gln Asn Gly Phe Val Tyr Lys Val Asp Glu
145                 150                 155                 160

Gly Pro Ala Pro Asp Gly Glu Glu Tyr Phe Ala Phe Ala Leu Leu Asn
                165                 170                 175

Ala Ser Ala Arg Trp Gly Asn Ser Gly Glu Phe Asn Tyr Tyr Asn Asp
            180                 185                 190

Ala Ile Ile Met Leu Asn Thr Ile Lys Asn Lys Leu Met Asp Asn Gln
        195                 200                 205

Ile Ile Arg Phe Ser Pro Tyr Ile Asp Asn Leu Thr Asp Pro Ser Tyr
    210                 215                 220

His Ile Pro Ala Phe Tyr Asp Tyr Phe Ala Asn Asn Val Thr Asn Gln
225                 230                 235                 240

Ala Asp Gln Thr Tyr Trp Arg Gln Val Ala Thr Lys Ser Arg Thr Leu
                245                 250                 255

Leu Lys Asn His Phe Thr Lys Val Ser Asp Asn Pro His Trp Asn Leu
            260                 265                 270

Pro Thr Phe Leu Ser Arg Leu Asp Gly Ser Pro Val Ile Gly Tyr Ile
        275                 280                 285

Phe Asn Gly Gln Ala Asn Pro Gly Gln Trp Tyr Glu Phe Asp Ala Trp
    290                 295                 300

Arg Val Ile Met Asn Val Gly Leu Asp Ala His Leu Met Gly Ala Gln

305                 310                 315                 320

Ala Trp His Lys Ser Ala Val Asn Lys Ala Leu Gly Phe Leu Ser Tyr
                325                 330                 335

Ala Lys Thr Asn Asn Ser Lys Tyr Cys Tyr Glu Gln Val Tyr Ser Tyr
            340                 345                 350

Gly Gly Ala Gln Asn Lys Gly Cys Ala Gly Glu Gly Gln Lys Ala Ala
        355                 360                 365

Asn Ala Val Ala Leu Leu Ala Ser Thr Asn Ala Gly Gln Ala Asn Glu
    370                 375                 380

Phe Phe Asn Glu Phe Trp Ser Leu Ser Gln Pro Thr Gly Ser Tyr Arg
385                 390                 395                 400

Tyr Tyr Asn Gly Ser Leu Tyr Met Leu Ala Met Leu His Val Ser Gly
                405                 410                 415

Asn Phe Lys Phe Tyr Asn Asn Thr Phe Asn
            420                 425

<210>  11
<211>  1281
<212>  DNA
<213>  strain Sp.10.2

<220>
<221>  CDS
<222>  (1)..(1281)

<400>  11
atg aaa gta ttt ttt aaa ata aca act tta ttg tta aca cta ata agc      48
Met Lys Val Phe Phe Lys Ile Thr Thr Leu Leu Leu Thr Leu Ile Ser
1               5                   10                  15

tat caa tca ctt gct gca ttt aat agt aac cca tcg agt gta ggc gcc      96
Tyr Gln Ser Leu Ala Ala Phe Asn Ser Asn Pro Ser Ser Val Gly Ala
            20                  25                  30

gac agt tca gga gca tac cgt aac ctc gca caa gaa atg ggt aaa aca      144
Asp Ser Ser Gly Ala Tyr Arg Asn Leu Ala Gln Glu Met Gly Lys Thr
        35                  40                  45

aat ata cag caa aaa gtg aat agt act ttt gac aat atg ttt ggc tat      192
Asn Ile Gln Gln Lys Val Asn Ser Thr Phe Asp Asn Met Phe Gly Tyr
    50                  55                  60

aac aac aca caa caa ctt tac tac ccg tac acc gaa aac ggt gtt tat      240
Asn Asn Thr Gln Gln Leu Tyr Tyr Pro Tyr Thr Glu Asn Gly Val Tyr
65                  70                  75                  80

aaa gca cac tac ata aaa gca att aac cca gac gaa ggc gac gat ata      288
Lys Ala His Tyr Ile Lys Ala Ile Asn Pro Asp Glu Gly Asp Asp Ile
                85                  90                  95

aga aca gaa ggg caa tcg tgg gga atg acc gct gct gtc atg ctt aat      336
Arg Thr Glu Gly Gln Ser Trp Gly Met Thr Ala Ala Val Met Leu Asn

            100                 105                 110

aaa caa gaa gaa ttt gat aac cta tgg cgc ttt gca aaa gcg tat caa 384
Lys Gln Glu Glu Phe Asp Asn Leu Trp Arg Phe Ala Lys Ala Tyr Gln
        115                 120                 125

aaa aat cca gac aac cac cct gat gct aaa aaa caa ggc gtt tac gcg 432
Lys Asn Pro Asp Asn His Pro Asp Ala Lys Lys Gln Gly Val Tyr Ala
    130                 135                 140

tgg aaa cta aag ctt aat caa aac ggc ttt gtt tat aaa gta gat gaa 480
Trp Lys Leu Lys Leu Asn Gln Asn Gly Phe Val Tyr Lys Val Asp Glu
145                 150                 155                 160

ggc ccc gct ccg gat ggc gaa gag tac ttt gct ttt gca cta ctt aat 528
Gly Pro Ala Pro Asp Gly Glu Glu Tyr Phe Ala Phe Ala Leu Leu Asn
                165                 170                 175

gcg tca gct cgt tgg ggt aat tcg ggg gag ttt aac tac tac aac gat 576
Ala Ser Ala Arg Trp Gly Asn Ser Gly Glu Phe Asn Tyr Tyr Asn Asp
            180                 185                 190

gct att atc atg tta aat aca att aaa aat aag ctg atg gaa aac caa 624
Ala Ile Ile Met Leu Asn Thr Ile Lys Asn Lys Leu Met Glu Asn Gln
        195                 200                 205

ata atc cgc ttt tcg cct tac att gat aac tta aca gac cct tct tac 672
Ile Ile Arg Phe Ser Pro Tyr Ile Asp Asn Leu Thr Asp Pro Ser Tyr
    210                 215                 220

cat ata cct gcg ttt tac gac tac ttt gct aat aac gta act aac caa 720
His Ile Pro Ala Phe Tyr Asp Tyr Phe Ala Asn Asn Val Thr Asn Gln
225                 230                 235                 240

gca gac aaa aat tac tgg cga caa gta gcc aca aaa agt aga acc tta 768
Ala Asp Lys Asn Tyr Trp Arg Gln Val Ala Thr Lys Ser Arg Thr Leu
                245                 250                 255

ctt aaa aac cat ttt acg aaa gta agt gat aat ccg cat tgg aat tta 816
Leu Lys Asn His Phe Thr Lys Val Ser Asp Asn Pro His Trp Asn Leu
            260                 265                 270

cct aca ttt tta tcg cgc tta gat ggc agc cct gtt att ggc tac att 864
Pro Thr Phe Leu Ser Arg Leu Asp Gly Ser Pro Val Ile Gly Tyr Ile
        275                 280                 285

ttt aac ggc caa gca aac cca ggt caa tgg tat gaa ttt gat gca tgg 912
Phe Asn Gly Gln Ala Asn Pro Gly Gln Trp Tyr Glu Phe Asp Ala Trp
    290                 295                 300

cgc gta att atg aat gtg ggt tta gac gcg cat tta atg ggt gct caa 960
Arg Val Ile Met Asn Val Gly Leu Asp Ala His Leu Met Gly Ala Gln
305                 310                 315                 320

gca tgg cat aaa agt gca gta aat aaa gca tta ggc ttt tta agt tat 1008
Ala Trp His Lys Ser Ala Val Asn Lys Ala Leu Gly Phe Leu Ser Tyr
                325                 330                 335

gca aaa aca aac aac agt aaa aac tgt tac gag caa gtg tat tcg tac 1056
Ala Lys Thr Asn Asn Ser Lys Asn Cys Tyr Glu Gln Val Tyr Ser Tyr
            340                 345                 350

ggt gga gcg caa aac aaa ggt tgt gca ggc gaa ggt caa aaa gcc gcg 1104
Gly Gly Ala Gln Asn Lys Gly Cys Ala Gly Glu Gly Gln Lys Ala Ala
        355                 360                 365

aat gca gta gcg tta ctt gct tca aca aat gct ggg caa gca aat gaa 1152
Asn Ala Val Ala Leu Leu Ala Ser Thr Asn Ala Gly Gln Ala Asn Glu

370                 375                 380

ttt ttt aac gag ttt tgg tct tta tcg caa cca aca ggt aac tac cgt      1200
Phe Phe Asn Glu Phe Trp Ser Leu Ser Gln Pro Thr Gly Asn Tyr Arg
385                 390                 395                 400

tac tac aat ggt tcg ctg tat atg tta gct atg ctg cat gtc tcg ggc      1248
Tyr Tyr Asn Gly Ser Leu Tyr Met Leu Ala Met Leu His Val Ser Gly
                405                 410                 415

aat ttt aag ttt tat aac aac acg ttt aat taa                          1281
Asn Phe Lys Phe Tyr Asn Asn Thr Phe Asn
            420                 425


<210>  12
<211>  426
<212>  PRT
<213>  strain Sp.10.2

<400>  12

Met Lys Val Phe Phe Lys Ile Thr Thr Leu Leu Leu Thr Leu Ile Ser
1               5                   10                  15

Tyr Gln Ser Leu Ala Ala Phe Asn Ser Asn Pro Ser Ser Val Gly Ala
            20                  25                  30

Asp Ser Ser Gly Ala Tyr Arg Asn Leu Ala Gln Glu Met Gly Lys Thr
        35                  40                  45

Asn Ile Gln Gln Lys Val Asn Ser Thr Phe Asp Asn Met Phe Gly Tyr
    50                  55                  60

Asn Asn Thr Gln Gln Leu Tyr Tyr Pro Tyr Thr Glu Asn Gly Val Tyr
65                  70                  75                  80

Lys Ala His Tyr Ile Lys Ala Ile Asn Pro Asp Glu Gly Asp Asp Ile
                85                  90                  95

Arg Thr Glu Gly Gln Ser Trp Gly Met Thr Ala Ala Val Met Leu Asn
            100                 105                 110

Lys Gln Glu Glu Phe Asp Asn Leu Trp Arg Phe Ala Lys Ala Tyr Gln
        115                 120                 125

Lys Asn Pro Asp Asn His Pro Asp Ala Lys Lys Gln Gly Val Tyr Ala
    130                 135                 140

Trp Lys Leu Lys Leu Asn Gln Asn Gly Phe Val Tyr Lys Val Asp Glu
145                 150                 155                 160

Gly Pro Ala Pro Asp Gly Glu Glu Tyr Phe Ala Phe Ala Leu Leu Asn
                165                 170                 175

Ala Ser Ala Arg Trp Gly Asn Ser Gly Glu Phe Asn Tyr Tyr Asn Asp
            180                 185                 190

Ala Ile Ile Met Leu Asn Thr Ile Lys Asn Lys Leu Met Glu Asn Gln
        195                 200                 205

Ile Ile Arg Phe Ser Pro Tyr Ile Asp Asn Leu Thr Asp Pro Ser Tyr
    210                 215                 220

His Ile Pro Ala Phe Tyr Asp Tyr Phe Ala Asn Asn Val Thr Asn Gln
225                 230                 235                 240

Ala Asp Lys Asn Tyr Trp Arg Gln Val Ala Thr Lys Ser Arg Thr Leu
                245                 250                 255

Leu Lys Asn His Phe Thr Lys Val Ser Asp Asn Pro His Trp Asn Leu
            260                 265                 270

Pro Thr Phe Leu Ser Arg Leu Asp Gly Ser Pro Val Ile Gly Tyr Ile
        275                 280                 285

Phe Asn Gly Gln Ala Asn Pro Gly Gln Trp Tyr Glu Phe Asp Ala Trp
    290                 295                 300

Arg Val Ile Met Asn Val Gly Leu Asp Ala His Leu Met Gly Ala Gln
305                 310                 315                 320

Ala Trp His Lys Ser Ala Val Asn Lys Ala Leu Gly Phe Leu Ser Tyr
                325                 330                 335

Ala Lys Thr Asn Asn Ser Lys Asn Cys Tyr Glu Gln Val Tyr Ser Tyr
            340                 345                 350

Gly Gly Ala Gln Asn Lys Gly Cys Ala Gly Glu Gly Gln Lys Ala Ala
        355                 360                 365

Asn Ala Val Ala Leu Leu Ala Ser Thr Asn Ala Gly Gln Ala Asn Glu
    370                 375                 380

Phe Phe Asn Glu Phe Trp Ser Leu Ser Gln Pro Thr Gly Asn Tyr Arg
385                 390                 395                 400

Tyr Tyr Asn Gly Ser Leu Tyr Met Leu Ala Met Leu His Val Ser Gly
                405                 410                 415

Asn Phe Lys Phe Tyr Asn Asn Thr Phe Asn
            420                 425

<210>  13
<211>  1281
<212>  DNA
<213>  strain Sp.11.2

<220>
<221> CDS
<222> (1)..(1281)

<400> 13
atg aaa gta ttt ttt aaa ata aca act tta ttg tta acg cta ata agc 48
Met Lys Val Phe Phe Lys Ile Thr Thr Leu Leu Leu Thr Leu Ile Ser
1               5                   10                  15

tat caa tca ctt gct gca ttt aat aat aac cca tcg agt gta ggc gcc 96
Tyr Gln Ser Leu Ala Ala Phe Asn Asn Asn Pro Ser Ser Val Gly Ala
            20                  25                  30

gac agt tca ggg aca tac cgt aac ctc gca caa gaa atg ggt aaa aca 144
Asp Ser Ser Gly Thr Tyr Arg Asn Leu Ala Gln Glu Met Gly Lys Thr
        35                  40                  45

aat ata cag caa aag gtg aat agt act ttt gac aat atg ttt ggc tat 192
Asn Ile Gln Gln Lys Val Asn Ser Thr Phe Asp Asn Met Phe Gly Tyr
    50                  55                  60

aac aac act caa caa ctt tac tac ccg tac acc gaa aac ggt gtt tat 240
Asn Asn Thr Gln Gln Leu Tyr Tyr Pro Tyr Thr Glu Asn Gly Val Tyr
65                  70                  75                  80

aaa gca cac tac ata aaa gca att aac cca gac gaa ggc gac gat ata 288
Lys Ala His Tyr Ile Lys Ala Ile Asn Pro Asp Glu Gly Asp Asp Ile
                85                  90                  95

aga aca gaa ggg caa tcg tgg gga atg acc gcc gct gtc atg ctt aac 336
Arg Thr Glu Gly Gln Ser Trp Gly Met Thr Ala Ala Val Met Leu Asn
            100                 105                 110

aaa caa gaa gaa ttt gat aac cta tgg cgc ttt gca aaa gcg tat caa 384
Lys Gln Glu Glu Phe Asp Asn Leu Trp Arg Phe Ala Lys Ala Tyr Gln
        115                 120                 125

aaa aat cca gac aat cac cct gat gct aaa aaa caa ggc gtt tac gcg 432
Lys Asn Pro Asp Asn His Pro Asp Ala Lys Lys Gln Gly Val Tyr Ala
    130                 135                 140

tgg aaa cta aag ctt aat caa aac ggc ttt gtt tat aaa gtg gat gag 480
Trp Lys Leu Lys Leu Asn Gln Asn Gly Phe Val Tyr Lys Val Asp Glu
145                 150                 155                 160

ggc ccc gct ccc gat ggt gaa gag tac ttt gcg ttt gca cta ctt aat 528
Gly Pro Ala Pro Asp Gly Glu Glu Tyr Phe Ala Phe Ala Leu Leu Asn
                165                 170                 175

gcc tct gct cgt tgg ggg aat tcg ggt gag ttt aac tac tac aac gat 576
Ala Ser Ala Arg Trp Gly Asn Ser Gly Glu Phe Asn Tyr Tyr Asn Asp
            180                 185                 190

gcc att acc atg tta aac aca att aaa aat aag ctg atg gaa aac caa 624
Ala Ile Thr Met Leu Asn Thr Ile Lys Asn Lys Leu Met Glu Asn Gln
        195                 200                 205

ata atc cgc ttt tca cct tat tta gat aac tta aca gat cct tca tac 672
Ile Ile Arg Phe Ser Pro Tyr Leu Asp Asn Leu Thr Asp Pro Ser Tyr
    210                 215                 220

cat ata cct gcg ttt tat gac tac ttt gca aat aac gta act aac caa 720
His Ile Pro Ala Phe Tyr Asp Tyr Phe Ala Asn Asn Val Thr Asn Gln
225                 230                 235                 240

gca gac aaa act tac tgg cga caa gta gcc aca aaa agt aga acc tta 768
Ala Asp Lys Thr Tyr Trp Arg Gln Val Ala Thr Lys Ser Arg Thr Leu
                245                 250                 255

ctt aaa aac cat ttt aca aaa gta agt ggt agc ccg cat tgg aac tta 816
Leu Lys Asn His Phe Thr Lys Val Ser Gly Ser Pro His Trp Asn Leu
            260                 265                 270

cct aca ttt tta tcg cgc tta gat ggc agc cct gtt att ggc tac att 864
Pro Thr Phe Leu Ser Arg Leu Asp Gly Ser Pro Val Ile Gly Tyr Ile
        275                 280                 285

ttt aac gac caa gca aac cca ggt caa tgg tat gaa ttt gat gca tgg 912
Phe Asn Asp Gln Ala Asn Pro Gly Gln Trp Tyr Glu Phe Asp Ala Trp
    290                 295                 300

cgc gta att atg aat gtg ggt tta gac gcg cat tta atg ggt gct caa 960
Arg Val Ile Met Asn Val Gly Leu Asp Ala His Leu Met Gly Ala Gln
305                 310                 315                 320

gcg tgg cat aaa agc gca gta aat aaa gca ctg ggc ttt tta agt tat 1008
Ala Trp His Lys Ser Ala Val Asn Lys Ala Leu Gly Phe Leu Ser Tyr
                325                 330                 335

gca aaa aca aac aac agt aaa aac tgc tac gag caa gtg tat tca tac 1056
Ala Lys Thr Asn Asn Ser Lys Asn Cys Tyr Glu Gln Val Tyr Ser Tyr
            340                 345                 350

ggt gga gcg caa aac aaa ggc tgt gca ggc gaa ggt caa aaa gcc gcg 1104
Gly Gly Ala Gln Asn Lys Gly Cys Ala Gly Glu Gly Gln Lys Ala Ala
        355                 360                 365

aat gca gta gcg tta ctt gct tca aca aac gct ggg caa gca aat gaa 1152
Asn Ala Val Ala Leu Leu Ala Ser Thr Asn Ala Gly Gln Ala Asn Glu
    370                 375                 380

ttt ttt aac gag ttt tgg tct tta tcg caa cca aca ggt agc tac cgt 1200
Phe Phe Asn Glu Phe Trp Ser Leu Ser Gln Pro Thr Gly Ser Tyr Arg
385                 390                 395                 400

tac tac aat ggt tcg tta tat atg tta gct atg ctg cat gta tcg ggc 1248
Tyr Tyr Asn Gly Ser Leu Tyr Met Leu Ala Met Leu His Val Ser Gly
                405                 410                 415

aat ttt aag ttt tat aac aac acg ttt aat taa 1281
Asn Phe Lys Phe Tyr Asn Asn Thr Phe Asn
            420                 425


<210> 14
<211> 426
<212> PRT
<213> strain Sp.11.2

<400> 14

Met Lys Val Phe Phe Lys Ile Thr Thr Leu Leu Leu Thr Leu Ile Ser
1               5                   10                  15


Tyr Gln Ser Leu Ala Ala Phe Asn Asn Asn Pro Ser Ser Val Gly Ala
            20                  25                  30


Asp Ser Ser Gly Thr Tyr Arg Asn Leu Ala Gln Glu Met Gly Lys Thr
        35                  40                  45


Asn Ile Gln Gln Lys Val Asn Ser Thr Phe Asp Asn Met Phe Gly Tyr
    50                  55                  60

Asn Asn Thr Gln Gln Leu Tyr Tyr Pro Tyr Thr Glu Asn Gly Val Tyr
65                  70                  75                  80

Lys Ala His Tyr Ile Lys Ala Ile Asn Pro Asp Glu Gly Asp Asp Ile
                85                  90                  95

Arg Thr Glu Gly Gln Ser Trp Gly Met Thr Ala Ala Val Met Leu Asn
            100                 105                 110

Lys Gln Glu Glu Phe Asp Asn Leu Trp Arg Phe Ala Lys Ala Tyr Gln
        115                 120                 125

Lys Asn Pro Asp Asn His Pro Asp Ala Lys Lys Gln Gly Val Tyr Ala
    130                 135                 140

Trp Lys Leu Lys Leu Asn Gln Asn Gly Phe Val Tyr Lys Val Asp Glu
145                 150                 155                 160

Gly Pro Ala Pro Asp Gly Glu Glu Tyr Phe Ala Phe Ala Leu Leu Asn
                165                 170                 175

Ala Ser Ala Arg Trp Gly Asn Ser Gly Glu Phe Asn Tyr Tyr Asn Asp
            180                 185                 190

Ala Ile Thr Met Leu Asn Thr Ile Lys Asn Lys Leu Met Glu Asn Gln
        195                 200                 205

Ile Ile Arg Phe Ser Pro Tyr Leu Asp Asn Leu Thr Asp Pro Ser Tyr
    210                 215                 220

His Ile Pro Ala Phe Tyr Asp Tyr Phe Ala Asn Asn Val Thr Asn Gln
225                 230                 235                 240

Ala Asp Lys Thr Tyr Trp Arg Gln Val Ala Thr Lys Ser Arg Thr Leu
                245                 250                 255

Leu Lys Asn His Phe Thr Lys Val Ser Gly Ser Pro His Trp Asn Leu
            260                 265                 270

Pro Thr Phe Leu Ser Arg Leu Asp Gly Ser Pro Val Ile Gly Tyr Ile
        275                 280                 285

Phe Asn Asp Gln Ala Asn Pro Gly Gln Trp Tyr Glu Phe Asp Ala Trp
    290                 295                 300

Arg Val Ile Met Asn Val Gly Leu Asp Ala His Leu Met Gly Ala Gln
305                 310                 315                 320

Ala Trp His Lys Ser Ala Val Asn Lys Ala Leu Gly Phe Leu Ser Tyr
                325                 330                 335

Ala Lys Thr Asn Asn Ser Lys Asn Cys Tyr Glu Gln Val Tyr Ser Tyr
            340                 345                 350

Gly Gly Ala Gln Asn Lys Gly Cys Ala Gly Glu Gly Gln Lys Ala Ala
        355                 360                 365

Asn Ala Val Ala Leu Leu Ala Ser Thr Asn Ala Gly Gln Ala Asn Glu
    370                 375                 380

Phe Phe Asn Glu Phe Trp Ser Leu Ser Gln Pro Thr Gly Ser Tyr Arg
385                 390                 395                 400

Tyr Tyr Asn Gly Ser Leu Tyr Met Leu Ala Met Leu His Val Ser Gly
                405                 410                 415

Asn Phe Lys Phe Tyr Asn Asn Thr Phe Asn
            420                 425

<210>  15
<211>  1281
<212>  DNA
<213>  strain Sp.23.2

<220>
<221>  CDS
<222>  (1)..(1281)

<400>  15
atg aaa gta ttt ttt aaa ata aca act tta ttg tta ata cta ata agc      48
Met Lys Val Phe Phe Lys Ile Thr Thr Leu Leu Leu Ile Leu Ile Ser
1               5                   10                  15

tat caa tca ctt gct gca ttt aat aat aac cca tcg agt gta ggc gcc      96
Tyr Gln Ser Leu Ala Ala Phe Asn Asn Asn Pro Ser Ser Val Gly Ala
            20                  25                  30

tac agt tca ggg aca tac cgt aac ctc gca caa gaa atg ggt aaa aca     144
Tyr Ser Ser Gly Thr Tyr Arg Asn Leu Ala Gln Glu Met Gly Lys Thr
        35                  40                  45

aat ata cag caa aaa gtg aat agt act ttt gac aat atg ttt ggc tat     192
Asn Ile Gln Gln Lys Val Asn Ser Thr Phe Asp Asn Met Phe Gly Tyr
    50                  55                  60

aac aac aca caa caa ctt tac tac ccg tac acc gaa aac ggt gtt tat     240
Asn Asn Thr Gln Gln Leu Tyr Tyr Pro Tyr Thr Glu Asn Gly Val Tyr
65                  70                  75                  80

aaa gca cac tac ata aaa gca att aac cca gac gaa ggc gac gat ata     288
Lys Ala His Tyr Ile Lys Ala Ile Asn Pro Asp Glu Gly Asp Asp Ile
                85                  90                  95

aga aca gaa ggg caa tcg tgg gga atg act gcc gct atc atg ctt aat     336
Arg Thr Glu Gly Gln Ser Trp Gly Met Thr Ala Ala Ile Met Leu Asn
            100                 105                 110

aaa caa gaa gaa ttt gat aat cta tgg cgc ttt gca aaa gcg tat caa     384
Lys Gln Glu Glu Phe Asp Asn Leu Trp Arg Phe Ala Lys Ala Tyr Gln
        115                 120                 125

aaa aat cca gac aac cac cct gat gta aaa aaa caa ggc gtt tac gcg 432
Lys Asn Pro Asp Asn His Pro Asp Val Lys Lys Gln Gly Val Tyr Ala
    130                 135                 140

tgg aaa cta aag ctt aat caa aac ggc ttt gtt tat aaa gta gat gaa 480
Trp Lys Leu Lys Leu Asn Gln Asn Gly Phe Val Tyr Lys Val Asp Glu
145                 150                 155                 160

ggc ccc gct ccg gat ggc gaa gag tac ttt gcg ttt gca cta ctt aat 528
Gly Pro Ala Pro Asp Gly Glu Glu Tyr Phe Ala Phe Ala Leu Leu Asn
                165                 170                 175

gcc tcc gct cgt tgg ggt aat tca ggg gag ttt aac tac tac aac gat 576
Ala Ser Ala Arg Trp Gly Asn Ser Gly Glu Phe Asn Tyr Tyr Asn Asp
            180                 185                 190

gca att acc atg tta aac aca att aaa aat aag ctg atg gaa aac caa 624
Ala Ile Thr Met Leu Asn Thr Ile Lys Asn Lys Leu Met Glu Asn Gln
        195                 200                 205

ata ata cgc ttt tcg cct tac att gat aac cta aca gac cct tct tac 672
Ile Ile Arg Phe Ser Pro Tyr Ile Asp Asn Leu Thr Asp Pro Ser Tyr
    210                 215                 220

cat ata cct gcg ttt tac gac tac ttt gct aat aac gta act aac caa 720
His Ile Pro Ala Phe Tyr Asp Tyr Phe Ala Asn Asn Val Thr Asn Gln
225                 230                 235                 240

gca gac aaa act tac tgg cga caa gta gcc aca aaa agt aga acc tta 768
Ala Asp Lys Thr Tyr Trp Arg Gln Val Ala Thr Lys Ser Arg Thr Leu
                245                 250                 255

ctc aaa aac cat ttt aca aaa gta agt ggt agc ccg cat tgg aac tta 816
Leu Lys Asn His Phe Thr Lys Val Ser Gly Ser Pro His Trp Asn Leu
            260                 265                 270

cct aca ttt tta tcg cgc tta gat ggc agc cct gtt att ggc tac att 864
Pro Thr Phe Leu Ser Arg Leu Asp Gly Ser Pro Val Ile Gly Tyr Ile
        275                 280                 285

ttt aac ggc caa gca aac cca ggt caa tgg tat gaa ttt gat gca tgc 912
Phe Asn Gly Gln Ala Asn Pro Gly Gln Trp Tyr Glu Phe Asp Ala Cys
    290                 295                 300

cgc gta ttt atg aat gtg ggg tta gag gcg cat ttt atg ggt gct caa 960
Arg Val Phe Met Asn Val Gly Leu Glu Ala His Phe Met Gly Ala Gln
305                 310                 315                 320

gcg tgg cat aaa agt gca gtt aat aaa gca ctg ggc ttt tta agt tat 1008
Ala Trp His Lys Ser Ala Val Asn Lys Ala Leu Gly Phe Leu Ser Tyr
                325                 330                 335

gca aaa aca aac aac agt aaa aac tgt tac gag caa gtg tat tcg tac 1056
Ala Lys Thr Asn Asn Ser Lys Asn Cys Tyr Glu Gln Val Tyr Ser Tyr
            340                 345                 350

ggt gga gcg caa aac aga ggc tgt gca ggc gaa ggt caa aaa gcc gcg 1104
Gly Gly Ala Gln Asn Arg Gly Cys Ala Gly Glu Gly Gln Lys Ala Ala
        355                 360                 365

aat gca gta gcg tta ctt gct tca aca aat gct ggg caa gca aat gag 1152
Asn Ala Val Ala Leu Leu Ala Ser Thr Asn Ala Gly Gln Ala Asn Glu
    370                 375                 380

ttt ttt aac gaa ttt tgg tct tta tcg caa cca acg ggt gac tac cgt 1200
Phe Phe Asn Glu Phe Trp Ser Leu Ser Gln Pro Thr Gly Asp Tyr Arg
385                 390                 395                 400

tac tat aat ggt tcg tta tat atg tta gct atg ctg cat gta tcg ggc      1248
Tyr Tyr Asn Gly Ser Leu Tyr Met Leu Ala Met Leu His Val Ser Gly
                405                 410                 415

aat ttt aag ttt tat aac aac acg ttt aat taa                          1281
Asn Phe Lys Phe Tyr Asn Asn Thr Phe Asn
            420                 425

<210>  16
<211>  426
<212>  PRT
<213>  strain Sp.23.2

<400>  16

Met Lys Val Phe Phe Lys Ile Thr Thr Leu Leu Leu Ile Leu Ile Ser
1               5                   10                  15

Tyr Gln Ser Leu Ala Ala Phe Asn Asn Asn Pro Ser Ser Val Gly Ala
            20                  25                  30

Tyr Ser Ser Gly Thr Tyr Arg Asn Leu Ala Gln Glu Met Gly Lys Thr
        35                  40                  45

Asn Ile Gln Gln Lys Val Asn Ser Thr Phe Asp Asn Met Phe Gly Tyr
    50                  55                  60

Asn Asn Thr Gln Gln Leu Tyr Tyr Pro Tyr Thr Glu Asn Gly Val Tyr
65                  70                  75                  80

Lys Ala His Tyr Ile Lys Ala Ile Asn Pro Asp Glu Gly Asp Asp Ile
                85                  90                  95

Arg Thr Glu Gly Gln Ser Trp Gly Met Thr Ala Ala Ile Met Leu Asn
            100                 105                 110

Lys Gln Glu Glu Phe Asp Asn Leu Trp Arg Phe Ala Lys Ala Tyr Gln
        115                 120                 125

Lys Asn Pro Asp Asn His Pro Asp Val Lys Lys Gln Gly Val Tyr Ala
    130                 135                 140

Trp Lys Leu Lys Leu Asn Gln Asn Gly Phe Val Tyr Lys Val Asp Glu
145                 150                 155                 160

Gly Pro Ala Pro Asp Gly Glu Glu Tyr Phe Ala Phe Ala Leu Leu Asn
                165                 170                 175

Ala Ser Ala Arg Trp Gly Asn Ser Gly Glu Phe Asn Tyr Tyr Asn Asp
            180                 185                 190

Ala Ile Thr Met Leu Asn Thr Ile Lys Asn Lys Leu Met Glu Asn Gln
        195                 200                 205

Ile Ile Arg Phe Ser Pro Tyr Ile Asp Asn Leu Thr Asp Pro Ser Tyr
    210                 215                 220

His Ile Pro Ala Phe Tyr Asp Tyr Phe Ala Asn Asn Val Thr Asn Gln
225                 230                 235                 240

Ala Asp Lys Thr Tyr Trp Arg Gln Val Ala Thr Lys Ser Arg Thr Leu
                245                 250                 255

Leu Lys Asn His Phe Thr Lys Val Ser Gly Ser Pro His Trp Asn Leu
            260                 265                 270

Pro Thr Phe Leu Ser Arg Leu Asp Gly Ser Pro Val Ile Gly Tyr Ile
        275                 280                 285

Phe Asn Gly Gln Ala Asn Pro Gly Gln Trp Tyr Glu Phe Asp Ala Cys
    290                 295                 300

Arg Val Phe Met Asn Val Gly Leu Glu Ala His Phe Met Gly Ala Gln
305                 310                 315                 320

Ala Trp His Lys Ser Ala Val Asn Lys Ala Leu Gly Phe Leu Ser Tyr
                325                 330                 335

Ala Lys Thr Asn Asn Ser Lys Asn Cys Tyr Glu Gln Val Tyr Ser Tyr
            340                 345                 350

Gly Gly Ala Gln Asn Arg Gly Cys Ala Gly Glu Gly Gln Lys Ala Ala
        355                 360                 365

Asn Ala Val Ala Leu Leu Ala Ser Thr Asn Ala Gly Gln Ala Asn Glu
    370                 375                 380

Phe Phe Asn Glu Phe Trp Ser Leu Ser Gln Pro Thr Gly Asp Tyr Arg
385                 390                 395                 400

Tyr Tyr Asn Gly Ser Leu Tyr Met Leu Ala Met Leu His Val Ser Gly
                405                 410                 415

Asn Phe Lys Phe Tyr Asn Asn Thr Phe Asn
            420                 425

<210>   17
<211>   34
<212>   DNA
<213>   sense primer

<400>   17
gggcatatga aagtattttt taaaataaca actt                                34

<210>   18
<211>   33
<212>   DNA

<213>  antisense primer

<400>  18
gggctcgagt taattaaacg tgttgttata aaa                                    33


<210>  19
<211>  162
<212>  DNA
<213>  artificial

<220>
<223>  artificial


<220>
<221>  misc_feature
<222>  (24)..(24)
<223>  n is c or t

<220>
<221>  misc_feature
<222>  (27)..(27)
<223>  n is t or c

<220>
<221>  misc_feature
<222>  (48)..(48)
<223>  n is c or a

<220>
<221>  misc_feature
<222>  (51)..(51)
<223>  n is g or a

<220>
<221>  misc_feature
<222>  (81)..(81)
<223>  n is t or c

<220>
<221>  misc_feature
<222>  (82)..(82)
<223>  n is g or a

<220>
<221>  misc_feature
<222>  (93)..(93)
<223>  n is a or c

<220>
<221>  misc_feature
<222>  (99)..(99)
<223>  n is g or a

<220>
<221>  misc_feature
<222>  (111)..(111)
<223>  n is a or g or c

<220>
<221>  misc_feature
<222>  (115)..(115)
<223>  n is t or g

<220>
<221>  misc_feature
<222>  (135)..(135)

<223>  n is g or a

<220>
<221>  misc_feature
<222>  (139)..(139)
<223>  n is g or a

<220>
<221>  misc_feature
<222>  (140)..(140)
<223>  n is a or g

<400>  19
tacggtggag cgcaaaacaa aggntgngca ggcgaaggtc aaaaagcngc naatgcagta      60

gcgttacttg cttcaacaaa nnctgggcaa gcnaatgant tttttaacga ntttnggtct     120

ttatcgcaac caacnggtnn ctaccgttac tacaatggtt cg                        162


<210>  20
<211>  1218
<212>  DNA
<213>  strain TAH2a


<220>
<221>  CDS
<222>  (1)..(1218)

<400>  20
gca ttt aat aat aat cca tcg agt gta ggc gcc gac agc tcg ggg aca       48
Ala Phe Asn Asn Asn Pro Ser Ser Val Gly Ala Asp Ser Ser Gly Thr
1               5                   10                  15

tac cgt aac ctt gca caa gaa atg ggt aaa acg aac ata cag caa aag       96
Tyr Arg Asn Leu Ala Gln Glu Met Gly Lys Thr Asn Ile Gln Gln Lys
            20                  25                  30

gtg aat agt act ttt aac aat atg ttt gga agt aat aat acc cag caa      144
Val Asn Ser Thr Phe Asn Asn Met Phe Gly Ser Asn Asn Thr Gln Gln
        35                  40                  45

ttg tat acc cat tac tca gaa aat gac gca tat aaa gca cac tac ata      192
Leu Tyr Thr His Tyr Ser Glu Asn Asp Ala Tyr Lys Ala His Tyr Ile
    50                  55                  60

aaa gca att aac cct gac gag ggt gat gat ata aga aca gaa ggg caa      240
Lys Ala Ile Asn Pro Asp Glu Gly Asp Asp Ile Arg Thr Glu Gly Gln
65                  70                  75                  80

tca tgg ggg atg act gct gct gtt atg ctc tac aag caa gaa gaa ttt      288
Ser Trp Gly Met Thr Ala Ala Val Met Leu Tyr Lys Gln Glu Glu Phe
                85                  90                  95

gat aac tta tgg cgc ttt gct aaa gcg tat caa aaa aat cca gac aat      336
Asp Asn Leu Trp Arg Phe Ala Lys Ala Tyr Gln Lys Asn Pro Asp Asn
            100                 105                 110

cac cct gat gta aaa aaa caa ggt gtt tac gcg tgg aaa cta aag ctt      384
His Pro Asp Val Lys Lys Gln Gly Val Tyr Ala Trp Lys Leu Lys Leu
        115                 120                 125

aat caa aac ggt ttt gtt tat aaa gta gat gaa ggc ccg gca cct gat      432
Asn Gln Asn Gly Phe Val Tyr Lys Val Asp Glu Gly Pro Ala Pro Asp
    130                 135                 140

ggt gaa gag tac ttt gct ttt gca cta ctt aat gcg tca gct cgt tgg      480

Gly Glu Glu Tyr Phe Ala Phe Ala Leu Leu Asn Ala Ser Ala Arg Trp
145                 150                 155                 160

ggt aat tcg ggc gaa ttt gat tat tat gct gat gcc att acc atg tta     528
Gly Asn Ser Gly Glu Phe Asp Tyr Tyr Ala Asp Ala Ile Thr Met Leu
                165                 170                 175

aac aca att aaa aac aag ctg atg caa aac caa ata atc cgt ttt tca     576
Asn Thr Ile Lys Asn Lys Leu Met Gln Asn Gln Ile Ile Arg Phe Ser
            180                 185                 190

cct tac att gat aac cta aca gac cct tct tat cat ata cct gcg ttt     624
Pro Tyr Ile Asp Asn Leu Thr Asp Pro Ser Tyr His Ile Pro Ala Phe
        195                 200                 205

tac gac tac ttt gca aat aac gtg act aat caa gcc gat aaa aat tac     672
Tyr Asp Tyr Phe Ala Asn Asn Val Thr Asn Gln Ala Asp Lys Asn Tyr
    210                 215                 220

tgg cga caa gtg gct aca aaa agt aga acc tta ctt aaa aac cat ttt     720
Trp Arg Gln Val Ala Thr Lys Ser Arg Thr Leu Leu Lys Asn His Phe
225                 230                 235                 240

aca aaa gtg aat gat aat ccg cat tgg aac tta cct aca ttt tta tcg     768
Thr Lys Val Asn Asp Asn Pro His Trp Asn Leu Pro Thr Phe Leu Ser
                245                 250                 255

cgc tta gat ggc agc cct gtt att ggt tac att ttt aac ggc caa gca     816
Arg Leu Asp Gly Ser Pro Val Ile Gly Tyr Ile Phe Asn Gly Gln Ala
            260                 265                 270

aac cca ggt caa tgg tat gag ttt gat gca tgg cgc gtt att atg aat     864
Asn Pro Gly Gln Trp Tyr Glu Phe Asp Ala Trp Arg Val Ile Met Asn
        275                 280                 285

gtt ggc tta gac gcg cac tta atg ggt gct cag gcg tgg cat aaa aat     912
Val Gly Leu Asp Ala His Leu Met Gly Ala Gln Ala Trp His Lys Asn
    290                 295                 300

gca gtt aat aaa gca ctg ggc ttt tta agt tat gca aaa aca aac aac     960
Ala Val Asn Lys Ala Leu Gly Phe Leu Ser Tyr Ala Lys Thr Asn Asn
305                 310                 315                 320

agt aaa aac tgc tac gag caa gtg tat tca tac ggt gga gcg caa aac     1008
Ser Lys Asn Cys Tyr Glu Gln Val Tyr Ser Tyr Gly Gly Ala Gln Asn
                325                 330                 335

aaa ggc tgt gca ggc gaa ggt caa aaa gcc gcg aat gca gta gcg tta     1056
Lys Gly Cys Ala Gly Glu Gly Gln Lys Ala Ala Asn Ala Val Ala Leu
            340                 345                 350

ctt gct tca aca aac gct ggg caa gca aat gaa ttt ttt aac gag ttt     1104
Leu Ala Ser Thr Asn Ala Gly Gln Ala Asn Glu Phe Phe Asn Glu Phe
        355                 360                 365

tgg tct tta tcg caa cca aca ggt agc tac cgt tac tac aat ggt tcg     1152
Trp Ser Leu Ser Gln Pro Thr Gly Ser Tyr Arg Tyr Tyr Asn Gly Ser
    370                 375                 380

tta tat atg tta gct atg ctg cat gta tcg ggc aat ttt aag ttt tat     1200
Leu Tyr Met Leu Ala Met Leu His Val Ser Gly Asn Phe Lys Phe Tyr
385                 390                 395                 400

aac aac acg ttt aat taa                                             1218
Asn Asn Thr Phe Asn
                405

<210>  21
<211>  405
<212>  PRT
<213>  strain TAH2a

<400>  21

Ala Phe Asn Asn Asn Pro Ser Ser Val Gly Ala Asp Ser Ser Gly Thr
1               5                   10                  15

Tyr Arg Asn Leu Ala Gln Glu Met Gly Lys Thr Asn Ile Gln Gln Lys
            20                  25                  30

Val Asn Ser Thr Phe Asn Asn Met Phe Gly Ser Asn Asn Thr Gln Gln
        35                  40                  45

Leu Tyr Thr His Tyr Ser Glu Asn Asp Ala Tyr Lys Ala His Tyr Ile
    50                  55                  60

Lys Ala Ile Asn Pro Asp Glu Gly Asp Asp Ile Arg Thr Glu Gly Gln
65                  70                  75                  80

Ser Trp Gly Met Thr Ala Ala Val Met Leu Tyr Lys Gln Glu Glu Phe
                85                  90                  95

Asp Asn Leu Trp Arg Phe Ala Lys Ala Tyr Gln Lys Asn Pro Asp Asn
            100                 105                 110

His Pro Asp Val Lys Lys Gln Gly Val Tyr Ala Trp Lys Leu Lys Leu
        115                 120                 125

Asn Gln Asn Gly Phe Val Tyr Lys Val Asp Glu Gly Pro Ala Pro Asp
    130                 135                 140

Gly Glu Glu Tyr Phe Ala Phe Ala Leu Leu Asn Ala Ser Ala Arg Trp
145                 150                 155                 160

Gly Asn Ser Gly Glu Phe Asp Tyr Tyr Ala Asp Ala Ile Thr Met Leu
                165                 170                 175

Asn Thr Ile Lys Asn Lys Leu Met Gln Asn Gln Ile Ile Arg Phe Ser
            180                 185                 190

Pro Tyr Ile Asp Asn Leu Thr Asp Pro Ser Tyr His Ile Pro Ala Phe
        195                 200                 205

Tyr Asp Tyr Phe Ala Asn Asn Val Thr Asn Gln Ala Asp Lys Asn Tyr
    210                 215                 220

Trp Arg Gln Val Ala Thr Lys Ser Arg Thr Leu Leu Lys Asn His Phe
225                 230                 235                 240

Thr Lys Val Asn Asp Asn Pro His Trp Asn Leu Pro Thr Phe Leu Ser

                245                 250                 255

Arg Leu Asp Gly Ser Pro Val Ile Gly Tyr Ile Phe Asn Gly Gln Ala
            260                 265                 270

Asn Pro Gly Gln Trp Tyr Glu Phe Asp Ala Trp Arg Val Ile Met Asn
        275                 280                 285

Val Gly Leu Asp Ala His Leu Met Gly Ala Gln Ala Trp His Lys Asn
    290                 295                 300

Ala Val Asn Lys Ala Leu Gly Phe Leu Ser Tyr Ala Lys Thr Asn Asn
305                 310                 315                 320

Ser Lys Asn Cys Tyr Glu Gln Val Tyr Ser Tyr Gly Gly Ala Gln Asn
                325                 330                 335

Lys Gly Cys Ala Gly Glu Gly Gln Lys Ala Ala Asn Ala Val Ala Leu
            340                 345                 350

Leu Ala Ser Thr Asn Ala Gly Gln Ala Asn Glu Phe Phe Asn Glu Phe
        355                 360                 365

Trp Ser Leu Ser Gln Pro Thr Gly Ser Tyr Arg Tyr Tyr Asn Gly Ser
    370                 375                 380

Leu Tyr Met Leu Ala Met Leu His Val Ser Gly Asn Phe Lys Phe Tyr
385                 390                 395                 400

Asn Asn Thr Phe Asn
                405

<210> 22
<211> 1218
<212> DNA
<213> strain TAH4a

<220>
<221> CDS
<222> (1)..(1218)

<400> 22
gca ttt aat aat aat cca tcg agt gta ggc gcc gac agc tcg ggg aca 48
Ala Phe Asn Asn Asn Pro Ser Ser Val Gly Ala Asp Ser Ser Gly Thr
1               5                   10                  15

tac cgt aac ctt gca caa gaa atg ggt aaa acg aac ata cag caa aag 96
Tyr Arg Asn Leu Ala Gln Glu Met Gly Lys Thr Asn Ile Gln Gln Lys
            20                  25                  30

gtg aat agt act ttt aac aat atg ttt gga agt aat aat acc cag caa 144
Val Asn Ser Thr Phe Asn Asn Met Phe Gly Ser Asn Asn Thr Gln Gln
        35                  40                  45

ttg tat tac cca tac tca gaa aat gac gca tat aaa aca cac tac ata 192
Leu Tyr Tyr Pro Tyr Ser Glu Asn Asp Ala Tyr Lys Thr His Tyr Ile

50                    55                    60

aaa gca att aac cct gac gag ggt gat gat ata aga aca gaa ggg caa      240
Lys Ala Ile Asn Pro Asp Glu Gly Asp Asp Ile Arg Thr Glu Gly Gln
65                  70                  75                  80

tca tgg ggg atg act gct gct gtt atg ctc tac aag caa gaa gaa ttt      288
Ser Trp Gly Met Thr Ala Ala Val Met Leu Tyr Lys Gln Glu Glu Phe
                85                  90                  95

gat aac tta tgg cgc ttt gct aaa gcg tat caa aaa aat cca gac aat      336
Asp Asn Leu Trp Arg Phe Ala Lys Ala Tyr Gln Lys Asn Pro Asp Asn
            100                 105                 110

cac cct gat gta aaa aaa caa ggt gtt tac gcg tgg aaa cta aag ctt      384
His Pro Asp Val Lys Lys Gln Gly Val Tyr Ala Trp Lys Leu Lys Leu
        115                 120                 125

aat caa aac ggt ttt gtt tat aaa gta gat gaa ggc ccg gca cct gat      432
Asn Gln Asn Gly Phe Val Tyr Lys Val Asp Glu Gly Pro Ala Pro Asp
    130                 135                 140

ggt gaa gag tac ttt gct ttt gca cta ctt aat gcg tca gct cgt tgg      480
Gly Glu Glu Tyr Phe Ala Phe Ala Leu Leu Asn Ala Ser Ala Arg Trp
145                 150                 155                 160

ggt aat tcg ggc gaa ttt gat tat tat gct gat gcc att acc atg tta      528
Gly Asn Ser Gly Glu Phe Asp Tyr Tyr Ala Asp Ala Ile Thr Met Leu
                165                 170                 175

aac aca att aaa aac aag ctg atg caa aac caa ata atc cgt ttt tca      576
Asn Thr Ile Lys Asn Lys Leu Met Gln Asn Gln Ile Ile Arg Phe Ser
            180                 185                 190

cct tac att gat aac cta aca gac cct tct tat cat ata cct gcg ttt      624
Pro Tyr Ile Asp Asn Leu Thr Asp Pro Ser Tyr His Ile Pro Ala Phe
        195                 200                 205

tac gac tac ttt gca aat aac gtg act aat caa gcc gat aaa aat tac      672
Tyr Asp Tyr Phe Ala Asn Asn Val Thr Asn Gln Ala Asp Lys Asn Tyr
    210                 215                 220

tgg cga caa gtg gct aca aaa agt aga acc tta ctt aaa aac cat ttt      720
Trp Arg Gln Val Ala Thr Lys Ser Arg Thr Leu Leu Lys Asn His Phe
225                 230                 235                 240

aca aaa gtg aat gat aat ccg cat tgg aac tta cct aca ttt tta tcg      768
Thr Lys Val Asn Asp Asn Pro His Trp Asn Leu Pro Thr Phe Leu Ser
                245                 250                 255

cgc tta gat ggc agc cct gtt att ggt tac att ttt aac ggc caa gca      816
Arg Leu Asp Gly Ser Pro Val Ile Gly Tyr Ile Phe Asn Gly Gln Ala
            260                 265                 270

aac cca ggt caa tgg tat gag ttt gat gca tgg cgc gtt att atg aat      864
Asn Pro Gly Gln Trp Tyr Glu Phe Asp Ala Trp Arg Val Ile Met Asn
        275                 280                 285

gtt ggc tta gac gcg cac tta atg ggt gct cag gcg tgg cat aaa aat      912
Val Gly Leu Asp Ala His Leu Met Gly Ala Gln Ala Trp His Lys Asn
    290                 295                 300

gca gtt aat aaa gca ctt ggc ttt tta agt tat gca aaa aca aac aac      960
Ala Val Asn Lys Ala Leu Gly Phe Leu Ser Tyr Ala Lys Thr Asn Asn
305                 310                 315                 320

agt aaa aac tgt tac gag caa gtg tat tcg tac ggt gga gcg caa aac     1008
Ser Lys Asn Cys Tyr Glu Gln Val Tyr Ser Tyr Gly Gly Ala Gln Asn

                    325                 330                 335

aaa ggc tgc gca ggc gaa ggt caa aaa gca gca aat gca gta gcg tta      1056
Lys Gly Cys Ala Gly Glu Gly Gln Lys Ala Ala Asn Ala Val Ala Leu
                340                 345                 350

ctt gct tca aca aac act ggg caa gca aat gag ttt ttt aac gac ttt      1104
Leu Ala Ser Thr Asn Thr Gly Gln Ala Asn Glu Phe Phe Asn Asp Phe
            355                 360                 365

tgg tct tta tcg caa cca acg ggt gac tac cgt tac tac aat ggt tcg      1152
Trp Ser Leu Ser Gln Pro Thr Gly Asp Tyr Arg Tyr Tyr Asn Gly Ser
    370                 375                 380

tta tat atg tta gct atg ctg cat gta tcg ggt aac ttt aag ttt tac      1200
Leu Tyr Met Leu Ala Met Leu His Val Ser Gly Asn Phe Lys Phe Tyr
385                 390                 395                 400

aac aac acg ttt aat taa                                              1218
Asn Asn Thr Phe Asn
                405

<210>  23
<211>  405
<212>  PRT
<213>  strain TAH4a

<400>  23

Ala Phe Asn Asn Asn Pro Ser Ser Val Gly Ala Asp Ser Ser Gly Thr
1               5                   10                  15

Tyr Arg Asn Leu Ala Gln Glu Met Gly Lys Thr Asn Ile Gln Gln Lys
            20                  25                  30

Val Asn Ser Thr Phe Asn Asn Met Phe Gly Ser Asn Asn Thr Gln Gln
        35                  40                  45

Leu Tyr Tyr Pro Tyr Ser Glu Asn Asp Ala Tyr Lys Thr His Tyr Ile
    50                  55                  60

Lys Ala Ile Asn Pro Asp Glu Gly Asp Asp Ile Arg Thr Glu Gly Gln
65                  70                  75                  80

Ser Trp Gly Met Thr Ala Ala Val Met Leu Tyr Lys Gln Glu Glu Phe
                85                  90                  95

Asp Asn Leu Trp Arg Phe Ala Lys Ala Tyr Gln Lys Asn Pro Asp Asn
            100                 105                 110

His Pro Asp Val Lys Lys Gln Gly Val Tyr Ala Trp Lys Leu Lys Leu
        115                 120                 125

Asn Gln Asn Gly Phe Val Tyr Lys Val Asp Glu Gly Pro Ala Pro Asp
    130                 135                 140

Gly Glu Glu Tyr Phe Ala Phe Ala Leu Leu Asn Ala Ser Ala Arg Trp
145                 150                 155                 160

Gly Asn Ser Gly Glu Phe Asp Tyr Tyr Ala Asp Ala Ile Thr Met Leu
                165                 170                 175

Asn Thr Ile Lys Asn Lys Leu Met Gln Asn Gln Ile Ile Arg Phe Ser
            180                 185                 190

Pro Tyr Ile Asp Asn Leu Thr Asp Pro Ser Tyr His Ile Pro Ala Phe
        195                 200                 205

Tyr Asp Tyr Phe Ala Asn Asn Val Thr Asn Gln Ala Asp Lys Asn Tyr
    210                 215                 220

Trp Arg Gln Val Ala Thr Lys Ser Arg Thr Leu Leu Lys Asn His Phe
225                 230                 235                 240

Thr Lys Val Asn Asp Asn Pro His Trp Asn Leu Pro Thr Phe Leu Ser
                245                 250                 255

Arg Leu Asp Gly Ser Pro Val Ile Gly Tyr Ile Phe Asn Gly Gln Ala
            260                 265                 270

Asn Pro Gly Gln Trp Tyr Glu Phe Asp Ala Trp Arg Val Ile Met Asn
        275                 280                 285

Val Gly Leu Asp Ala His Leu Met Gly Ala Gln Ala Trp His Lys Asn
    290                 295                 300

Ala Val Asn Lys Ala Leu Gly Phe Leu Ser Tyr Ala Lys Thr Asn Asn
305                 310                 315                 320

Ser Lys Asn Cys Tyr Glu Gln Val Tyr Ser Tyr Gly Gly Ala Gln Asn
                325                 330                 335

Lys Gly Cys Ala Gly Glu Gly Gln Lys Ala Ala Asn Ala Val Ala Leu
            340                 345                 350

Leu Ala Ser Thr Asn Thr Gly Gln Ala Asn Glu Phe Phe Asn Asp Phe
        355                 360                 365

Trp Ser Leu Ser Gln Pro Thr Gly Asp Tyr Arg Tyr Tyr Asn Gly Ser
    370                 375                 380

Leu Tyr Met Leu Ala Met Leu His Val Ser Gly Asn Phe Lys Phe Tyr
385                 390                 395                 400

Asn Asn Thr Phe Asn
                405

<210>  24
<211>  1218

<212>  DNA
<213>  strain TAH7a


<220>
<221>  CDS
<222>  (1)..(1218)

<400>  24
gca ttt aat aat aat cct acg agt gta ggc ccc gca agc ttg ggg aca      48
Ala Phe Asn Asn Asn Pro Thr Ser Val Gly Pro Ala Ser Leu Gly Thr
1               5                   10                  15

tac cgt tac ctt gca caa gaa ttg cgt aaa cgg acc ata cgc caa agc      96
Tyr Arg Tyr Leu Ala Gln Glu Leu Arg Lys Arg Thr Ile Arg Gln Ser
            20                  25                  30

gtg aat agt act ttt aac aat atg ttt gga agt aat aat acc ctt cac      144
Val Asn Ser Thr Phe Asn Asn Met Phe Gly Ser Asn Asn Thr Leu His
        35                  40                  45

ccg tat tac cca tac tca gaa aat gac gca tat aaa gca cac tac ata      192
Pro Tyr Tyr Pro Tyr Ser Glu Asn Asp Ala Tyr Lys Ala His Tyr Ile
    50                  55                  60

aaa gca att aac cct gac gag ggt gat gat ata aga aca gaa ggg caa      240
Lys Ala Ile Asn Pro Asp Glu Gly Asp Asp Ile Arg Thr Glu Gly Gln
65                  70                  75                  80

tca tgg ggg atg act gct gct gtt atg ctc tac aag caa gaa gaa ttt      288
Ser Trp Gly Met Thr Ala Ala Val Met Leu Tyr Lys Gln Glu Glu Phe
                85                  90                  95

gat aac tta tgg cgc ttt gct aaa gcg tat caa aaa aat cca gcc aat      336
Asp Asn Leu Trp Arg Phe Ala Lys Ala Tyr Gln Lys Asn Pro Ala Asn
            100                 105                 110

cac cct gat gta aaa aaa caa ggt gtt tac gcg tgg aaa cta aag ctt      384
His Pro Asp Val Lys Lys Gln Gly Val Tyr Ala Trp Lys Leu Lys Leu
        115                 120                 125

aat caa aac ggt ttt gtt tat aaa gta gat gaa ggc ccg gca cct gat      432
Asn Gln Asn Gly Phe Val Tyr Lys Val Asp Glu Gly Pro Ala Pro Asp
    130                 135                 140

ggt gaa gag tac ttt gct ttt gca cta ctt aat gcg tca gct cgt tgg      480
Gly Glu Glu Tyr Phe Ala Phe Ala Leu Leu Asn Ala Ser Ala Arg Trp
145                 150                 155                 160

ggt aat tcg ggc gaa ttt gat tat tat gct gat gcc att acc atg tta      528
Gly Asn Ser Gly Glu Phe Asp Tyr Tyr Ala Asp Ala Ile Thr Met Leu
                165                 170                 175

aac aca att aaa aac aag ctg atg caa aac caa ata atc cgt ttt tca      576
Asn Thr Ile Lys Asn Lys Leu Met Gln Asn Gln Ile Ile Arg Phe Ser
            180                 185                 190

cct tac att gat aac cta aca gac cct tct tat cat ata cct gcg ttt      624
Pro Tyr Ile Asp Asn Leu Thr Asp Pro Ser Tyr His Ile Pro Ala Phe
        195                 200                 205

tac gac tac ttt gca aat aac gtg act aat caa gcc gat aaa aat tac      672
Tyr Asp Tyr Phe Ala Asn Asn Val Thr Asn Gln Ala Asp Lys Asn Tyr
    210                 215                 220

tgg cga caa gtg gct aca aaa agt aga acc tta ctt aaa aac cat ttt      720
Trp Arg Gln Val Ala Thr Lys Ser Arg Thr Leu Leu Lys Asn His Phe
225                 230                 235                 240

aca aaa gtg aat gat aat ccg cat tgg aac tta cct aca ttt tta tcg 768
Thr Lys Val Asn Asp Asn Pro His Trp Asn Leu Pro Thr Phe Leu Ser
                245                 250                 255

cgc tta gat ggc agc cct gtt att ggt tac att ttt aac ggc caa gca 816
Arg Leu Asp Gly Ser Pro Val Ile Gly Tyr Ile Phe Asn Gly Gln Ala
            260                 265                 270

aac cca ggt caa tgg tat gag ttt gat gca tgg cgc gtt att atg aat 864
Asn Pro Gly Gln Trp Tyr Glu Phe Asp Ala Trp Arg Val Ile Met Asn
        275                 280                 285

gtt ggc tta gac gcg cac tta atg ggt gct cag gcg tgg cat aaa aat 912
Val Gly Leu Asp Ala His Leu Met Gly Ala Gln Ala Trp His Lys Asn
    290                 295                 300

gca gtt aat aaa gca ctt ggc ttt tta agt tat gca aaa aca aac aac 960
Ala Val Asn Lys Ala Leu Gly Phe Leu Ser Tyr Ala Lys Thr Asn Asn
305                 310                 315                 320

agt aaa aac tgt tac gag caa gtg tat tcg tac ggt gga gcg caa aac 1008
Ser Lys Asn Cys Tyr Glu Gln Val Tyr Ser Tyr Gly Gly Ala Gln Asn
                325                 330                 335

aaa ggc tgc gca ggc gaa ggt caa aaa gca gca aat gca gta gcg tta 1056
Lys Gly Cys Ala Gly Glu Gly Gln Lys Ala Ala Asn Ala Val Ala Leu
            340                 345                 350

ctt gct tca aca aac act ggg caa gcc aat gag ttt ttt aac gac ttt 1104
Leu Ala Ser Thr Asn Thr Gly Gln Ala Asn Glu Phe Phe Asn Asp Phe
        355                 360                 365

ggg tct tta tcg caa cca acg ggt gac tac cgt tac tac aat ggt tcg 1152
Gly Ser Leu Ser Gln Pro Thr Gly Asp Tyr Arg Tyr Tyr Asn Gly Ser
    370                 375                 380

tta tat atg tta gct atg ctg cat gta tcg ggg aac ttt aag ttt tac 1200
Leu Tyr Met Leu Ala Met Leu His Val Ser Gly Asn Phe Lys Phe Tyr
385                 390                 395                 400

aac aac acg ttt aat taa 1218
Asn Asn Thr Phe Asn
                405

<210> 25
<211> 405
<212> PRT
<213> strain TAH7a

<400> 25

Ala Phe Asn Asn Asn Pro Thr Ser Val Gly Pro Ala Ser Leu Gly Thr
1               5                   10                  15

Tyr Arg Tyr Leu Ala Gln Glu Leu Arg Lys Arg Thr Ile Arg Gln Ser
            20                  25                  30

Val Asn Ser Thr Phe Asn Asn Met Phe Gly Ser Asn Asn Thr Leu His
        35                  40                  45

Pro Tyr Tyr Pro Tyr Ser Glu Asn Asp Ala Tyr Lys Ala His Tyr Ile
    50                  55                  60

Lys Ala Ile Asn Pro Asp Glu Gly Asp Asp Ile Arg Thr Glu Gly Gln
65                  70                  75                  80

Ser Trp Gly Met Thr Ala Ala Val Met Leu Tyr Lys Gln Glu Glu Phe
                85                  90                  95

Asp Asn Leu Trp Arg Phe Ala Lys Ala Tyr Gln Lys Asn Pro Ala Asn
            100                 105                 110

His Pro Asp Val Lys Lys Gln Gly Val Tyr Ala Trp Lys Leu Lys Leu
        115                 120                 125

Asn Gln Asn Gly Phe Val Tyr Lys Val Asp Glu Gly Pro Ala Pro Asp
    130                 135                 140

Gly Glu Glu Tyr Phe Ala Phe Ala Leu Leu Asn Ala Ser Ala Arg Trp
145                 150                 155                 160

Gly Asn Ser Gly Glu Phe Asp Tyr Tyr Ala Asp Ala Ile Thr Met Leu
                165                 170                 175

Asn Thr Ile Lys Asn Lys Leu Met Gln Asn Gln Ile Ile Arg Phe Ser
            180                 185                 190

Pro Tyr Ile Asp Asn Leu Thr Asp Pro Ser Tyr His Ile Pro Ala Phe
        195                 200                 205

Tyr Asp Tyr Phe Ala Asn Asn Val Thr Asn Gln Ala Asp Lys Asn Tyr
    210                 215                 220

Trp Arg Gln Val Ala Thr Lys Ser Arg Thr Leu Leu Lys Asn His Phe
225                 230                 235                 240

Thr Lys Val Asn Asp Asn Pro His Trp Asn Leu Pro Thr Phe Leu Ser
                245                 250                 255

Arg Leu Asp Gly Ser Pro Val Ile Gly Tyr Ile Phe Asn Gly Gln Ala
            260                 265                 270

Asn Pro Gly Gln Trp Tyr Glu Phe Asp Ala Trp Arg Val Ile Met Asn
        275                 280                 285

Val Gly Leu Asp Ala His Leu Met Gly Ala Gln Ala Trp His Lys Asn
    290                 295                 300

Ala Val Asn Lys Ala Leu Gly Phe Leu Ser Tyr Ala Lys Thr Asn Asn
305                 310                 315                 320

Ser Lys Asn Cys Tyr Glu Gln Val Tyr Ser Tyr Gly Gly Ala Gln Asn
                325                 330                 335

Lys Gly Cys Ala Gly Glu Gly Gln Lys Ala Ala Asn Ala Val Ala Leu
            340                 345                 350

Leu Ala Ser Thr Asn Thr Gly Gln Ala Asn Glu Phe Phe Asn Asp Phe
        355                 360                 365

Gly Ser Leu Ser Gln Pro Thr Gly Asp Tyr Arg Tyr Tyr Asn Gly Ser
    370                 375                 380

Leu Tyr Met Leu Ala Met Leu His Val Ser Gly Asn Phe Lys Phe Tyr
385                 390                 395                 400

Asn Asn Thr Phe Asn
                405

<210>  26
<211>  1218
<212>  DNA
<213>  strain Sp.10.1

<220>
<221>  CDS
<222>  (1)..(1218)

<400>  26
gca ttt aat aat aac cca tcg agt gta ggc gcc gac agt tca gga gca      48
Ala Phe Asn Asn Asn Pro Ser Ser Val Gly Ala Asp Ser Ser Gly Ala
1               5                   10                  15

tac cgt aac ctc gca caa gaa atg ggt aaa aca aat ata cag caa aaa      96
Tyr Arg Asn Leu Ala Gln Glu Met Gly Lys Thr Asn Ile Gln Gln Lys
            20                  25                  30

gtg aat agt act ttt gac aat atg ttt ggc tat aac aac aca caa caa      144
Val Asn Ser Thr Phe Asp Asn Met Phe Gly Tyr Asn Asn Thr Gln Gln
        35                  40                  45

ctt tac tac ccg tac acc gaa aac ggt gtt tat aaa gca cac tac ata      192
Leu Tyr Tyr Pro Tyr Thr Glu Asn Gly Val Tyr Lys Ala His Tyr Ile
    50                  55                  60

aaa gca att aac cct gac gag ggt gat gat ata aga aca gaa ggg caa      240
Lys Ala Ile Asn Pro Asp Glu Gly Asp Asp Ile Arg Thr Glu Gly Gln
65                  70                  75                  80

tcg tgg gga atg acc gcc gct gtc atg ctt aat aaa caa gaa gaa ttt      288
Ser Trp Gly Met Thr Ala Ala Val Met Leu Asn Lys Gln Glu Glu Phe
                85                  90                  95

gat aac cta tgg cgc ttt gca aaa gcg tat caa aaa aat cca gac aac      336
Asp Asn Leu Trp Arg Phe Ala Lys Ala Tyr Gln Lys Asn Pro Asp Asn
            100                 105                 110

cac cct gat gct aaa aaa caa ggc gtt tac gcg tgg aaa cta aag ctt      384
His Pro Asp Ala Lys Lys Gln Gly Val Tyr Ala Trp Lys Leu Lys Leu
        115                 120                 125

aat caa aac ggc ttt gtt tat aaa gta gat gaa ggc ccc gct cct gat      432
Asn Gln Asn Gly Phe Val Tyr Lys Val Asp Glu Gly Pro Ala Pro Asp
    130                 135                 140

ggc gaa gag tac ttt gct ttt gca cta ctt aat gcg tca gct cgt tgg 480
Gly Glu Glu Tyr Phe Ala Phe Ala Leu Leu Asn Ala Ser Ala Arg Trp
145                 150                 155                 160

ggt aat tcg ggg gag ttt aac tac tac aac gat gct att atc atg tta 528
Gly Asn Ser Gly Glu Phe Asn Tyr Tyr Asn Asp Ala Ile Ile Met Leu
                165                 170                 175

aat aca att aaa aat aag ctg atg gat aac caa ata atc cgc ttt tcg 576
Asn Thr Ile Lys Asn Lys Leu Met Asp Asn Gln Ile Ile Arg Phe Ser
            180                 185                 190

cct tac att gat aac tta aca gac cct tct tac cat ata cct gcg ttt 624
Pro Tyr Ile Asp Asn Leu Thr Asp Pro Ser Tyr His Ile Pro Ala Phe
        195                 200                 205

tac gac tac ttt gct aat aac gta act aac caa gca gat caa act tac 672
Tyr Asp Tyr Phe Ala Asn Asn Val Thr Asn Gln Ala Asp Gln Thr Tyr
    210                 215                 220

tgg cga caa gta gcc aca aaa agt aga acc tta ctt aaa aac cat ttt 720
Trp Arg Gln Val Ala Thr Lys Ser Arg Thr Leu Leu Lys Asn His Phe
225                 230                 235                 240

acg aaa gta agt gat aat ccg cat tgg aat tta cct aca ttt tta tcg 768
Thr Lys Val Ser Asp Asn Pro His Trp Asn Leu Pro Thr Phe Leu Ser
                245                 250                 255

cgc tta gat ggc agc cct gtt att ggc tac att ttt aac ggc caa gca 816
Arg Leu Asp Gly Ser Pro Val Ile Gly Tyr Ile Phe Asn Gly Gln Ala
            260                 265                 270

aac cca ggt caa tgg tat gaa ttt gat gca tgg cgc gta att atg aat 864
Asn Pro Gly Gln Trp Tyr Glu Phe Asp Ala Trp Arg Val Ile Met Asn
        275                 280                 285

gtg ggt tta gac gca cat tta atg ggt gct caa gca tgg cat aaa agt 912
Val Gly Leu Asp Ala His Leu Met Gly Ala Gln Ala Trp His Lys Ser
    290                 295                 300

gca gta aat aaa gca tta ggc ttt tta agt tat gca aaa aca aac aac 960
Ala Val Asn Lys Ala Leu Gly Phe Leu Ser Tyr Ala Lys Thr Asn Asn
305                 310                 315                 320

agt aaa tac tgt tac gag caa gtg tat tcg tac ggt gga gcg caa aac 1008
Ser Lys Tyr Cys Tyr Glu Gln Val Tyr Ser Tyr Gly Gly Ala Gln Asn
                325                 330                 335

aaa ggc tgt gca ggc gaa ggt caa aaa gcc gcg aat gca gta gcg tta 1056
Lys Gly Cys Ala Gly Glu Gly Gln Lys Ala Ala Asn Ala Val Ala Leu
            340                 345                 350

ctt gct tca aca aac gct ggg caa gca aat gaa ttt ttt aac gag ttt 1104
Leu Ala Ser Thr Asn Ala Gly Gln Ala Asn Glu Phe Phe Asn Glu Phe
        355                 360                 365

tgg tct tta tcg caa cca aca ggt agc tac cgt tac tac aat ggt tcg 1152
Trp Ser Leu Ser Gln Pro Thr Gly Ser Tyr Arg Tyr Tyr Asn Gly Ser
    370                 375                 380

tta tat atg tta gct atg ctg cat gta tcg ggc aat ttt aag ttt tat 1200
Leu Tyr Met Leu Ala Met Leu His Val Ser Gly Asn Phe Lys Phe Tyr
385                 390                 395                 400

aac aac acg ttt aat taa 1218
Asn Asn Thr Phe Asn
                405

<210>  27
<211>  405
<212>  PRT
<213>  strain Sp.10.1

<400>  27

Ala Phe Asn Asn Asn Pro Ser Ser Val Gly Ala Asp Ser Ser Gly Ala
1               5                   10                  15

Tyr Arg Asn Leu Ala Gln Glu Met Gly Lys Thr Asn Ile Gln Gln Lys
            20                  25                  30

Val Asn Ser Thr Phe Asp Asn Met Phe Gly Tyr Asn Asn Thr Gln Gln
        35                  40                  45

Leu Tyr Tyr Pro Tyr Thr Glu Asn Gly Val Tyr Lys Ala His Tyr Ile
    50                  55                  60

Lys Ala Ile Asn Pro Asp Glu Gly Asp Asp Ile Arg Thr Glu Gly Gln
65                  70                  75                  80

Ser Trp Gly Met Thr Ala Ala Val Met Leu Asn Lys Gln Glu Glu Phe
                85                  90                  95

Asp Asn Leu Trp Arg Phe Ala Lys Ala Tyr Gln Lys Asn Pro Asp Asn
            100                 105                 110

His Pro Asp Ala Lys Lys Gln Gly Val Tyr Ala Trp Lys Leu Lys Leu
        115                 120                 125

Asn Gln Asn Gly Phe Val Tyr Lys Val Asp Glu Gly Pro Ala Pro Asp
    130                 135                 140

Gly Glu Glu Tyr Phe Ala Phe Ala Leu Leu Asn Ala Ser Ala Arg Trp
145                 150                 155                 160

Gly Asn Ser Gly Glu Phe Asn Tyr Tyr Asn Asp Ala Ile Ile Met Leu
                165                 170                 175

Asn Thr Ile Lys Asn Lys Leu Met Asp Asn Gln Ile Ile Arg Phe Ser
            180                 185                 190

Pro Tyr Ile Asp Asn Leu Thr Asp Pro Ser Tyr His Ile Pro Ala Phe
        195                 200                 205

Tyr Asp Tyr Phe Ala Asn Asn Val Thr Asn Gln Ala Asp Gln Thr Tyr
    210                 215                 220

Trp Arg Gln Val Ala Thr Lys Ser Arg Thr Leu Leu Lys Asn His Phe
225                 230                 235                 240

Thr Lys Val Ser Asp Asn Pro His Trp Asn Leu Pro Thr Phe Leu Ser
                245                 250                 255

Arg Leu Asp Gly Ser Pro Val Ile Gly Tyr Ile Phe Asn Gly Gln Ala
            260                 265                 270

Asn Pro Gly Gln Trp Tyr Glu Phe Asp Ala Trp Arg Val Ile Met Asn
        275                 280                 285

Val Gly Leu Asp Ala His Leu Met Gly Ala Gln Ala Trp His Lys Ser
    290                 295                 300

Ala Val Asn Lys Ala Leu Gly Phe Leu Ser Tyr Ala Lys Thr Asn Asn
305                 310                 315                 320

Ser Lys Tyr Cys Tyr Glu Gln Val Tyr Ser Tyr Gly Gly Ala Gln Asn
                325                 330                 335

Lys Gly Cys Ala Gly Glu Gly Gln Lys Ala Ala Asn Ala Val Ala Leu
            340                 345                 350

Leu Ala Ser Thr Asn Ala Gly Gln Ala Asn Glu Phe Phe Asn Glu Phe
        355                 360                 365

Trp Ser Leu Ser Gln Pro Thr Gly Ser Tyr Arg Tyr Tyr Asn Gly Ser
    370                 375                 380

Leu Tyr Met Leu Ala Met Leu His Val Ser Gly Asn Phe Lys Phe Tyr
385                 390                 395                 400

Asn Asn Thr Phe Asn
                405

<210>  28
<211>  1218
<212>  DNA
<213>  strain Sp.10.2

<220>
<221>  CDS
<222>  (1)..(1218)

<400>  28
gca ttt aat agt aac cca tcg agt gta ggc gcc gac agt tca gga gca        48
Ala Phe Asn Ser Asn Pro Ser Ser Val Gly Ala Asp Ser Ser Gly Ala
1               5                   10                  15

tac cgt aac ctc gca caa gaa atg ggt aaa aca aat ata cag caa aaa        96
Tyr Arg Asn Leu Ala Gln Glu Met Gly Lys Thr Asn Ile Gln Gln Lys
            20                  25                  30

gtg aat agt act ttt gac aat atg ttt ggc tat aac aac aca caa caa       144
Val Asn Ser Thr Phe Asp Asn Met Phe Gly Tyr Asn Asn Thr Gln Gln
        35                  40                  45

ctt tac tac ccg tac acc gaa aac ggt gtt tat aaa gca cac tac ata       192

Leu Tyr Tyr Pro Tyr Thr Glu Asn Gly Val Tyr Lys Ala His Tyr Ile
    50                  55                  60

aaa gca att aac cca gac gaa ggc gac gat ata aga aca gaa ggg caa      240
Lys Ala Ile Asn Pro Asp Glu Gly Asp Asp Ile Arg Thr Glu Gly Gln
65                  70                  75                  80

tcg tgg gga atg acc gct gct gtc atg ctt aat aaa caa gaa gaa ttt      288
Ser Trp Gly Met Thr Ala Ala Val Met Leu Asn Lys Gln Glu Glu Phe
                85                  90                  95

gat aac cta tgg cgc ttt gca aaa gcg tat caa aaa aat cca gac aac      336
Asp Asn Leu Trp Arg Phe Ala Lys Ala Tyr Gln Lys Asn Pro Asp Asn
            100                 105                 110

cac cct gat gct aaa aaa caa ggc gtt tac gcg tgg aaa cta aag ctt      384
His Pro Asp Ala Lys Lys Gln Gly Val Tyr Ala Trp Lys Leu Lys Leu
        115                 120                 125

aat caa aac ggc ttt gtt tat aaa gta gat gaa ggc ccc gct ccg gat      432
Asn Gln Asn Gly Phe Val Tyr Lys Val Asp Glu Gly Pro Ala Pro Asp
    130                 135                 140

ggc gaa gag tac ttt gct ttt gca cta ctt aat gcg tca gct cgt tgg      480
Gly Glu Glu Tyr Phe Ala Phe Ala Leu Leu Asn Ala Ser Ala Arg Trp
145                 150                 155                 160

ggt aat tcg ggg gag ttt aac tac tac aac gat gct att atc atg tta      528
Gly Asn Ser Gly Glu Phe Asn Tyr Tyr Asn Asp Ala Ile Ile Met Leu
                165                 170                 175

aat aca att aaa aat aag ctg atg gaa aac caa ata atc cgc ttt tcg      576
Asn Thr Ile Lys Asn Lys Leu Met Glu Asn Gln Ile Ile Arg Phe Ser
            180                 185                 190

cct tac att gat aac tta aca gac cct tct tac cat ata cct gcg ttt      624
Pro Tyr Ile Asp Asn Leu Thr Asp Pro Ser Tyr His Ile Pro Ala Phe
        195                 200                 205

tac gac tac ttt gct aat aac gta act aac caa gca gac aaa aat tac      672
Tyr Asp Tyr Phe Ala Asn Asn Val Thr Asn Gln Ala Asp Lys Asn Tyr
    210                 215                 220

tgg cga caa gta gcc aca aaa agt aga acc tta ctt aaa aac cat ttt      720
Trp Arg Gln Val Ala Thr Lys Ser Arg Thr Leu Leu Lys Asn His Phe
225                 230                 235                 240

acg aaa gta agt gat aat ccg cat tgg aat tta cct aca ttt tta tcg      768
Thr Lys Val Ser Asp Asn Pro His Trp Asn Leu Pro Thr Phe Leu Ser
                245                 250                 255

cgc tta gat ggc agc cct gtt att ggc tac att ttt aac ggc caa gca      816
Arg Leu Asp Gly Ser Pro Val Ile Gly Tyr Ile Phe Asn Gly Gln Ala
            260                 265                 270

aac cca ggt caa tgg tat gaa ttt gat gca tgg cgc gta att atg aat      864
Asn Pro Gly Gln Trp Tyr Glu Phe Asp Ala Trp Arg Val Ile Met Asn
        275                 280                 285

gtg ggt tta gac gcg cat tta atg ggt gct caa gca tgg cat aaa agt      912
Val Gly Leu Asp Ala His Leu Met Gly Ala Gln Ala Trp His Lys Ser
    290                 295                 300

gca gta aat aaa gca tta ggc ttt tta agt tat gca aaa aca aac aac      960
Ala Val Asn Lys Ala Leu Gly Phe Leu Ser Tyr Ala Lys Thr Asn Asn
305                 310                 315                 320

agt aaa aac tgt tac gag caa gtg tat tcg tac ggt gga gcg caa aac     1008

Ser Lys Asn Cys Tyr Glu Gln Val Tyr Ser Tyr Gly Gly Ala Gln Asn
                325                 330                 335

aaa ggt tgt gca ggc gaa ggt caa aaa gcc gcg aat gca gta gcg tta      1056
Lys Gly Cys Ala Gly Glu Gly Gln Lys Ala Ala Asn Ala Val Ala Leu
            340                 345                 350

ctt gct tca aca aat gct ggg caa gca aat gaa ttt ttt aac gag ttt      1104
Leu Ala Ser Thr Asn Ala Gly Gln Ala Asn Glu Phe Phe Asn Glu Phe
        355                 360                 365

tgg tct tta tcg caa cca aca ggt aac tac cgt tac tac aat ggt tcg      1152
Trp Ser Leu Ser Gln Pro Thr Gly Asn Tyr Arg Tyr Tyr Asn Gly Ser
    370                 375                 380

ctg tat atg tta gct atg ctg cat gtc tcg ggc aat ttt aag ttt tat      1200
Leu Tyr Met Leu Ala Met Leu His Val Ser Gly Asn Phe Lys Phe Tyr
385                 390                 395                 400

aac aac acg ttt aat taa                                              1218
Asn Asn Thr Phe Asn
                405


<210>  29
<211>  405
<212>  PRT
<213>  strain Sp.10.2

<400>  29

Ala Phe Asn Ser Asn Pro Ser Ser Val Gly Ala Asp Ser Ser Gly Ala
1               5                   10                  15

Tyr Arg Asn Leu Ala Gln Glu Met Gly Lys Thr Asn Ile Gln Gln Lys
            20                  25                  30

Val Asn Ser Thr Phe Asp Asn Met Phe Gly Tyr Asn Asn Thr Gln Gln
        35                  40                  45

Leu Tyr Tyr Pro Tyr Thr Glu Asn Gly Val Tyr Lys Ala His Tyr Ile
    50                  55                  60

Lys Ala Ile Asn Pro Asp Glu Gly Asp Asp Ile Arg Thr Glu Gly Gln
65                  70                  75                  80

Ser Trp Gly Met Thr Ala Ala Val Met Leu Asn Lys Gln Glu Glu Phe
                85                  90                  95

Asp Asn Leu Trp Arg Phe Ala Lys Ala Tyr Gln Lys Asn Pro Asp Asn
            100                 105                 110

His Pro Asp Ala Lys Lys Gln Gly Val Tyr Ala Trp Lys Leu Lys Leu
        115                 120                 125

Asn Gln Asn Gly Phe Val Tyr Lys Val Asp Glu Gly Pro Ala Pro Asp
    130                 135                 140

Gly Glu Glu Tyr Phe Ala Phe Ala Leu Leu Asn Ala Ser Ala Arg Trp

145                 150                 155                 160

Gly Asn Ser Gly Glu Phe Asn Tyr Tyr Asn Asp Ala Ile Ile Met Leu
                165                 170                 175

Asn Thr Ile Lys Asn Lys Leu Met Glu Asn Gln Ile Ile Arg Phe Ser
            180                 185                 190

Pro Tyr Ile Asp Asn Leu Thr Asp Pro Ser Tyr His Ile Pro Ala Phe
        195                 200                 205

Tyr Asp Tyr Phe Ala Asn Asn Val Thr Asn Gln Ala Asp Lys Asn Tyr
    210                 215                 220

Trp Arg Gln Val Ala Thr Lys Ser Arg Thr Leu Leu Lys Asn His Phe
225                 230                 235                 240

Thr Lys Val Ser Asp Asn Pro His Trp Asn Leu Pro Thr Phe Leu Ser
                245                 250                 255

Arg Leu Asp Gly Ser Pro Val Ile Gly Tyr Ile Phe Asn Gly Gln Ala
            260                 265                 270

Asn Pro Gly Gln Trp Tyr Glu Phe Asp Ala Trp Arg Val Ile Met Asn
        275                 280                 285

Val Gly Leu Asp Ala His Leu Met Gly Ala Gln Ala Trp His Lys Ser
    290                 295                 300

Ala Val Asn Lys Ala Leu Gly Phe Leu Ser Tyr Ala Lys Thr Asn Asn
305                 310                 315                 320

Ser Lys Asn Cys Tyr Glu Gln Val Tyr Ser Tyr Gly Gly Ala Gln Asn
                325                 330                 335

Lys Gly Cys Ala Gly Glu Gly Gln Lys Ala Ala Asn Ala Val Ala Leu
            340                 345                 350

Leu Ala Ser Thr Asn Ala Gly Gln Ala Asn Glu Phe Phe Asn Glu Phe
        355                 360                 365

Trp Ser Leu Ser Gln Pro Thr Gly Asn Tyr Arg Tyr Tyr Asn Gly Ser
    370                 375                 380

Leu Tyr Met Leu Ala Met Leu His Val Ser Gly Asn Phe Lys Phe Tyr
385                 390                 395                 400

Asn Asn Thr Phe Asn
                405

<210>  30

<211>  1218
<212>  DNA
<213>  strain Sp.11.2


<220>
<221>  CDS
<222>  (1)..(1218)

<400>  30
gca ttt aat aat aac cca tcg agt gta ggc gcc gac agt tca ggg aca        48
Ala Phe Asn Asn Asn Pro Ser Ser Val Gly Ala Asp Ser Ser Gly Thr
1               5                   10                  15

tac cgt aac ctc gca caa gaa atg ggt aaa aca aat ata cag caa aag        96
Tyr Arg Asn Leu Ala Gln Glu Met Gly Lys Thr Asn Ile Gln Gln Lys
            20                  25                  30

gtg aat agt act ttt gac aat atg ttt ggc tat aac aac act caa caa       144
Val Asn Ser Thr Phe Asp Asn Met Phe Gly Tyr Asn Asn Thr Gln Gln
        35                  40                  45

ctt tac tac ccg tac acc gaa aac ggt gtt tat aaa gca cac tac ata       192
Leu Tyr Tyr Pro Tyr Thr Glu Asn Gly Val Tyr Lys Ala His Tyr Ile
    50                  55                  60

aaa gca att aac cca gac gaa ggc gac gat ata aga aca gaa ggg caa       240
Lys Ala Ile Asn Pro Asp Glu Gly Asp Asp Ile Arg Thr Glu Gly Gln
65                  70                  75                  80

tcg tgg gga atg acc gcc gct gtc atg ctt aac aaa caa gaa gaa ttt       288
Ser Trp Gly Met Thr Ala Ala Val Met Leu Asn Lys Gln Glu Glu Phe
                85                  90                  95

gat aac cta tgg cgc ttt gca aaa gcg tat caa aaa aat cca gac aat       336
Asp Asn Leu Trp Arg Phe Ala Lys Ala Tyr Gln Lys Asn Pro Asp Asn
            100                 105                 110

cac cct gat gct aaa aaa caa ggc gtt tac gcg tgg aaa cta aag ctt       384
His Pro Asp Ala Lys Lys Gln Gly Val Tyr Ala Trp Lys Leu Lys Leu
        115                 120                 125

aat caa aac ggc ttt gtt tat aaa gtg gat gag ggc ccc gct ccc gat       432
Asn Gln Asn Gly Phe Val Tyr Lys Val Asp Glu Gly Pro Ala Pro Asp
    130                 135                 140

ggt gaa gag tac ttt gcg ttt gca cta ctt aat gcc tct gct cgt tgg       480
Gly Glu Glu Tyr Phe Ala Phe Ala Leu Leu Asn Ala Ser Ala Arg Trp
145                 150                 155                 160

ggg aat tcg ggt gag ttt aac tac tac aac gat gcc att acc atg tta       528
Gly Asn Ser Gly Glu Phe Asn Tyr Tyr Asn Asp Ala Ile Thr Met Leu
                165                 170                 175

aac aca att aaa aat aag ctg atg gaa aac caa ata atc cgc ttt tca       576
Asn Thr Ile Lys Asn Lys Leu Met Glu Asn Gln Ile Ile Arg Phe Ser
            180                 185                 190

cct tat tta gat aac tta aca gat cct tca tac cat ata cct gcg ttt       624
Pro Tyr Leu Asp Asn Leu Thr Asp Pro Ser Tyr His Ile Pro Ala Phe
        195                 200                 205

tat gac tac ttt gca aat aac gta act aac caa gca gac aaa act tac       672
Tyr Asp Tyr Phe Ala Asn Asn Val Thr Asn Gln Ala Asp Lys Thr Tyr
    210                 215                 220

tgg cga caa gta gcc aca aaa agt aga acc tta ctt aaa aac cat ttt       720
Trp Arg Gln Val Ala Thr Lys Ser Arg Thr Leu Leu Lys Asn His Phe

225                 230                 235                 240

aca aaa gta agt ggt agc ccg cat tgg aac tta cct aca ttt tta tcg       768
Thr Lys Val Ser Gly Ser Pro His Trp Asn Leu Pro Thr Phe Leu Ser
                245                 250                 255

cgc tta gat ggc agc cct gtt att ggc tac att ttt aac gac caa gca       816
Arg Leu Asp Gly Ser Pro Val Ile Gly Tyr Ile Phe Asn Asp Gln Ala
            260                 265                 270

aac cca ggt caa tgg tat gaa ttt gat gca tgg cgc gta att atg aat       864
Asn Pro Gly Gln Trp Tyr Glu Phe Asp Ala Trp Arg Val Ile Met Asn
        275                 280                 285

gtg ggt tta gac gcg cat tta atg ggt gct caa gcg tgg cat aaa agc       912
Val Gly Leu Asp Ala His Leu Met Gly Ala Gln Ala Trp His Lys Ser
    290                 295                 300

gca gta aat aaa gca ctg ggc ttt tta agt tat gca aaa aca aac aac       960
Ala Val Asn Lys Ala Leu Gly Phe Leu Ser Tyr Ala Lys Thr Asn Asn
305                 310                 315                 320

agt aaa aac tgc tac gag caa gtg tat tca tac ggt gga gcg caa aac      1008
Ser Lys Asn Cys Tyr Glu Gln Val Tyr Ser Tyr Gly Gly Ala Gln Asn
                325                 330                 335

aaa ggc tgt gca ggc gaa ggt caa aaa gcc gcg aat gca gta gcg tta      1056
Lys Gly Cys Ala Gly Glu Gly Gln Lys Ala Ala Asn Ala Val Ala Leu
            340                 345                 350

ctt gct tca aca aac gct ggg caa gca aat gaa ttt ttt aac gag ttt      1104
Leu Ala Ser Thr Asn Ala Gly Gln Ala Asn Glu Phe Phe Asn Glu Phe
        355                 360                 365

tgg tct tta tcg caa cca aca ggt agc tac cgt tac tac aat ggt tcg      1152
Trp Ser Leu Ser Gln Pro Thr Gly Ser Tyr Arg Tyr Tyr Asn Gly Ser
    370                 375                 380

tta tat atg tta gct atg ctg cat gta tcg ggc aat ttt aag ttt tat      1200
Leu Tyr Met Leu Ala Met Leu His Val Ser Gly Asn Phe Lys Phe Tyr
385                 390                 395                 400

aac aac acg ttt aat taa                                              1218
Asn Asn Thr Phe Asn
                405


<210>  31
<211>  405
<212>  PRT
<213>  strain Sp.11.2

<400>  31

Ala Phe Asn Asn Asn Pro Ser Ser Val Gly Ala Asp Ser Ser Gly Thr
1               5                   10                  15


Tyr Arg Asn Leu Ala Gln Glu Met Gly Lys Thr Asn Ile Gln Gln Lys
            20                  25                  30


Val Asn Ser Thr Phe Asp Asn Met Phe Gly Tyr Asn Asn Thr Gln Gln
        35                  40                  45


Leu Tyr Tyr Pro Tyr Thr Glu Asn Gly Val Tyr Lys Ala His Tyr Ile
    50                  55                  60

Lys Ala Ile Asn Pro Asp Glu Gly Asp Asp Ile Arg Thr Glu Gly Gln
65                  70                  75                  80

Ser Trp Gly Met Thr Ala Ala Val Met Leu Asn Lys Gln Glu Glu Phe
                85                  90                  95

Asp Asn Leu Trp Arg Phe Ala Lys Ala Tyr Gln Lys Asn Pro Asp Asn
            100                 105                 110

His Pro Asp Ala Lys Lys Gln Gly Val Tyr Ala Trp Lys Leu Lys Leu
        115                 120                 125

Asn Gln Asn Gly Phe Val Tyr Lys Val Asp Glu Gly Pro Ala Pro Asp
    130                 135                 140

Gly Glu Glu Tyr Phe Ala Phe Ala Leu Leu Asn Ala Ser Ala Arg Trp
145                 150                 155                 160

Gly Asn Ser Gly Glu Phe Asn Tyr Tyr Asn Asp Ala Ile Thr Met Leu
                165                 170                 175

Asn Thr Ile Lys Asn Lys Leu Met Glu Asn Gln Ile Ile Arg Phe Ser
            180                 185                 190

Pro Tyr Leu Asp Asn Leu Thr Asp Pro Ser Tyr His Ile Pro Ala Phe
        195                 200                 205

Tyr Asp Tyr Phe Ala Asn Asn Val Thr Asn Gln Ala Asp Lys Thr Tyr
    210                 215                 220

Trp Arg Gln Val Ala Thr Lys Ser Arg Thr Leu Leu Lys Asn His Phe
225                 230                 235                 240

Thr Lys Val Ser Gly Ser Pro His Trp Asn Leu Pro Thr Phe Leu Ser
                245                 250                 255

Arg Leu Asp Gly Ser Pro Val Ile Gly Tyr Ile Phe Asn Asp Gln Ala
            260                 265                 270

Asn Pro Gly Gln Trp Tyr Glu Phe Asp Ala Trp Arg Val Ile Met Asn
        275                 280                 285

Val Gly Leu Asp Ala His Leu Met Gly Ala Gln Ala Trp His Lys Ser
    290                 295                 300

Ala Val Asn Lys Ala Leu Gly Phe Leu Ser Tyr Ala Lys Thr Asn Asn
305                 310                 315                 320

Ser Lys Asn Cys Tyr Glu Gln Val Tyr Ser Tyr Gly Gly Ala Gln Asn
                325                 330                 335

Lys Gly Cys Ala Gly Glu Gly Gln Lys Ala Ala Asn Ala Val Ala Leu
            340                 345                 350

Leu Ala Ser Thr Asn Ala Gly Gln Ala Asn Glu Phe Phe Asn Glu Phe
        355                 360                 365

Trp Ser Leu Ser Gln Pro Thr Gly Ser Tyr Arg Tyr Tyr Asn Gly Ser
    370                 375                 380

Leu Tyr Met Leu Ala Met Leu His Val Ser Gly Asn Phe Lys Phe Tyr
385                 390                 395                 400

Asn Asn Thr Phe Asn
                405

<210>  32
<211>  1218
<212>  DNA
<213>  strain Sp.23.2

<220>
<221>  CDS
<222>  (1)..(1218)

<400>  32
gca ttt aat aat aac cca tcg agt gta ggc gcc tac agt tca ggg aca      48
Ala Phe Asn Asn Asn Pro Ser Ser Val Gly Ala Tyr Ser Ser Gly Thr
1               5                   10                  15

tac cgt aac ctc gca caa gaa atg ggt aaa aca aat ata cag caa aaa      96
Tyr Arg Asn Leu Ala Gln Glu Met Gly Lys Thr Asn Ile Gln Gln Lys
            20                  25                  30

gtg aat agt act ttt gac aat atg ttt ggc tat aac aac aca caa caa      144
Val Asn Ser Thr Phe Asp Asn Met Phe Gly Tyr Asn Asn Thr Gln Gln
        35                  40                  45

ctt tac tac ccg tac acc gaa aac ggt gtt tat aaa gca cac tac ata      192
Leu Tyr Tyr Pro Tyr Thr Glu Asn Gly Val Tyr Lys Ala His Tyr Ile
    50                  55                  60

aaa gca att aac cca gac gaa ggc gac gat ata aga aca gaa ggg caa      240
Lys Ala Ile Asn Pro Asp Glu Gly Asp Asp Ile Arg Thr Glu Gly Gln
65                  70                  75                  80

tcg tgg gga atg act gcc gct atc atg ctt aat aaa caa gaa gaa ttt      288
Ser Trp Gly Met Thr Ala Ala Ile Met Leu Asn Lys Gln Glu Glu Phe
                85                  90                  95

gat aat cta tgg cgc ttt gca aaa gcg tat caa aaa aat cca gac aac      336
Asp Asn Leu Trp Arg Phe Ala Lys Ala Tyr Gln Lys Asn Pro Asp Asn
            100                 105                 110

cac cct gat gta aaa aaa caa ggc gtt tac gcg tgg aaa cta aag ctt      384
His Pro Asp Val Lys Lys Gln Gly Val Tyr Ala Trp Lys Leu Lys Leu
        115                 120                 125

aat caa aac ggc ttt gtt tat aaa gta gat gaa ggc ccc gct ccg gat      432
Asn Gln Asn Gly Phe Val Tyr Lys Val Asp Glu Gly Pro Ala Pro Asp
    130                 135                 140

ggc gaa gag tac ttt gcg ttt gca cta ctt aat gcc tcc gct cgt tgg 480
Gly Glu Glu Tyr Phe Ala Phe Ala Leu Leu Asn Ala Ser Ala Arg Trp
145                 150                 155                 160

ggt aat tca ggg gag ttt aac tac tac aac gat gca att acc atg tta 528
Gly Asn Ser Gly Glu Phe Asn Tyr Tyr Asn Asp Ala Ile Thr Met Leu
                165                 170                 175

aac aca att aaa aat aag ctg atg gaa aac caa ata ata cgc ttt tcg 576
Asn Thr Ile Lys Asn Lys Leu Met Glu Asn Gln Ile Ile Arg Phe Ser
            180                 185                 190

cct tac att gat aac cta aca gac cct tct tac cat ata cct gcg ttt 624
Pro Tyr Ile Asp Asn Leu Thr Asp Pro Ser Tyr His Ile Pro Ala Phe
        195                 200                 205

tac gac tac ttt gct aat aac gta act aac caa gca gac aaa act tac 672
Tyr Asp Tyr Phe Ala Asn Asn Val Thr Asn Gln Ala Asp Lys Thr Tyr
    210                 215                 220

tgg cga caa gta gcc aca aaa agt aga acc tta ctc aaa aac cat ttt 720
Trp Arg Gln Val Ala Thr Lys Ser Arg Thr Leu Leu Lys Asn His Phe
225                 230                 235                 240

aca aaa gta agt ggt agc ccg cat tgg aac tta cct aca ttt tta tcg 768
Thr Lys Val Ser Gly Ser Pro His Trp Asn Leu Pro Thr Phe Leu Ser
                245                 250                 255

cgc tta gat ggc agc cct gtt att ggc tac att ttt aac ggc caa gca 816
Arg Leu Asp Gly Ser Pro Val Ile Gly Tyr Ile Phe Asn Gly Gln Ala
            260                 265                 270

aac cca ggt caa tgg tat gaa ttt gat gca tgc cgc gta ttt atg aat 864
Asn Pro Gly Gln Trp Tyr Glu Phe Asp Ala Cys Arg Val Phe Met Asn
        275                 280                 285

gtg ggg tta gag gcg cat ttt atg ggt gct caa gcg tgg cat aaa agt 912
Val Gly Leu Glu Ala His Phe Met Gly Ala Gln Ala Trp His Lys Ser
    290                 295                 300

gca gtt aat aaa gca ctg ggc ttt tta agt tat gca aaa aca aac aac 960
Ala Val Asn Lys Ala Leu Gly Phe Leu Ser Tyr Ala Lys Thr Asn Asn
305                 310                 315                 320

agt aaa aac tgt tac gag caa gtg tat tcg tac ggt gga gcg caa aac 1008
Ser Lys Asn Cys Tyr Glu Gln Val Tyr Ser Tyr Gly Gly Ala Gln Asn
                325                 330                 335

aga ggc tgt gca ggc gaa ggt caa aaa gcc gcg aat gca gta gcg tta 1056
Arg Gly Cys Ala Gly Glu Gly Gln Lys Ala Ala Asn Ala Val Ala Leu
            340                 345                 350

ctt gct tca aca aat gct ggg caa gca aat gag ttt ttt aac gaa ttt 1104
Leu Ala Ser Thr Asn Ala Gly Gln Ala Asn Glu Phe Phe Asn Glu Phe
        355                 360                 365

tgg tct tta tcg caa cca acg ggt gac tac cgt tac tat aat ggt tcg 1152
Trp Ser Leu Ser Gln Pro Thr Gly Asp Tyr Arg Tyr Tyr Asn Gly Ser
    370                 375                 380

tta tat atg tta gct atg ctg cat gta tcg ggc aat ttt aag ttt tat 1200
Leu Tyr Met Leu Ala Met Leu His Val Ser Gly Asn Phe Lys Phe Tyr
385                 390                 395                 400

aac aac acg ttt aat taa 1218
Asn Asn Thr Phe Asn
                405

<210>  33
<211>  405
<212>  PRT
<213>  strain Sp.23.2

<400>  33

Ala Phe Asn Asn Asn Pro Ser Ser Val Gly Ala Tyr Ser Ser Gly Thr
1               5                   10                  15

Tyr Arg Asn Leu Ala Gln Glu Met Gly Lys Thr Asn Ile Gln Gln Lys
            20                  25                  30

Val Asn Ser Thr Phe Asp Asn Met Phe Gly Tyr Asn Asn Thr Gln Gln
        35                  40                  45

Leu Tyr Tyr Pro Tyr Thr Glu Asn Gly Val Tyr Lys Ala His Tyr Ile
    50                  55                  60

Lys Ala Ile Asn Pro Asp Glu Gly Asp Asp Ile Arg Thr Glu Gly Gln
65                  70                  75                  80

Ser Trp Gly Met Thr Ala Ala Ile Met Leu Asn Lys Gln Glu Glu Phe
                85                  90                  95

Asp Asn Leu Trp Arg Phe Ala Lys Ala Tyr Gln Lys Asn Pro Asp Asn
            100                 105                 110

His Pro Asp Val Lys Lys Gln Gly Val Tyr Ala Trp Lys Leu Lys Leu
        115                 120                 125

Asn Gln Asn Gly Phe Val Tyr Lys Val Asp Glu Gly Pro Ala Pro Asp
    130                 135                 140

Gly Glu Glu Tyr Phe Ala Phe Ala Leu Leu Asn Ala Ser Ala Arg Trp
145                 150                 155                 160

Gly Asn Ser Gly Glu Phe Asn Tyr Tyr Asn Asp Ala Ile Thr Met Leu
                165                 170                 175

Asn Thr Ile Lys Asn Lys Leu Met Glu Asn Gln Ile Ile Arg Phe Ser
            180                 185                 190

Pro Tyr Ile Asp Asn Leu Thr Asp Pro Ser Tyr His Ile Pro Ala Phe
        195                 200                 205

Tyr Asp Tyr Phe Ala Asn Asn Val Thr Asn Gln Ala Asp Lys Thr Tyr
    210                 215                 220

Trp Arg Gln Val Ala Thr Lys Ser Arg Thr Leu Leu Lys Asn His Phe
225                 230                 235                 240

Thr Lys Val Ser Gly Ser Pro His Trp Asn Leu Pro Thr Phe Leu Ser
                245                 250                 255

Arg Leu Asp Gly Ser Pro Val Ile Gly Tyr Ile Phe Asn Gly Gln Ala
            260                 265                 270

Asn Pro Gly Gln Trp Tyr Glu Phe Asp Ala Cys Arg Val Phe Met Asn
        275                 280                 285

Val Gly Leu Glu Ala His Phe Met Gly Ala Gln Ala Trp His Lys Ser
    290                 295                 300

Ala Val Asn Lys Ala Leu Gly Phe Leu Ser Tyr Ala Lys Thr Asn Asn
305                 310                 315                 320

Ser Lys Asn Cys Tyr Glu Gln Val Tyr Ser Tyr Gly Gly Ala Gln Asn
                325                 330                 335

Arg Gly Cys Ala Gly Glu Gly Gln Lys Ala Ala Asn Ala Val Ala Leu
            340                 345                 350

Leu Ala Ser Thr Asn Ala Gly Gln Ala Asn Glu Phe Phe Asn Glu Phe
        355                 360                 365

Trp Ser Leu Ser Gln Pro Thr Gly Asp Tyr Arg Tyr Tyr Asn Gly Ser
    370                 375                 380

Leu Tyr Met Leu Ala Met Leu His Val Ser Gly Asn Phe Lys Phe Tyr
385                 390                 395                 400

Asn Asn Thr Phe Asn
                405